(19) Europäisches Patentamt
European Patent Office
Office européen des brevets

(11) EP 4 778 511 A1

(12) **EUROPEAN PATENT APPLICATION**
published in accordance with Art. 153(4) EPC

(43) Date of publication:
**22.07.2026 Bulletin 2026/30**

(21) Application number: **24864755.4**

(22) Date of filing: **13.09.2024**

(51) International Patent Classification (IPC):
***A61K 9/00** (2006.01)* ***A61K 47/34** (2017.01)*
***A61K 31/57** (2006.01)* ***A61K 31/519** (2006.01)*
***A61K 31/198** (2006.01)* ***A61P 25/18** (2006.01)*
***A61P 15/18** (2006.01)* ***A61P 5/14** (2006.01)*

(52) Cooperative Patent Classification (CPC):
**A61K 9/00; A61K 31/198; A61K 31/519; A61K 31/57; A61K 47/34; A61P 5/14; A61P 15/18; A61P 25/18**

(86) International application number:
**PCT/CN2024/118883**

(87) International publication number:
**WO 2025/056040 (20.03.2025 Gazette 2025/12)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **13.09.2023 CN 202311180554**
**13.09.2023 CN 202311180555**
**13.09.2023 CN 202311180553**

(71) Applicant: **SHENYANG XINGHUA PHARMACEUTICAL TECHNOLOGY CO., LTD**
**Shenyang, Liaoning 110000 (CN)**

(72) Inventors:
- **YANG, Xinggang**
  **Shenyang, Liaoning 110000 (CN)**
- **LI, Dan**
  **Shenyang, Liaoning 110000 (CN)**
- **SUN, Yuanhang**
  **Shenyang, Liaoning 110000 (CN)**
- **SHEN, Rui**
  **Shenyang, Liaoning 110000 (CN)**
- **DONG, Hongyang**
  **Shenyang, Liaoning 110000 (CN)**
- **LIU, Baolin**
  **Shenyang, Liaoning 110000 (CN)**
- **SHENG, Huali**
  **Shenyang, Liaoning 110000 (CN)**
- **MU, Chengqiao**
  **Shenyang, Liaoning 110000 (CN)**
- **WANG, Tianyuan**
  **Shenyang, Liaoning 110000 (CN)**

(74) Representative: **Michalski Hüttermann & Partner mbB**
**Kaistraße 16A**
**40221 Düsseldorf (DE)**

# (54) PHARMACEUTICAL COMPOSITION, DRUG-LOADED ROD CORE, IMPLANT, PREPARATION METHOD THEREFOR, AND USE THEREOF

(57) Provided in the present invention are a pharmaceutical composition, a drug-loaded rod core, an implant, a preparation method therefor, and the use thereof. The pharmaceutical composition comprises 1-1000 parts of a drug active ingredient, 100 parts of R-vinyl silicone rubber, hydrogen-containing silicone oil, and 0.000002-1 part of a catalyst. R in the R-vinyl silicone rubber is a substituted or unsubstituted $C_1$-$C_5$ linear alkane or branched alkane or a substituted or unsubstituted $C_6$-$C_{20}$ aromatic hydrocarbon; and the content of vinyl in the R-vinyl silicone rubber is 0. 10-0.50 mol%. The content of Si-H group in the hydrogen-containing silicone oil is 0.15-1.6 mol%, and the molar ratio of the Si-H group in the hydrogen-containing silicone oil to the vinyl in the R-vinyl silicone rubber is (0.5-20): 1. The implant provided by the present invention can reduce drug loss, reduce the phenomenon of burst release, achieve more stable drug release and involve a greatly improved production speed.

EP 4 778 511 A1

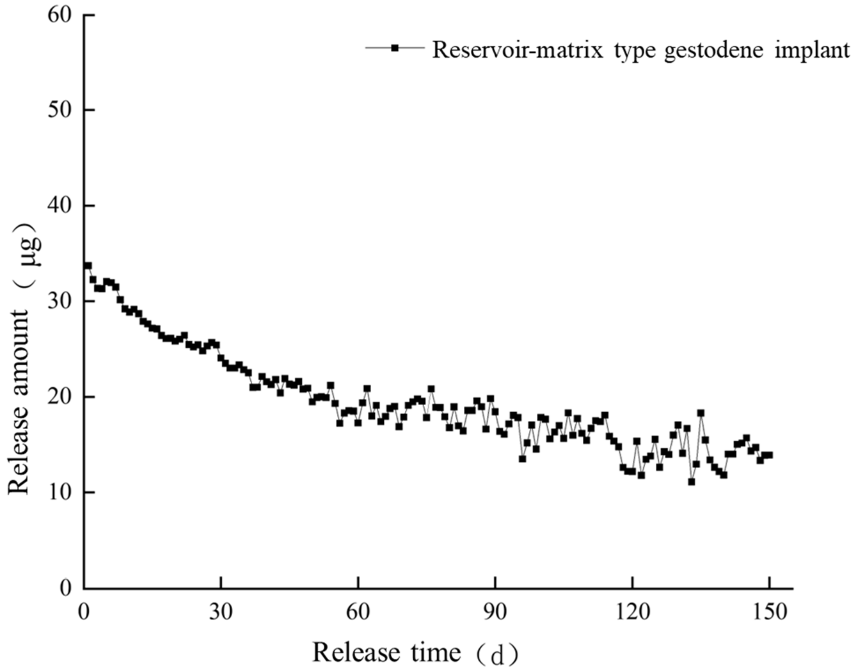
60
50
40
30
20
10
0
Reservoir-matrix type gestodene implant
Release amount（μg）
0
30
60
90
120
150
Release time（d）

FIG. 2

## Description

The present disclosure claims the priority of Chinese patent application 202311180554.X filed on September 13, 2023. The contents of the Chinese patent application are incorporated herein by reference in their entireties.
The present application claims the priority of Chinese patent application 202311180553.5 filed on September 13, 2023. The contents of the Chinese patent application are incorporated herein by reference in their entireties.
The present application claims the priority of Chinese patent application 202311180555.4 filed on September 13, 2023. The contents of the Chinese patent application are incorporated herein by reference in their entireties.

## TECHNICAL FIELD

The present disclosure relates to a pharmaceutical composition, a drug-loaded rod core, an implant and a preparation method therefor and use thereof.

## BACKGROUND

Implantable drug delivery system (IDDS) is a drug delivery system that can stably release drugs to specific sites of action. The main advantage is that the drug is delivered at a sustained and stable rate, and less drugs are needed in the process of treating diseases, so the toxic and side effects of drugs can be greatly reduced to achieve better therapeutic effects. Implantable drug delivery system can reduce the frequency of drug administration from once or several times a day to once a few months, once a year or once every three to five years, reduce the side effects caused by frequent drug administration, and avoid problems such as first pass and peak valley of oral administration.
According to the differences in the forms and ways of use of drugs in preparations, IDDS can be mainly divided into solid drug-loaded implants, injection implants and implant pump preparations. Solid drug-loaded implants refer to drugs dispersed in carrier materials, which are implanted and administered by surgery in the form of columns, rods, pills, tablets, etc., and can be implanted subcutaneously or in specific target sites. It is the most widely used IDDS at present.
The solid drug-loaded implant has the advantages of simple and convenient use process, safe and stable carrier material and low price. At the same time, compared with the solid drug-loaded implants implanted in specific target sites, the technology of subcutaneous implanted solid drug-loaded implants is more mature. Subcutaneous implants such as Norplant and Implanon have been used in the field of contraception for decades, and their safety and effectiveness have been fully confirmed.
Implantation administration has a wide range of uses. In addition to the well-known field of contraception, clinical experimental research has been gradually carried out in other long-term drug fields, such as tumor treatment. The development of local implantation technology has enabled many traditional anti-tumor drugs to have new uses. In the prevention and treatment of AIDS, long-term implantation can replace daily oral administration to avoid the phenomenon of missing medication affecting the drug effect. In addition, implantable drug delivery system is also applied to the treatment of diabetes, mental system diseases, withdrawal addiction, cardiovascular diseases and other long-term drug use fields, and the application prospect is expected in the future.
Traditional solid drug-loaded implants are mainly prepared by filling drug powder into silicone tubes. The filling process of drug powder is slow, and at the same time, a large amount of drug powder will adsorb onto the inner wall of the silicone tube, resulting in drug waste and low dispensing speed. In practical use, a series of drawbacks also exist, such as severe burst release, poor stability of drug release, and low production efficiency and etc. Therefore, it is urgent to study a new type of solid drug-loaded implant.

## SUMMARY

The technical problem to be solved in the present disclosure is for overcoming the defects of serious drug waste, serious sudden release phenomenon, poor stability of drug release of the solid drug loaded implant and slow production speed of the implant in the prior art, and provide a pharmaceutical composition, a rod core and an implant as well as a preparation method therefor and use thereof. The implant provided by the present disclosure can reduce drug loss, reduce the phenomenon of burst release, achieve more stable drug release and involve a greatly improved production speed.
The present disclosure solves the aforementioned technical problem through the following technical solutions:
**The present disclosure provides a pharmaceutical composition,** which comprises the following raw material components in parts by weight:

1-1000 parts of active pharmaceutical ingredient;
100 parts of R-vinyl silicone rubber;
hydrogen-containing silicone oil;

0.000002-1 part of catalyst;
wherein:

in the R-vinyl silicone rubber, R is a substituted or unsubstituted $C_1$-$C_5$ linear alkane or branched alkane, or a substituted or unsubstituted $C_6$-$C_{20}$ aromatic hydrocarbon;
the content of vinyl in the R-vinyl silicone rubber is 0.10%-0.50 mol%;
the content of Si-H groups in the hydrogen-containing silicone oil is 0.15-1.6 mol%;
the molar ratio of Si-H groups in the hydrogen-containing silicone oil to vinyl in the R-vinyl silicone rubber is (0.5-20):1.

In the present disclosure, the structural formula of the R- vinyl silicone rubber can be as follows:

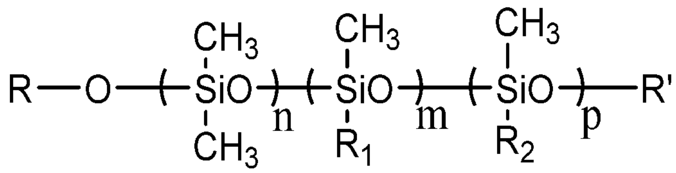

wherein m, n and p are natural numbers, $R_1$ and $R_2$ can be conventional substituent groups in the field, R and R' can be conventional end-capping groups in the field.
For example, when $R_1$=$R_2$=$CH_3$, it corresponds to dimethyl silicone rubber;

For example, when $R_1$=$CH_3$, $R_2$=CH=$CH_2$, it corresponds to methyl vinyl silicone rubber;
For example, when $R_1$=Ph, $R_2$=CH=$CH_2$, it corresponds to methyl phenyl vinyl silicone rubber;
For example, when $R_1$=$CH_3CH_2CF_3$, $R_2$=CH=$CH_2$, it corresponds to fluorosilicone rubber;
For example, when $R_1$=$CH_3CH_2CN$, $R_2$=CH=$CH_2$, it corresponds to nitrile silicone rubber.

In the present disclosure, the active pharmaceutical ingredient can be a conventional slow-release pharmaceutical ingredient in this field that requires long-term drug release, preferably an active drug with a solubility of ≤ 100mg/mL (using water as a solvent), more preferably comprising the active pharmaceutical ingredient acting on the reproductive system, or, the active pharmaceutical ingredient acting on the urinary system, or, the active pharmaceutical ingredient acting on chronic diseases related to metabolism and nutrition, or, the active pharmaceutical ingredient for treating chronic diseases related to connective tissue and rheumatism, or, the active pharmaceutical ingredient for treating hyperlipidemia, tumors, neuropsychiatric diseases, chronic dental diseases, simple obesity, chronic low back pain, or leukemia, or, the active pharmaceutical ingredient acting on the circulatory system, or, the active pharmaceutical ingredient acting on the respiratory system, or, the active pharmaceutical ingredient acting on the digestive system, or, the active pharmaceutical ingredient acting on the blood system, or, the active pharmaceutical ingredient acting on the endocrine system.
Wherein, the active pharmaceutical ingredient acting on the reproductive system may include a contraceptive active pharmaceutical ingredient.
The contraceptive active pharmaceutical ingredient may be conventional in the art, preferably including levonorgestrel, gestodene, gestrinone or steroidal estrogen.
The steroidal estrogen is preferably estradiol.
Wherein, the active pharmaceutical ingredient acting on the urinary system preferably includes an active pharmaceutical ingredient for treating chronic prostatitis, chronic nephritis, chronic renal failure, or, chronic prostatitis.
The active pharmaceutical ingredient for treating chronic prostatitis is preferably a non-steroidal anti-inflammatory drug, more preferably ibuprofen. Generally, the drug for treating chronic prostatitis may be: antibiotics (e.g., fluoroquinolones (e.g., norfloxacin, enoxacin, ofloxacin, ciprofloxacin), macrolides (e.g., erythromycin, roxithromycin, azithromycin, acetylspiramycin), tetracyclines (tetracycline), α-receptor blockers (e.g., doxazosin, terazosin), non-steroidal anti-inflammatory drugs (e.g., ibuprofen, diclofenac, loxoprofen, flurbiprofen axetil, ketorolac, celecoxib), analgesics (e.g., acetaminophen), opioids (e.g., buprenorphine, fentanyl, nalbuphine, pentazocine), anti-neuropathic pain drugs (e.g., 5-hydroxytryptamine, duloxetine, venlafaxine), antiepileptics (e.g., pregabalin), M-receptor blockers (e.g., solifenacin, tolterodine).
The active pharmaceutical ingredient for treating chronic nephritis may generally be: angiotensin-converting enzyme inhibitors (e.g., benazepril, ramipril, fosinopril, perindopril, cilazapril or enalapril), angiotensin II receptor blockers (e.g., irbesartan, valsartan or losartan), calcium channel blockers (e.g., amlodipine, felodipine or nifedipine), β-receptor blockers (e.g., atenolol, bisoprolol, carvedilol or propranolol), diuretics (e.g., furosemide, spironolactone, hydrochlorothiazide, bendroflumethiazide or bumetanide), immunosuppressants (e.g., prednisone, leflunomide, methylprednisolone, cyclophosphamide or dexamethasone), or statin lipid-lowering drugs (e.g., fluvastatin, simvastatin or pravastatin).

The active pharmaceutical ingredient for treating chronic renal failure may generally be: angiotensin-converting enzyme inhibitors (e.g., benazepril, ramipril, fosinopril, perindopril, cilazapril, enalapril), angiotensin II receptor blockers (e.g., irbesartan, valsartan, losartan), diuretics (e.g., furosemide, spironolactone, hydrochlorothiazide, bendroflumethiazide, bumetanide), or iron supplements (e.g., ferrous fumarate), compound amino acids, $\alpha$-keto acids, recombinant human erythropoietin.

The drug for treating chronic prostatitis may be: antibiotics (e.g., fluoroquinolones (norfloxacin, enoxacin, ofloxacin, ciprofloxacin), macrolides (erythromycin, roxithromycin, azithromycin, acetylspiramycin), tetracyclines (tetracycline), $\alpha$-receptor blockers (e.g., doxazosin, terazosin), non-steroidal anti-inflammatory drugs (e.g., ibuprofen, diclofenac, loxoprofen, flurbiprofen axetil, ketorolac, celecoxib), analgesics (e.g., acetaminophen), opioids (e.g., buprenorphine, fentanyl, nalbuphine, pentazocine), anti-neuropathic pain drugs (e.g., 5-hydroxytryptamine, duloxetine, venlafaxine), antiepileptics (e.g., pregabalin), or M-receptor blockers (e.g., solifenacin, tolterodine), preferably non-steroidal anti-inflammatory drugs, more preferably ibuprofen.

Wherein, the active pharmaceutical ingredient acting on chronic diseases related to metabolism and nutrition preferably includes an active pharmaceutical ingredient for anti-diabetes, treating nutritional deficiency diseases, anti-gout, or anti-osteoporosis.

The active pharmaceutical ingredient for anti-diabetes may generally be an anti-type 1 diabetes drug (e.g., insulin) or an anti-type 2 diabetes drug. The anti-type 2 diabetes drug may be a sulfonylurea (e.g., glimepiride, gliquidone), a glinide (e.g., repaglinide, nateglinide), a metformin (e.g., metformin), an $\alpha$-glucosidase inhibitor (e.g., acarbose, voglibose), a DPP-4 inhibitor (e.g., sitagliptin), a GLP-1 receptor agonist (e.g., liraglutide, exenatide), or a SGLT-2 inhibitor (e.g., dapagliflozin, empagliflozin).

The active pharmaceutical ingredient for treating nutritional deficiency diseases may generally be a drug targeting gastrointestinal diseases (e.g., omeprazole, mosapride) or a nutritional supplement (e.g., vitamin E).

The active pharmaceutical ingredient for anti-gout is an anti-gout drug (e.g., colchicine, indomethacin, diclofenac, ibuprofen, rofecoxib, prednisone, hydrocortisone, prednisolone, aspirin, diflunisal, para-aminosalicylic acid, salsalate, benorilate), a uric acid excretion drug (e.g., benzbromarone, probenecid), or a uric acid synthesis inhibitor drug (e.g., allopurinol), preferably anti-gout is an anti-gout drug, more preferably ibuprofen.

The active pharmaceutical ingredient for anti-osteoporosis may generally be a bisphosphonate drug (e.g., alendronate sodium, ibandronate sodium, pamidronate sodium, aminobisphosphonate, clodronate disodium, zoledronate sodium, risedronate sodium), a calcitonin drug (e.g., salmon calcitonin), an estrogen drug (e.g., estradiol, estradiol benzoate, estradiol acetate, estradiol valerate), a selective estrogen receptor modulator (SERM) drug (e.g., raloxifene, benzothiophene), a RANKL inhibitor drug, a parathyroid hormone drug, a glutamine monofluorophosphate drug, a strontium salt drug (e.g., strontium ranelate), active vitamin D and an analogue thereof (e.g., calcitriol, alfacalcidol), or vitamin K (e.g., menatetrenone).

Wherein, the active pharmaceutical ingredient for treating chronic diseases related to connective tissue and rheumatism preferably includes an active pharmaceutical ingredient for treating rheumatoid arthritis, systemic lupus erythematosus, ankylosing spondylitis, Sjogren's syndrome, vasculitis, idiopathic inflammatory myopathy, systemic sclerosis, or osteoarthritis.

The active pharmaceutical ingredient for treating rheumatoid arthritis may be a glucocorticoid drug (e.g., hydrocortisone, dexamethasone, prednisone) or a slow-acting antirheumatic drug (e.g., methotrexate, cyclophosphamide, azathioprine, cyclosporine, leflunomide) or a non-steroidal anti-inflammatory drug (e.g., aspirin, meloxicam, celecoxib, ibuprofen, nimesulide, nabumetone), preferably a non-steroidal anti-inflammatory drug, more preferably meloxicam or ibuprofen.

The active pharmaceutical ingredient for treating osteoarthritis is preferably an analgesic drug, such as ibuprofen, voltaren, meloxicam, celecoxib, loxoprofen sodium nimesulide, more preferably ibuprofen or meloxicam. Generally, the drug for treating osteoarthritis may be a cartilage-nourishing drug or an opioid drug (e.g., oxycodone, tramadol).

The active pharmaceutical ingredient for treating systemic lupus erythematosus may generally be a non-steroidal anti-inflammatory drug (e.g., naproxen, fenbid), an antimalarial drug, a glucocorticoid drug (e.g., prednisone acetate, methylprednisolone), an immunosuppressant drug (e.g., methotrexate, cyclophosphamide, azathioprine, methotrexate, cyclosporine, mycophenolate mofetil, tacrolimus), or a biologic agent drug (e.g., rituximab, belimumab).

The active pharmaceutical ingredient for treating ankylosing spondylitis may generally be a non-steroidal drug (e.g., diclofenac sodium, etoricoxib, celecoxib, nabumetone, imrecoxib), a disease-modifying antirheumatic drug (e.g., sulfasalazine, methotrexate, hydroxychloroquine), a glucocorticoid (e.g., prednisone, prednisolone, diprospan), or a biological TNF-$\alpha$ antagonist (e.g., etanercept).

The active pharmaceutical ingredient for treating Sjogren's syndrome may generally be a systemic therapy drug (e.g., levamisole, transfer factor coenzyme Q10, thymosin), a glucocorticoid drug (e.g., prednisone), or an immunosuppressant drug (e.g., hydroxychloroquine, azathioprine, iguratimod).

The active pharmaceutical ingredient for treating vasculitis may generally be a glucocorticoid drug (e.g., prednisone, methylprednisolone, dexamethasone) or an immunosuppressant drug (e.g., cyclophosphamide, cyclosporine, azathioprine).

The active pharmaceutical ingredient for treating idiopathic inflammatory myopathy may generally be a glucocorticoid drug (e.g., dexamethasone, prednisone, hydrocortisone, methylprednisolone) or an immunosuppressant drug (e.g., cyclophosphamide, azathioprine, methotrexate, cyclosporine, tacrolimus, mycophenolate mofetil, mycophenolate).

The active pharmaceutical ingredient for treating systemic sclerosis may generally be an antifibrotic drug (e.g., colchicine), a vasoactive drug (e.g., Nifedipine, Adalat), naproxen, nifedipine, bosentan, sildenafil, epoprostenol, nintedanib or tocilizumab.

Wherein, the active pharmaceutical ingredient for treating neuropsychiatric diseases may generally be an antipsychotic drug for schizophrenia, an antidepressant drug, a drug for treating opioid abuse, or an anxiolytic drug, preferably an antipsychotic drug for schizophrenia.

The drug for treating schizophrenia is preferably a phenothiazine (e.g., chlorpromazine), a thioxanthene (e.g., chlorprothixene), a butyrophenone (e.g., haloperidol), a dibenzodiazepine (e.g., olanzapine, clozapine), a benzisoxazole (e.g., risperidone, paliperidone), a benzisothiazole (e.g., ziprasidone), a dibenzothiazepine (e.g., quetiapine), or a quinolone (e.g., aripiprazole), more preferably a benzisoxazole (e.g., paliperidone).

The antidepressant drug may generally be a tricyclic antidepressant (e.g., imipramine, clomipramine, amitriptyline), a monoamine oxidase inhibitor (e.g., moclobemide), a selective serotonin reuptake inhibitor (e.g., sertraline), a serotonin-norepinephrine reuptake inhibitor (e.g., venlafaxine), a serotonin antagonist and reuptake inhibitor (e.g., trazodone), a norepinephrine-dopamine reuptake inhibitor (e.g., bupropion), a norepinephrine inhibitor (e.g., reboxetine), or an $\alpha$2-adrenergic receptor blocker (e.g., mirtazapine).

The drug for treating opioid abuse may generally be buprenorphine or methadone.

The drug for treating anxiety disorders may generally be a benzodiazepine (e.g., diazepam), a 5-HT1A receptor partial agonist (e.g., buspirone), a $\beta$-adrenergic receptor blocker (e.g., propranolol), or a valproate.

Wherein, the active pharmaceutical ingredient for treating hyperlipidemia may generally be a statin drug (e.g., simvastatin, atorvastatin, pravastatin), a fibrate drug (e.g., fenofibrate, bezafibrate, gemfibrozil), or a niacin (e.g., nicotinic acid).

Wherein, the active pharmaceutical ingredient for treating tumors may generally be an anti-breast cancer drug (e.g., azacitidine, docetaxel, buserelin, tamoxifen, mitoxantrone, adriamycin, paclitaxel, capecitabine, goserelin, cyclophosphamide, megestrol, cetuximab, or leuprorelin), an anti-prostate cancer drug (e.g., degarelix, leuprorelin, histrelin, flutamide, estramustine, cyproterone), an anti-ovarian cancer drug (e.g., carboplatin, topotecan, methotrexate), an anti-rectal cancer drug (e.g., panitumumab), an anti-colon cancer drug (e.g., bevacizumab, oxaliplatin), an anti-liver cancer drug (e.g., sorafenib), an anti-lung cancer drug (e.g., erlotinib, gefitinib, decitabine), an anti-kidney cancer drug (e.g., pazopanib, everolimus, temsirolimus), an anti-gastric cancer drug (e.g., fluorouracil, mitomycin, cisplatin, adriamycin, etoposide), an anti-pancreatic cancer drug (e.g., nimotuzumab), an anti-esophageal cancer drug (e.g., docetaxel, paclitaxel, cisplatin, gemcitabine, tegafur, irinotecan, oxaliplatin, gefitinib, trastuzumab, anlotinib), an anti-skin cancer drug (e.g., fluorouracil), an anti-lymphoma drug (e.g., vincristine, bexarotene, dacarbazine, etoposide), an anti-myeloma drug (e.g., bortezomib), an anti-cervical cancer drug (e.g., bleomycin), or an anti-bladder cancer drug (e.g., epirubicin, BCG vaccine).

Wherein, the active pharmaceutical ingredient for treating chronic dental diseases may generally be nitroimidazole drug (e.g., metronidazole, tinidazole, ornidazole), penicillin drug, minocycline or chlorhexidine diacetate. The treatment of chronic dental diseases generally refers to dental caries or periodontal disease.

Wherein, the drug for treating simple obesity may generally be orlistat.

Wherein, the active pharmaceutical ingredient for treating chronic low back pain is preferably a non-steroidal analgesic drug, such as ibuprofen, celecoxib, tramadol, oxycodone, meloxicam, loxoprofen, acetaminophen-codeine, or voltaren, more preferably ibuprofen or meloxicam.

Wherein, the active pharmaceutical ingredient for treating leukemia is generally a drug that interferes with nucleic acid biosynthesis (e.g., cytarabine, methotrexate, 6-mercaptopurine), a drug that directly affects the structure and function of DNA in cancer cells (e.g., busulfan, mitomycin, chlorambucil, melphalan, cyclophosphamide), a drug that interferes with transcription process and prevents RNA synthesis (e.g., daunorubicin, doxorubicin, adriamycin, aclacinomycin), a drug that inhibits protein synthesis and function (e.g., vindesine, vincristine, L-asparaginase, homoharringtonine), interferon, fludarabine, arsenic trioxide, etoposide or carmustine.

Wherein, the active pharmaceutical ingredient acting on the circulatory system preferably includes an active pharmaceutical ingredient for treating chronic heart failure, coronary heart disease, congenital heart disease, chronic infective endocarditis, or chronic pericarditis.

The active pharmaceutical ingredient for treating coronary heart disease may generally be a drug that improves

angina symptoms (e.g., puerarin, isosorbide mononitrate), a drug that inhibits platelet aggregation (e.g., aspirin, clopidogrel bisulfate, ticagrelor), a drug that lowers lipids and stabilizes plaques (e.g., atorvastatin, rosuvastatin, pravastatin), a drug that inhibits sympathetic nerve activity (e.g., metoprolol, bisoprolol fumarate), or improves myocardial remodeling (e.g., angiotensin-converting enzyme inhibitors and angiotensin II receptor antagonists), preferably a drug that improves angina symptoms, more preferably puerarin.

The active pharmaceutical ingredient for treating chronic heart failure may generally be a cardiotonic drug (e.g., digitalis, digoxin, cedilanid), a vasodilator drug (e.g., sodium nitroprusside, nitroglycerin), a converting enzyme inhibitor (e.g., enalapril, lisinopril, etc.), or a diuretic drug (e.g., furosemide, hydrochlorothiazide, spironolactone).

The active pharmaceutical ingredient for treating congenital heart disease may generally be digitalis, furosemide, spironolactone, phentolamine, quinidine, digoxin, hydrochlorothiazide, or coenzyme Q10.

The active pharmaceutical ingredient for treating chronic infective endocarditis may generally be an antibiotic (e.g., vancomycin, cephalosporin, penicillin, aminoglycoside).

The active pharmaceutical ingredient for treating chronic pericarditis may generally be digitalis.

Wherein, the active pharmaceutical ingredient acting on the respiratory system may generally include an active pharmaceutical ingredient for treating chronic obstructive pulmonary emphysema, asthma, chronic pulmonary heart disease, chronic respiratory failure, or silicosis, or pulmonary fibrosis.

The active pharmaceutical ingredient for treating chronic obstructive pulmonary emphysema may generally be a bronchodilator (including β-receptor agonists and anticholinergics), an inhaled corticosteroid (e.g., budesonide, fluticasone), a theophylline-based antiasthmatic (e.g., theophylline), an expectorant (e.g., carbocisteine, fudosteine), and, if required, a glucocorticoid or an antibiotic (e.g., penicillins, glycosides, and cephalosporins), as appropriate.

The active pharmaceutical ingredient for treating asthma may generally be a common inhaled drug (e.g., beclomethasone, budesonide, fluticasone, mometasone), a β2-agonist (e.g., salbutamol), a sustained-release theophylline, a leukotriene modifier (which can be used in combination), an anticholinergic (e.g., isopropyl scopolamine), or an antihistamine (e.g., astemizole, ketotifen).

The active pharmaceutical ingredient for treating chronic pulmonary heart disease may generally be an antibiotic (e.g., amoxicillin, ceftizoxime, cefuroxime, levofloxacin), a corticosteroid anti-inflammatory bronchodilator drug (e.g., selective β2-receptor agonists, theophylline drugs), a drug that eliminates nonspecific airway inflammation (e.g., prednisone), an inhaled drug (e.g., becotide), or a respiratory stimulant (e.g., lobeline, doxapram, duxil, etc.).

The active pharmaceutical ingredient for treating chronic respiratory failure may generally be a bronchospasm relieving and expectorant drug (e.g., salbutamol, acetylcysteine, etc.).

The active pharmaceutical ingredient for treating silicosis may generally be Siping, acetylcysteine, aluminum preparations, polyvinylpyridine oxide, or salvia miltiorrhiza.

The active pharmaceutical ingredient for treating pulmonary fibrosis may generally be pirfenidone, nintedanib, glucocorticoids (e.g., methylprednisolone, prednisone), immunosuppressants (e.g., azathioprine, methotrexate, etc.), colchicine, interferon, ACEI, or statins, etc.

Wherein, the active pharmaceutical ingredient acting on the digestive system may generally include an active pharmaceutical ingredient for treating chronic gastritis, peptic ulcers, intestinal tuberculosis, chronic enteritis, chronic diarrhea, chronic hepatitis, liver cirrhosis, chronic pancreatitis, or chronic cholecystitis.

The active pharmaceutical ingredient for treating chronic gastritis may generally be an analgesic drug (e.g., atropine, propantheline bromide), a proton pump inhibitor (PPI) for increased gastric acidity (e.g., lansoprazole, omeprazole), a $H_2$-receptor antagonist for mild symptoms (e.g., cimetidine, ranitidine, aluminum amine hydroxide), a digestive aid (pancreatin), or a drug for bile reflux (e.g., metoclopramide, domperidone, cholestyramine, sucralfate, which can bind with bile acids).

The active pharmaceutical ingredient for treating peptic ulcers may generally be levofloxacin, tinidazole, or omeprazole.

The active pharmaceutical ingredient for treating intestinal tuberculosis may generally be rifampin.

The active pharmaceutical ingredient for treating chronic enteritis may generally be an anti-inflammatory analgesic, a probiotic, or an antispasmodic analgesic (e.g., atropine, propantheline bromide).

The active pharmaceutical ingredient for treating chronic diarrhea may generally be an antidiarrheal drug (e.g., montmorillonite powder, diphenoxylate, loperamide), an intestinal microbial preparation (e.g., lactobacillus, bifidobacterium), or an antispasmodic analgesic (e.g., pinaverium bromide).

The active pharmaceutical ingredient for treating chronic hepatitis may generally be a hepatoprotective drug (e.g., silymarin preparations, schisandra preparations, etc.), an antifibrotic drug (e.g., oral preparations of Chinese patent medicines), an antiviral (e.g., standard interferon and pegylated interferon), an oral nucleoside antiviral drug (e.g., lamivudine, adefovir dipivoxil, telbivudine, entecavir), or an immunosuppressant (azathioprine).

The active pharmaceutical ingredient for treating liver cirrhosis may generally be a drug for treating hepatitis B (e.g., nucleoside analogs), a drug for treating autoimmune hepatitis (e.g., glucocorticoids), an anti-inflammatory drug, a hepatoprotective drug, an antifibrotic drug (e.g., reduced glutathione, polyene phosphatidylcholine, magnesium isoglycyrrhizinate, etc.), a drug for treating spontaneous bacterial peritonitis (e.g., antibiotics), or a drug for treating portal hypertension (e.g., carvedilol).

The active pharmaceutical ingredient for treating chronic pancreatitis may generally be an analgesic drug (e.g., buprenorphine and fentanyl) or a pancreatic enzyme therapy drug (e.g., pancreatin).

The active pharmaceutical ingredient for treating chronic cholecystitis may generally be an antibacterial and anti-inflammatory drug (e.g., levofloxacin, ciprofloxacin, amoxicillin), an antispasmodic analgesic drug, or a choleretic drug (e.g., ursodeoxycholic acid).

Wherein, the active pharmaceutical ingredient acting on the blood system may generally include an active pharmaceutical ingredient for treating chronic anemia, chronic myeloid leukemia, or chronic lymphocytic leukemia.

The active pharmaceutical ingredient for treating chronic anemia may generally be trace elements (e.g., folic acid, vitamin B12), bone marrow stimulants (e.g., strychnine nitrate, securinine, tropane (hyoscyamine)), adenosylcobalamin, glucocorticoids (e.g., prednisone, meprednisone, betamethasone, beclomethasone propionate, prednisolone, hydrocortisone, dexamethasone, prednisone), iron supplements (e.g., ferrous fumarate, ferrous gluconate, ferrous succinate, ferrous lactate, ferric saccharate, low molecular weight iron dextran, ferric carboxymaltose, iron isomaltoside, ferric gluconate, iron oxide nanoparticles, iron sorbitol), or erythropoiesis-stimulating agents (e.g., recombinant human erythropoietin $\alpha$, darbepoetin $\alpha$).

The active pharmaceutical ingredient for treating chronic myeloid leukemia may generally be: tyrosine kinase inhibitors (e.g., imatinib, nilotinib, bosutinib, ponatinib, etc.) or homoharringtonine.

The active pharmaceutical ingredient for treating chronic lymphocytic leukemia may generally be: chemotherapy drugs (e.g., nimustine, fludarabine, chlorambucil, bendamustine, etc.), targeted drugs (e.g., idelalisib, venetoclax, ibrutinib, imatinib, dasatinib, etc.), or monoclonal antibodies (e.g., ofatumumab, rituximab, obinutuzumab, alemtuzumab, etc.).

Wherein, the active pharmaceutical ingredient acting on the endocrine system may generally include an active pharmaceutical ingredient for treating chronic lymphocytic thyroiditis, hyperthyroidism, or hypothyroidism.

The active pharmaceutical ingredient for treating chronic lymphocytic thyroiditis may generally be: thyroid hormones (e.g., levothyroxine, thyroxine) or glucocorticoids (e.g., prednisone, meprednisone, betamethasone, beclomethasone propionate, prednisolone, hydrocortisone, dexamethasone, prednisone).

The active pharmaceutical ingredient for treating hyperthyroidism may generally be: thiouracils (e.g., propylthiouracil, methylthiouracil), imidazoles (e.g., methimazole, carbimazole), iodine or iodides (e.g., Lugol's solution), radioactive iodine (e.g., iodine-131), or $\beta$-blockers (e.g., metoprolol, atenolol, bisoprolol, carvedilol, propranolol).

The active pharmaceutical ingredient for treating hypothyroidism may generally be: thyroid hormones (e.g., levothyroxine, levothyroxine sodium, thyroxine).

In the present disclosure, the amount of the active pharmaceutical ingredient is preferably 5-500 parts, more preferably, 5-100 parts, such as 10 parts, 20 parts, 50 parts, or 100 parts.

In the present disclosure, the R-vinyl silicone rubber is preferably methyl vinyl silicone rubber.

In the present disclosure, the content of vinyl in the R-vinyl silicone rubber is preferably 0.17-0.23mol%, such as 0.17 mol%, 0.18 mol%, 0.19 mol%, 0.20 mol%, 0.21 mol%, 0.22 mol% or 0.23 mol%.

In the present disclosure, the amount of the hydrogen-containing silicone oil can be 0.3-20 parts, preferably, 0.3-10 parts, such as 0.3 part, 0.667 part, or 1 part.

In the present disclosure, the content of Si-H groups in the hydrogen-containing silicone oil is preferably 0.5-1.0 mol%, such as 0.75 mol%.

In the present disclosure, the molar ratio of Si-H groups in the hydrogen-containing silicone oil to vinyl in the R-vinyl silicone rubber is preferably (1-10):1, such as 1.5:1, 2:1, 3:1, 3.5:1, 4:1, 4.5:1 or 6:1.

In the present disclosure, the catalyst can be a conventional catalyst in the art capable of catalyzing the addition reaction between the R-vinyl silicone rubber and the hydrogen-containing silicone oil, such as a platinum catalyst, a platinum-containing catalyst, a rhodium catalyst, a palladium catalyst, or a nickel catalyst, preferably a platinum catalyst and/or a platinum-containing catalyst. When the catalyst is a platinum catalyst, the concentration of platinum in the platinum catalyst is preferably $1\times 10^3$ to $1\times10^5$ ppm, such as $1\times10^3$ ppm, $1\times10^4$ ppm or $1\times 10^5$ ppm.

In the present disclosure, the amount of the catalyst is preferably 0.000002-0.5 part, more preferably, 0.05-0.4 part, such as 0.1 part, 0.20 part, 0.21 part, 0.25 part, 0.27 part, or 0.30 part.

In the present disclosure, the pharmaceutical composition may preferably further include an inhibitor, which generally refers to an inhibitor capable of inhibiting the addition reaction between the R-vinyl silicone rubber and the hydrogen-containing silicone oil. The inhibitor can be a hydrocarbon. preferably, the molecular structure of the hydrocarbon contains at least one alkynyl group and one hydroxyl group, such as 2-methyl-3-butyn-2-ol. The amount of the inhibitor is preferably 0.03-2.0 parts, such as 0.49 part or 0.98 part.

In the present disclosure, when the active pharmaceutical ingredient is the active pharmaceutical ingredient acting on the reproductive system, the particle size of the active pharmaceutical ingredient may be 0.001-200 $\mu$m, preferably 2-80 $\mu$m, for example 2-3 $\mu$m.

In the present disclosure, when the active pharmaceutical ingredient is the active pharmaceutical ingredient acting on the reproductive system, the amount of the active pharmaceutical ingredient is preferably 5-500 parts, such as 10 parts, 20 parts, 50 parts, 80 parts or 100 parts.

In the present disclosure, when the active pharmaceutical ingredient is the active pharmaceutical ingredient acting on the reproductive system, the amount of the hydrogen-containing silicone oil may be 0.3-20 parts, preferably 0.3-5 parts, such as 0.3 part, 0.5 part, 0.667 part or 1 part.

In the present disclosure, when the active pharmaceutical ingredient is the active pharmaceutical ingredient acting on the reproductive system, the amount of the catalyst is preferably 0.10-0.30 part, such as 0.10 part, 0.20 part, 0.21 part, 0.25 part or 0.30 part, more preferably 0.20-0.25 part.

In the present disclosure, when the active pharmaceutical ingredient is the active pharmaceutical ingredient acting on the reproductive system, the vinyl content in the R- vinyl silicone rubber may be 0.10%-0.50 mol%, preferably 0.10%-0.30 mol%, such as 0.18 mol%.

In the present disclosure, when the active pharmaceutical ingredient is the active pharmaceutical ingredient acting on the reproductive system, the content of Si-H groups in the hydrogen-containing silicone oil may be 0.15- 1.6 mol%, preferably 0.15- 1.2 mol%, such as 0.75 mol%.

In the present disclosure, when the active pharmaceutical ingredient is the active pharmaceutical ingredient acting on the reproductive system, the molar ratio of Si-H groups in the hydrogen-containing silicone oil and vinyl in the R-vinyl silicone rubber is preferably (1.5-10):1, such as 1.5:1, 3.0:1 or 4.5:1, more preferably (3.0-4.5): 1.

In the present disclosure, the pharmaceutical composition preferably comprises the following raw material components:

10-500 parts of gestodene;
100 parts of R-vinyl silicone rubber;
0.3-20 parts of hydrogen-containing silicone oil;
0.1-0.3 part of catalyst;
wherein:

in the R-vinyl silicone rubber, R is a substituted or unsubstituted $C_1$-$C_5$ linear alkane or branched alkane, or a substituted or unsubstituted $C_6$-$C_{20}$ aromatic hydrocarbon;
the content of vinyl in the R-vinyl silicone rubber is 0.10%-0.50 mol%;
the content of Si-H groups in the hydrogen-containing silicone oil is 0.15-1.6 mol%;
the molar ratio of Si-H groups in the hydrogen-containing silicone oil to vinyl in the R-vinyl silicone rubber is (1.5-6):1.

In the present disclosure, the pharmaceutical composition, more preferably, comprises the following raw material components:

20-200 parts of gestodene;
100 parts of R-vinyl silicone rubber;
0.3-20 parts of hydrogen-containing silicone oil;
0.2-0.25 part of catalyst;
wherein:

in the R-vinyl silicone rubber, R is a substituted or unsubstituted $C_1$-$C_5$ linear alkane or branched alkane, or a substituted or unsubstituted $C_6$-$C_{20}$ aromatic hydrocarbon;
the content of vinyl in the R-vinyl silicone rubber is 0.10%-0.50 mol%;
the content of Si-H groups in the hydrogen-containing silicone oil is 0.15-1.6 mol%;
the molar ratio of Si-H groups in the hydrogen-containing silicone oil to vinyl in the R-vinyl silicone rubber is (2-6):1.

In a preferred embodiment, the pharmaceutical composition consists of the following raw material components: 100 parts of gestodene, 100 parts of methyl vinyl silicone rubber, hydrogen-containing silicone oil, 0.21 part of platinum catalyst, 0.98 part of 2- methyl -3-butyne -2-ol;
Wherein, the content of vinyl in the methyl vinyl silicone rubber is 0.18 mol%; the content of Si-H groups in the hydrogen-containing silicone oil is 0.75 mol%; the molar ratio of Si-H groups in the hydrogen-containing silicone oil to vinyl in the methyl vinyl silicone rubber is 4.5:1.

In a more preferred embodiment, the pharmaceutical composition consists of the follow raw material components: 100 parts of gestodene, 100 parts of methyl vinyl silicone rubber, hydrogen-containing silicone oil, 0.21 part of platinum catalyst, 0.98 part of 2- methyl -3- butyne -2-ol;

Wherein, the content of vinyl in the methyl vinyl silicone rubber is 0.18 mol%, the content of Si-H groups in the hydrogen-containing silicone oil is 0.75 mol%, the molar ratio of Si-H groups in the hydrogen-containing silicone oil to vinyl in the methyl vinyl silicone rubber is 4.5:1, the concentration of platinum in the platinum catalyst is $1\times10^4$ ppm.

In a preferred embodiment, the pharmaceutical composition consists of the following raw material components: 20 parts of gestodene, 100 parts of methyl vinyl silicone rubber, hydrogen-containing silicone oil, 0.21 part of platinum catalyst, 0.98 part of 2- methyl -3- butyn -2-ol;

Wherein, the content of vinyl in the methyl vinyl silicone rubber is 0.18 mol%; the content of Si-H groups in the hydrogen-containing silicone oil is 0.75 mol%; the molar ratio of Si-H groups in the hydrogen-containing silicone oil to vinyl in the methyl vinyl silicone rubber is 4.5:1.

In a more preferred embodiment, the pharmaceutical composition consists of the follow raw material components: 20 parts of gestodene, 100 parts of methyl vinyl silicone rub, hydrogen-containing silicone oil, 0.21 part of platinum catalyst, 0.98 part of 2- methyl -3-butyne -2-ol;

Wherein, the content of vinyl in the methyl vinyl silicone rubber is 0.18mol%, the content of Si-H groups in the hydrogen-containing silicone oil is 0.75 mol%, the molar ratio of Si-H groups in the hydrogen-containing silicone oil to vinyl in the methyl vinyl silicone rubber is 4.5:1, the concentration of platinum in the platinum catalyst is $1\times10^4$ ppm.

In the present disclosure, the pharmaceutical composition preferably comprises the following raw material components:

5-200 parts of levonorgestrel;
100 parts of R-vinyl silicone rubber;
0.3-20 parts of hydrogen-containing silicone oil;
0.2-0.3 part of catalyst;
wherein:

in the R-vinyl silicone rubber, R is a substituted or unsubstituted $C_1$-$C_5$ linear alkane or branched alkane, or a substituted or unsubstituted $C_6$-$C_{20}$ aromatic hydrocarbon;
the content of vinyl in the R-vinyl silicone rubber is 0.10%-0.50 mol%;
the content of Si-H groups in the hydrogen-containing silicone oil is 0.15-1.6 mol%;
the molar ratio of Si-H groups in the hydrogen-containing silicone oil to vinyl in the R-vinyl silicone rubber is (1.5-4.5):1.

In a specific embodiment, the pharmaceutical composition consists of the following raw material components: 10 parts of levonorgestrel, 100 parts of methyl vinyl silicone rubber, 0.667 part of hydrogen-containing silicone oil, 0.25 part of platinum catalyst, 0.49 part of 2-methyl -3- butyn -2-ol;

Wherein, the content of vinyl in the methyl vinyl silicone rubber is 0.18 mol%; the content of Si-H groups in the hydrogen-containing silicone oil is 0.75 mol%; the molar ratio of Si-H groups in the hydrogen-containing silicone oil to vinyl in the methyl vinyl silicone rubber is 1.5:1.

In a specific embodiment, the pharmaceutical composition consists of the following raw material components: 50 parts of levonorgestrel, 100 parts of methyl vinyl silicone rubber, 0.667 part of hydrogen-containing silicone oil, 0.25 part of platinum catalyst, 0.49 part of 2-methyl -3- butyn -2-ol;

Wherein, the content of vinyl in the methyl vinyl silicone rubber is 0.18 mol%; the content of Si-H groups in the hydrogen-containing silicone oil is 0.75 mol%; the molar ratio of Si-H groups in the hydrogen-containing silicone oil to vinyl in the methyl vinyl silicone rubber is 1.5:1.

In the present disclosure, when the active pharmaceutical ingredient is the active pharmaceutical ingredient acting on the endocrine system, the particle size of the active pharmaceutical ingredient may be 0.001-180 μm, such as 0.01 μm, 0.1 μm, 0.5 μm, 1 μm, 5 μm, 20 μm, 60 μm, 80 μm or 100 μm.

In the present disclosure, when the active pharmaceutical ingredient is the active pharmaceutical ingredient acting on the endocrine system, the amount of the active pharmaceutical ingredient is preferably 10-500 parts, such as 100 parts, 150 parts or 200 parts.

In the present disclosure, when the active pharmaceutical ingredient is the active pharmaceutical ingredient acting on the endocrine system, the amount of the hydrogen-containing silicone oil may be 0.3-20 parts, preferably 0.3-5 parts, such as 0.3 part, 0.5 part or 1 part.

In the present disclosure, when the active pharmaceutical ingredient is the active pharmaceutical ingredient acting on the endocrine system, the amount of the catalyst is preferably 0.20-0.30 part, such as 0.20 part, 0.25 part, 0.27 part or 0.30 part, more preferably 0.20-0.27 part.

In the present disclosure, when the active pharmaceutical ingredient is the active pharmaceutical ingredient acting on the endocrine system, the content of vinyl in the R- vinyl silicone rubber is 0.10%-0.30 mol%, such as 0.17 mol%.

In the present disclosure, when the active pharmaceutical ingredient is the active pharmaceutical ingredient

acting on the endocrine system, the content of Si-H groups in the hydrogen-containing silicone oil is 0.15- 1.2 mol%, such as 0.75 mol%.

In the present disclosure, when the active pharmaceutical ingredient is the active pharmaceutical ingredient acting on the endocrine system, the molar ratio of Si-H groups in the hydrogen-containing silicone oil and vinyl in the R-vinyl silicone rubber is preferably (1-6):1, preferably (2-6): 1, such as 2:1, 3:1, 3.5:1 or 4:1, more preferably (3.0-3.5): 1.

In the present disclosure, the pharmaceutical composition preferably comprises the following raw material components:

20-500 parts of levothyroxine sodium;
100 parts of R-vinyl silicone rubber;
0.3-20 parts of hydrogen-containing silicone oil;
0.2-0.3 part of catalyst;
wherein:

in the R-vinyl silicone rubber, R is a substituted or unsubstituted $C_1$-$C_5$ linear alkane or branched alkane, or a substituted or unsubstituted $C_6$-$C_{20}$ aromatic hydrocarbon;
the content of vinyl in the R-vinyl silicone rubber is 0.10%-0.30 mol%;
the content of Si-H groups in the hydrogen-containing silicone oil is 0.15-1.2 mol%;
the molar ratio of Si-H groups in the hydrogen-containing silicone oil to vinyl in the R-vinyl silicone rubber is (1-6):1.

In a preferred embodiment, the pharmaceutical composition consists of the follow raw material components: 100 parts of levothyroxine sodium, 100 parts of methyl vinyl silicone rubber, hydrogen-containing silicone oil, 0.27 part of platinum catalyst, 0.49 part of 2- methyl -3- butyne -2-ol;
Wherein, the content of vinyl in the methyl vinyl silicone rubber is 0.17 mol%; the content of Si-H groups in the hydrogen-containing silicone oil is 0.75 mol%; the molar ratio of Si-H groups in the hydrogen-containing silicone oil to vinyl in the methyl vinyl silicone rubber is 3:1.

In a more preferred embodiment, the pharmaceutical composition consists of the follow raw material components: 100 parts of levothyroxine sodium, 100 parts of methyl vinyl silicone rubber, hydrogen-containing silicone oil, 0.27 part of platinum catalyst, 0.49 part of 2-methyl -3- butyne -2-ol;
Wherein, the content of vinyl in the methyl vinyl silicone rubber is 0.17mol%, the content of Si-H groups in the hydrogen-containing silicone oil is 0.75 mol%, the molar ratio of Si-H groups in the hydrogen-containing silicone oil to vinyl in the methyl vinyl silicone rubber is 3:1, the concentration of platinum in the platinum catalyst is $1\times10^4$ ppm.

In a preferred embodiment, the pharmaceutical composition consists of the follow raw material components: 100 parts of levothyroxine sodium, 100 parts of methyl vinyl silicone rubber, hydrogen-containing silicone oil, 0.27 part of platinum catalyst, 0.49 part of 2- methyl -3- butyne -2-ol;
Wherein, the content of vinyl in the methyl vinyl silicone rubber is 0.17 mol%; the content of Si-H groups in the hydrogen-containing silicone oil is 0.75 mol%; the molar ratio of Si-H groups in the hydrogen-containing silicone oil to vinyl in the methyl vinyl silicone rubber is 3.5:1.

In a more preferred embodiment, the pharmaceutical composition consists of the follow raw material components: 100 parts of levothyroxine sodium, 100 parts of methyl vinyl silicone rubber, hydrogen-containing silicone oil, 0.27 part of platinum catalyst, 0.49 part of 2-methyl -3- butyne -2-ol;
Wherein, the content of vinyl in the methyl vinyl silicone rubber is 0.17mol%, the content of Si-H groups in the hydrogen-containing silicone oil is 0.75 mol%, the molar ratio of Si-H groups in the hydrogen-containing silicone oil to vinyl in the methyl vinyl silicone rubber is 3.5:1, the concentration of platinum in the platinum catalyst is $1\times10^4$ ppm.

**The present disclosure further provides a rod core**, the raw material components of which comprise the pharmaceutical composition as mentioned above. The active pharmaceutical ingredient is dispersed in the cross-linked structure of the rod core. The existing form of the active pharmaceutical ingredient in the rod core can be molecular, amorphous or crystalline. The rod core can be used alone or in combination with silicone tube. When the rod core is used in combination with the silicone tube, an appropriate formula can be selected according to the actual situation.

**The present disclosure further provides a preparation method for the rod core as mentioned above,** which comprises the following steps:

S1: dividing the R-vinyl silicone rubber into component A1 and component A2; mixing component A1 with the catalyst to obtain component B1, mixing component A2 with the hydrogen-containing silicone oil to obtain component B2;
S2: compounding the component B1 and the component B2 to obtain a compounded rubber;
S3: compounding the compounded rubber and the active pharmaceutical ingredient to obtain a drug-loaded silicone;
S4: subjecting the drug-loaded silicone to extrusion molding and vulcanization to obtain the rod core.

In S1, when the pharmaceutical composition further comprises an inhibitor, mixing the component A2 with the hydrogen-containing silicone oil and the inhibitor to obtain a component B2.

In S1, the R-vinyl silicone rubber may be pretreated using conventional methods in the art, such as drying at 30-60°C (e.g., 40°C) for 1-24 h (e.g., 12 h) for later use.

In S1, the preparation method of the component B1 or the component B2 may be conventional in the art. Generally, the components are uniformly mixed in an open mill, then performed thin-pass process for 4-6 times (e.g., 5 times), and then discharged in the form of sheets.

In S2, the step of compounding the component B1 and the component B2 may comprise the following steps: the component B1 and the component B2 are fed into an open mill according to the weight ratio of 1:1 for compounding and thin-pass process for 4-6 times (e.g., 5 times) so that the component B1 and the component B2 are sufficiently and uniformly mixed to form a compounded rubber.

In S3, the compounding may comprise the following steps: the active pharmaceutical ingredient and the compounded rubber are fed into an open mill for compounding according to the weight ratio of 1:(0.1-10) (e.g., 1:1 or 1:5), so that the compounded rubber and the active pharmaceutical ingredient are sufficiently and uniformly mixed to form a drug-loaded silicone. The drug-loaded silicone can be cut into strips.

In S4, the extrusion is generally carried out in an extruder.

In S4, the vulcanization is generally carried out in an oven. The vulcanization time may be 0.5-24h, such as 0.5h, 1h, 2h, 3h, 4h, 6h, 8h, 12h or 24h. The vulcanization temperature may be 50-160°C, such as 50°C, 70°C, 90°C or 110°C.

In S4, when the active pharmaceutical ingredient is gestodene, the vulcanization time is preferably 1-24h, such as 1h, 2h, 3h, 4h, 6h, 8h, 12h or 24h, and more preferably 1-3h. The vulcanization temperature is preferably 70-110°C, such as 70°C, 90°C or 110°C.

In a preferred embodiment, in S4, the active pharmaceutical ingredient is gestodene, the vulcanization temperature is 90°C, and the vulcanization time is 1h.

In a preferred embodiment, in S4, the active pharmaceutical ingredient is levothyroxine sodium, the vulcanization temperature is 50°C, and the vulcanization time is 0.5h.

In S4, when the active pharmaceutical ingredient is levonorgestrel, the vulcanization time is preferably 0.5-2h, such as 0.5h, 0.6h, 0.8h, 1h or 2h, and more preferably 0.5-1.5h. The vulcanization temperature is preferably 70-110°C, such as 70°C, 90°C or 110°C, and more preferably 90-110°C.

In a preferred embodiment, in S4, the active pharmaceutical ingredient is levonorgestrel, the vulcanization temperature is 110°C, and the vulcanization time is 1h.

**The present disclosure further provides a rod core**, which is prepared by the method described above.

In the present disclosure, the diameter of the rod core is preferably 2.0-10mm, such as 2.0mm, 2.4mm, 2.6mm, 3mm or 4 mm.

The length of the rod core is preferably 5-100 mm, such as 5mm, 10mm, 15mm, 20mm, 25mm, 30mm, 35mm, 40mm or 50mm.

The preferred drug release area of the rod core is 25-450 $mm^2$, such as 31.41 $mm^2$, 37.69 $mm^2$, 62.8 $mm^2$, 69.08 $mm^2$, 94.23 $mm^2$, 94.24 $mm^2$, 100.48 $mm^2$, 125.6 $mm^2$, 131.88 $mm^2$, 157.07 $mm^2$, 163.38 $mm^2$, 188.4$mm^2$, 194.68 $mm^2$, 202.53 $mm^2$, 214.71 $mm^2$, 219.91 $mm^2$, 226.28 $mm^2$, 251.2 $mm^2$, 257.48 $mm^2$, 296.73 $mm^2$, 310.48 $mm^2$, 314 $mm^2$, 320.28 $mm^2$, 390.93 $mm^2$ or 418.81 $mm^2$.

**The present disclosure further provides an implant,** which comprises the rod core and a silicone tube.

In the present disclosure, the silicone tube may be conventional in the art. Preferably, the raw material composition of the silicone tube comprises the following components in parts by weight:

100 parts of R-vinyl silicone rubber;
0.3-4.0 parts of hydrogen-containing silicone oil;
0.000002-0.5 part of catalyst;
20-80 parts of reinforcing agent;
wherein, in the R-vinyl silicone rubber, R is a substituted or unsubstituted $C_1$-$C_5$ linear alkane or branched alkane, or a substituted or unsubstituted $C_6$-$C_{20}$ aromatic hydrocarbon;
the content of vinyl in the R-vinyl silicone rubber is 0.10%-0.50 mol%;
the content of Si-H groups in the hydrogen-containing silicone oil is 0.18-1.6 mol%;
the molar ratio of Si-H groups in the hydrogen-containing silicone oil to vinyl in the R-vinyl silicone rubber is (0.5-5):1.

Wherein, the raw material composition may preferably further include an inhibitor; the inhibitor is an inhibitor capable of inhibiting the addition reaction between the R-vinyl silicone rubber and the hydrogen-containing silicone oil. The inhibitor may be an acetylenic alcohol compound, a nitrogen-containing compound, or an organic peroxide, for example, methylbutynol, for another example, 2-methyl-3-butyn-2-ol. The amount of the inhibitor may be 0.03-2.0 parts, preferably 0.3-1.0 part, for another example, 0.3 part, 0.5 part, 0.69 part, 0.7 part, 0.74 part or 0.9 part.

Wherein, the amount of the hydrogen-containing silicone oil is preferably 0.4-2.8 parts, such as 0.42 part, 0.533 part, 0.667 part, 0.67 part, 0.84 part, 1.01 parts, 1.13 parts, 1.26 parts, 1.36 parts, 1.51 parts, 1.68 parts or 1.8 parts, more preferably 0.84-1.51 parts.

Wherein, the amount of the catalyst is preferably 0.000002-0.1 part, such as 0.01 part.

Wherein, the catalyst may be a conventional catalyst in the art capable of catalyzing the addition reaction between R-vinyl silicone rubber and hydrogen-containing silicone oil, such as a platinum catalyst, a rhodium catalyst, a palladium catalyst, or a platinum-containing catalyst, preferably a platinum-containing catalyst. When the catalyst is a platinum catalyst, the concentration of platinum in the platinum catalyst may be 3000 ppm, meaning that the mass concentration of platinum in the platinum catalyst is 3000 parts per million.

wherein, the content of vinyl in the R-vinyl silicone rubber is 0.18-0.23 mol%, such as 0.18 mol%.

Wherein, the content of Si-H groups in the hydrogen-containing silicone oil is preferably 0.36-1.6 mol%, such as 0.36 mol%, 0.5 mol%, 0.75 mol%, 1.0 mol% or 1.6 mol%.

Wherein, the molar ratio of Si-H groups in the hydrogen-containing silicone oil to vinyl in the R-vinyl silicone rubber is (0.8-4):1, such as 0.8:1, 1:1, 1.2:1, 1.5:1, 1.8:1 or 2:1, preferably (1.2-1.8):1 or (1.2-1.5):1;

Wherein, the amount of the reinforcing agent is preferably 20-80 parts, more preferably, 30-60 parts, such as 30 parts, 35 parts, 40 parts, 45 parts, 50 parts, or 60 parts.

Wherein, the reinforcing agent may be a conventional white carbon black in the art, such as, one or more of white carbon black, diatomite, quartz powder, silica powder, calcium carbonate, aluminum hydroxide, magnesium oxide, titanium white powder, magnesium silicate, carbon black, zinc oxide, iron oxide, titanium dioxide, zirconium silicate, and calcium carbonate, such as white carbon black. The white carbon black is preferably fumed white carbon black, precipitated white carbon black, gel white carbon black, or surface-treated white carbon black, for example, fumed white carbon black.

In the present disclosure, preferably, the raw material composition of the silicone material comprises the following components in parts by weight:

100 parts of methyl vinyl silicone rubber;
30-45 parts of reinforcing agent;
0.42-2.52 parts of hydrogen-containing silicone oil;
0.000002-0.015 part of catalyst;
0.3-0.9 part of inhibitor;
wherein, the content of vinyl in the methyl vinyl silicone rubber is 0.17-0.23 mol%;
the content of Si-H groups in the hydrogen-containing silicone oil is 0.5-1.0 mol%;
the molar ratio of Si-H groups in the hydrogen-containing silicone oil to vinyl in the methyl vinyl silicone rubber is (1.2-1.8):1.

In a specific embodiment, the raw material composition of the silicone material comprises the following components in parts by weight:

100 parts of methyl vinyl silicone rubber;
40 parts of reinforcing agent;
0.667 part of hydrogen-containing silicone oil;

a) part of platinum catalyst;

0.69 part of inhibitor;
wherein: the content of vinyl in the methyl vinyl silicone rubber is 0.18 mol%;
the content of Si-H groups in the hydrogen-containing silicone oil is 0.75 mol%;
the molar ratio of Si-H groups in the hydrogen-containing silicone oil to vinyl in the methyl vinyl silicone rubber is 1.5:1;

In the platinum catalyst, the concentration of platinum is 3000 ppm.

In a specific embodiment, the raw material composition of the silicone material comprises the following components in parts by weight:

100 parts of methyl vinyl silicone rubber;
50 parts of reinforcing agent;
0.533 part of hydrogen-containing silicone oil;
b) part of platinum catalyst;
0.74 part of inhibitor;

wherein: the content of vinyl in the methyl vinyl silicone rubber is 0.18 mol%;
the content of Si-H groups in the hydrogen-containing silicone oil is 0.75 mol%;
the molar ratio of Si-H groups in the hydrogen-containing silicone oil to vinyl in the methyl vinyl silicone rubber is 1.2:1;

In the platinum catalyst, the concentration of platinum is 3000ppm.
In the present disclosure, the preparation method of the silicone tube can adopt method I or method II;

method I: when the raw material composition of the silicone tube does not comprise an inhibitor, the preparation method of the silicone tube comprises the following steps:

S1: mixing the R-vinyl silicone rubber and the reinforcing agent uniformly to obtain a mixture A;
S2: mixing the mixture A and the hydrogen-containing silicone oil in the presence of the catalyst to obtain a mixture B;
S3: The mixture B is formed into a tubular shape by an extrusion process, followed by catalytic addition;

method II: when the raw material composition of the silicone tube comprises an inhibitor, the preparation method of the silicone tube comprises the following steps:

S1: mixing the R-vinyl silicone rubber and the reinforcing agent uniformly to obtain a mixture A;
S2: dividing the mixture A into a component A1 and a component A2, mixing the component A1 with the catalyst to obtain a component B1, and mixing the component A2 with the hydrogen-containing silicone oil and the inhibitor to obtain a component B2;
S3: mixing the component B1 and the component B2 to obtain a mixture B;
S4: the mixture B is formed into a tubular shape by an extrusion process, followed by catalytic addition.

In the present disclosure, in the method I and method II, the reinforcing agent may be pretreated using conventional methods in the art, for example, drying at 100-210°C for 1-24 hours for later use, for another example, drying at 130 °C for 18 hours for later use.
In the present disclosure, in the method I and method II, the R-vinyl silicone rubber may be pretreated using conventional methods in the art, for example, drying at 30-60°C for 1-24 hours for later use, for another example, drying at 40 °C for 18 hours for later use.
In the present disclosure, in the method I and method II, the steps of mixing in the S1 can be: wrapping the R-vinyl silicone rubber with the reinforcing agent, then performing extruding and thin-pass process through an open mill, and sheeting;
In the present disclosure, in the method I and method II, the steps of mixing in the S1 are preferably: kneading the R-vinyl silicone rubber and the reinforcing agent in a kneader at 30°C for 30 minutes, then removing them; performing triangular wrapping and thin-pass process through an open mill at a roll spacing of 1-10 mm (e.g., 1mm), rolling, sheeting, then being left for 18 hours to obtain the mixture A.
In the present disclosure, in the method I and method II, the mixture A may be left in a desiccator at room temperature for 18-72 hours.
In the present disclosure, in the method I, prior to the catalytic addition, the mixture B can be subjected to the following post-treatment: performing triangular wrapping and thin-pass process through an open mill 4-6 times, then uniformly cutting and sheeting.
In the present disclosure, in the method II, the steps of mixing the component B1 and the component B2 may be as follows: the component B1 and the component B2 are fed into an open mill according to the weight ratio of 1:1, then performing triangular wrapping and thin-pass process through the open mill 4-6 times, and uniformly cutting and sheeting.
In the present disclosure, in the method I, the step of the catalytic addition in S3 can be: the mixture B is subjected to the first vulcanization treatment and the second vulcanization treatment in turn.
Wherein, the temperature of the first vulcanization treatment may be 250-330°C, such as 270°C, 300°C, or 330°C.
Wherein, the time of the first vulcanization treatment may be 2- 30s, such as 15s.
Wherein, the temperature of vulcanization treatment may be 150-280°C, such as 150°C, 180°C, 210°C or 240°C.
Wherein, the time of the second vulcanization treatment may be 0.1-5min, such as 2min.
In the present disclosure, in the method II, the step of the catalytic addition in S4 may be as mentioned above.
In the present disclosure, according to the conventional practice in the art, the inner diameter of the silicone tube is generally equal to the outer diameter of the rod core. The outer diameter of the silicone tube is preferably 2.0-5.0 mm, such as 2.4 mm or 2.6 mm.
The length of the silicone tube is preferably 0.9-5 cm, such as 0.9cm, 1cm, 1.9cm, 2cm, 2.9cm, 3cm, 3.9cm, 4cm,

4.4cm or 5cm.

The wall thickness of the silicone tube is preferably 0.2-0.5 mm, such as 0.2 mm, 0.3 mm, 0.4 mm, or 0.5 mm.

In a specific embodiment, the outer diameter of the silicone tube is 2.4 mm, the length of the silicone tube is 0.9 cm, and the wall thickness of the silicone tube is 0.2 mm.

**The present disclosure provides a preparation method of an implant, which comprises the following steps:** filling the rod core into the silicone tube.

**The present disclosure further provides an implant prepared by the preparation method as described above.**

**The present disclosure further provides a use of the rod core as a release rate-modulating medium in sustained and controlled release formulation.**

The implant may usually be divided into matrix-type implant and reservoir-type implant. The matrix-type implant refers to a pharmaceutical preparation which has certain mechanical properties and can be implanted under the skin of human body after mixing drugs with carriers under the conditions of catalyst, crosslinking agent and high temperature by extrusion or molding. The reservoir-type implant is generally a pharmaceutical preparation prepared by filling raw materials of drug directly or by encapsulating the matrix-type implant into a silicone tube.

In practical use, there are many drugs whose daily release need to reach more than milligram level to take effect (such as paliperidone, risperidone, ketotifen fumarate, celecoxib, meloxicam, etc.). Since the drug needs to diffuse into the body through the silicone tube, the daily release amount of the reservoir-type implant is generally only several tens of micrograms, making it difficult to meet the release requirements of high-dose drugs; the matrix-type implant has the advantages of a fast drug release rate and a large release amount; their daily drug release amount is approximately several to dozens of times that of the reservoir-type implant, however, their drug release is unstable, and the release rate tends to decrease gradually, resulting in unstable plasma drug concentrations and even failure to produce a therapeutic effect.

In the prior art, a method of adding a pH regulator and punching a silicone tube is usually adopted to improve the drug release rate, but the improvement of the drug release rate is not obvious, and it is difficult to achieve a daily milligram-level drug release rate.

In view of the defects in the prior art that the reservoir-type implant has a small drug release amount and high requirements for drug molecular weight and solubility, and the matrix-type implant exhibits unstable drug release, the present disclosure adopts active pharmaceutical ingredients with an average particle size of 1-1000 nm, which can significantly improve drug solubility; and they have better compatibility with silicone rubber, and the resulting matrix-type implant and the reservoir-type implant show significantly increased drug release rate and stable drug release behavior; at the same time, they have a wide use range, which are suitable for various drugs, and achieve stable release at a high dose.

The present disclosure solves the aforementioned technical problem through the following technical solutions.

In the process of research and development, the applicant creatively found that the average particle size of the active pharmaceutical ingredients of ordinary drugs is 2-180 μm, and the release rate of the active pharmaceutical ingredients in the release medium can be significantly improved whether it is the matrix-type implant or the reservoir-type implant; at the same time, the compatibility between the active pharmaceutical ingredients and silicone rubber is further improved after nanonization, and more kinds of drugs are applicable.

**The present disclosure provides a raw material composition of a drug-loaded rod core**, which comprises the following components in parts by weight:

1-1000 parts of active pharmaceutical ingredient;
100 parts of R-vinyl silicone rubber;
0.45-20 parts of hydrogen-containing silicone oil;
≥ 0.000002 part of catalyst, preferably 0.000002-1 part of catalyst;
wherein:

The average particle size of the active pharmaceutical ingredients is 1-1000 nm;
In the R-vinyl silicone rubber, R is a substituted or unsubstituted $C_1$-$C_5$ linear alkane or branched alkane, or a substituted or unsubstituted $C_6$-$C_{20}$ aromatic hydrocarbon;
the content of vinyl in the R-vinyl silicone rubber is 0.1-0.5 mol%;
the content of Si-H groups in the hydrogen-containing silicone oil is 0.18-1.6 mol%;
the molar ratio of Si-H groups in the hydrogen-containing silicone oil to vinyl in the R-vinyl silicone rubber is (0.5-20):1;
optionally, the raw material composition further comprises an inhibitor, and the inhibitor is an inhibitor capable of inhibiting the addition reaction between the R-vinyl silicone rubber and the hydrogen-containing silicone oil.

In the present disclosure, the active pharmaceutical ingredient may comprise the active pharmaceutical ingredient acting on the reproductive system, the active pharmaceutical ingredient acting on the urinary system, or,

the active pharmaceutical ingredient acting on chronic diseases related to metabolism and nutrition, or, the active pharmaceutical ingredient for treating chronic diseases related to connective tissue and rheumatism, or, the active pharmaceutical ingredient for treating hyperlipidemia, tumors, neuropsychiatric disorders, chronic dental diseases (dental caries, periodontal diseases), simple obesity, chronic low back pain, or leukemia, or, the active pharmaceutical ingredient acting on the circulatory system, or, the active pharmaceutical ingredient acting on the respiratory system, or, the active pharmaceutical ingredient acting on the digestive system, or, the active pharmaceutical ingredient acting on the blood system, or, the active pharmaceutical ingredient acting on the endocrine system.

Wherein, the active pharmaceutical ingredient acting on the reproductive system may include a contraceptive active pharmaceutical ingredient or steroidal estrogen.

The contraceptive active pharmaceutical ingredient may be conventional in the art, preferably including levonorgestrel, gestodene, or gestrinone.

The steroidal estrogen is preferably estradiol.

Wherein, the active pharmaceutical ingredient acting on the urinary system may be as mentioned above.

Wherein, the active pharmaceutical ingredient acting on chronic diseases related to metabolism and nutrition may be as mentioned above.

Wherein, the active pharmaceutical ingredient for treating chronic diseases related to connective tissue and rheumatism may be as mentioned above.

Wherein, the active pharmaceutical ingredient for treating neuropsychiatric disorders may be as mentioned above.

Wherein, the drug for treating hyperlipidemia may be as mentioned above.

Wherein, the anti-tumor active pharmaceutical ingredient may be as mentioned above.

Wherein, the active pharmaceutical ingredient for treating chronic dental diseases (such as dental caries and periodontal diseases) may be as mentioned above.

Wherein, the active pharmaceutical ingredient for treating simple obesity may be as mentioned above.

Wherein, the active pharmaceutical ingredient for treating chronic low back pain may be as mentioned above.

Wherein, the active pharmaceutical ingredient for treating leukemia may be as mentioned above

Wherein, the active pharmaceutical ingredient acting on the circulatory system may be as mentioned above.

Wherein, the active pharmaceutical ingredient acting on the respiratory system may be as mentioned above.

Wherein, the active pharmaceutical ingredient acting on the digestive system may be as mentioned above.

Wherein, the active pharmaceutical ingredient acting on the blood system may be as mentioned above.

Wherein, the active pharmaceutical ingredient acting on the endocrine system may be as mentioned above.

In the present disclosure, the active pharmaceutical ingredient is not particularly limited, and can be a conventional sustained-release pharmaceutical ingredient requiring long-term drug release, preferably an active drug with a solubility of ≤100mg/mL (using water as a solvent).

In the present disclosure, the amount of the active pharmaceutical ingredient is preferably 1-800 parts, such as 100 parts.

In the present disclosure, the average particle size of the active pharmaceutical ingredient may be 10-900nm, preferably 20nm, 40nm, 60nm, 80nm, 100nm, 120nm, 150nm, 198nm, 200nm, 208nm, 212nm, 220nm, 300nm, 308nm, 350nm, 400nm, 430nm, 500nm, 600nm, 646nm, 700nm, 800nm, 820nm or 900nm. Within the particle size range of the present disclosure, the smaller the particle size, the faster the release rate and the greater the daily release amount. If the particle size of the active pharmaceutical ingredient is too large, the release effect is equivalent to the conventional micron level; if the particle size of pharmaceutical active ingredients is too small, its surface area is too small, which is easy to agglomerate, making the stability of products poor.

In the present disclosure, in the R-vinyl silicone rubber, R may be a substituted or unsubstituted $C_1$-$C_5$ linear alkane, such as methyl.

When the R is methyl, the R-vinyl silicone rubber is methyl vinyl silicone rubber.

Wherein, the methyl vinyl silicone rubber may be a conventional methyl vinyl silicone rubber in the art, such as methyl vinyl silicone rubber with a relative molecular weight of 100000-800000 g/mol.

In the present disclosure, the content of vinyl in the R-vinyl silicone rubber is preferably 0.17-0.23mol%, such as 0.17 mol%, 0.18 mol%, 0.19 mol%, 0.20 mol%, 0.21 mol%, 0.22 mol% or 0.23 mol%.

In the present disclosure, the hydrogen-containing silicone oil may be conventional hydrogen-containing silicone oil in the art, such as hydrogen-containing silicone oil purchased from Guangdong Silicon Ye New Material Technology Co., Ltd.

In the present disclosure, the amount of the hydrogen-containing silicone oil may be 0.5-20 parts, preferably, 1.2-6.3 parts, such as 1.33 parts, 2.67 parts, 3.56 parts, or 4.44 parts.

In the present disclosure, the content of Si-H groups in the hydrogen-containing silicone oil may be 0.36-1.6 mol%, such as 0.36 mol%, 0.5 mol%, 0.75 mol%, 1.0 mol%, or 1.6 mol%, preferably 0.5-1 mol%.

In the present disclosure, the molar ratio of Si-H groups in the hydrogen-containing silicone oil to vinyl in the R-

vinyl silicone rubber is preferably (0.8-20):1, such as 0.8:1, 1.0:1, 1.2:1, 1.5:1, 1.8:1, 2.0:1, 3.0:1, 4.0:1, 4.2:1, 5.0:1, 8.0:1, 10.0:1 or 15.0:1.

In the present disclosure, the catalyst may be a conventional catalyst in the art capable of catalyzing the addition reaction between methyl vinyl silicone rubber and hydrogen-containing silicone oil, such as a rhodium catalyst, a palladium catalyst, or a platinum-containing catalyst, preferably a platinum-containing catalyst.

Wherein, the concentration of platinum in the platinum-containing catalyst is preferably $1\times10^3$-$1\times10^5$ppm, such as $1\times10^4$ppm.

In the present disclosure, the amount of the catalyst can be $2\times10^{-6}$-1 part, such as 0.000005 part, 0.00001 part, 0.00002 part, 0.00003 part, 0.1 part, 0.2 part, 0.21 part, 0.25 part, or 0.3 part. Preferably 0.03-0.3 part, more preferably 0.1-0.3 part.

In the present disclosure, the inhibitor may be a conventional inhibitor in the art capable of inhibiting the addition reaction between methyl vinyl silicone rubber and hydrogen-containing silicone oil at room temperature, for example, an acetylenic alcohol compound, a nitrogen-containing compound, or an organic peroxide, for another example, methylbutynol, for another example, 2-methyl-3-butyn-2-ol.

In the present disclosure, the amount of the inhibitor may be 0.03-2.5 parts, preferably, 0.1-2.0 parts, more preferably, 0.3-1.0 part, such as 0.3 part, 0.5 part, 0.7 part, 0.9 part, or 0.98 part.

In some preferred embodiments of the present disclosure, the raw material composition of the drug-loaded rod core may comprise the following components in parts by weight:

(1) nano active pharmaceutical ingredient, which an average particle size is 10-900 nm, and an addition amount is 100 PHR;
(2) methyl vinyl silicone rubber, which the content of vinyl is 0.18 mol%, and an addition amount is 100 PHR;
(3) hydrogen-containing silicone oil, which the content of Si-H group is 0.75 mol%, and an addition amount is 4.44 PHR;
(4) platinum catalyst, which the concentration of platinum is 10000 ppm, and an addition amount is 0.21 PHR;
(5) 2- methyl -3- butyne -2-ol, which an addition amount is 0.98 PHR;

Wherein, the molar ratio of Si-H groups in hydrogen-containing silicone oil to vinyl in R-vinyl silicone rubber is 5:1.

In some preferred embodiments of the present disclosure, the raw material composition of the drug-loaded rod core may comprise the following components in parts by weight:

(1) nano paliperidone, which an average particle size is 220nm, 430nm, 646nm or 820nm, and the addition amount is 100 PHR;
(2) methyl vinyl silicone rubber, which the content of vinyl is 0.18 mol%, and an addition amount is 100 PHR;
(3) hydrogen-containing silicone oil, which the content of Si-H group is 0.75 mol%, and an addition amount is 4.44 PHR;
(4) platinum catalyst, which the concentration of platinum is 10000ppm, and an addition amount is 0.21 PHR;
(5) 2- methyl -3- butyne -2-ol, which an addition amount is 0.98 PHR;

Wherein, the molar ratio of Si-H groups in hydrogen-containing silicone oil to vinyl in R-vinyl silicone rubber is 5:1.

**The present disclosure further provides a drug-loaded rod core**, which is prepared by the raw material composition of the drug-loaded rod core as described above.

In the present disclosure, the drug-loaded rod core may be a matrix-type drug-loaded rod core or a hollow matrix-type drug-loaded rod core. The drug-loaded rod core of the present disclosure can be independently used as the matrix-type implant mentioned in the background.

In the present disclosure, when the drug-loaded rod core is a matrix-type drug-loaded rod core, the outer diameter of the drug-loaded rod core may be 1-10mm, preferably 1-6 mm, such as 2 mm, 3.5 mm, 4 mm, 4.5 mm or 5 mm.

In the present disclosure, when the drug-loaded rod core is a hollow matrix-type drug-loaded rod core, the outer diameter of the drug-loaded rod core may be 1-10 mm, preferably 1-6 mm, such as 2 mm, 3.5 mm, 4 mm, 4.5 mm or 5 mm.

In the present disclosure, when the drug-loaded rod core is a hollow matrix-type drug-loaded rod core, the inner diameter of the drug-loaded rod core may be 0.1 mm-4.0 mm, preferably 0.4 mm-2.0 mm, such as 0.4 mm, 0.6 mm, 0.8 mm, 1.0 mm, 1.2 mm, 1.4 mm, 1.6 mm or 2.0 mm.

When the drug-loaded rod core is a hollow matrix-type drug-loaded rod core, the diameter ratio of the outer diameter to the inner diameter of the drug-loaded rod core is preferably (1.5-50):1, such as 2.14:1, 3:1, 4:1, 5:1, 8:1, 10:1, 15:1, 20:1, 25:1, 30:1, 35:1 or 40:1.

**The present disclosure further provides a preparation method for the drug-loaded rod core as mentioned above**, which comprises the following steps:

(1) dividing the R-vinyl silicone rubberinto component A1 and component B1, mixing the component A1 with the hydrogen-containing silicone oil and the inhibitor to obtain component A2, mixing the component B1 with the catalyst to obtain component B2; compounding the component A2 with the component B2 to obtain a compounded rubber;
(2) compounding the compounded rubber with the active pharmaceutical ingredient to obtain a drug-loaded silicone;
(3) subjecting the drug-loaded silicone to extrusion molding and vulcanization to obtain a drug-loaded rod core.

In step (1), the R-vinyl silicone rubber may be pretreated using conventional methods in the art, such as drying at 30-60°C (e.g., 40°C) for 1-24 h (e.g., 12 h) for later use.

In step (1), the preparation method of the component B1 or the component B2 may be conventional in the art. Generally, the components are uniformly mixed in an open mill, then performed thin-pass process for 4-6 times (e.g., 6 times), and then discharged in the form of sheets.

In step (1), the step of compounding the component A2 and the component B2 may comprise the following steps: the component A2 and the component B2 are fed into an open mill according to the weight ratio of 1:1 for compounding and thin-pass process for 4-6 times (e.g., 4 or 6 times) so that the component A2 and the component B2 are sufficiently and uniformly mixed to form a compounded rubber.

In step (2), the active pharmaceutical ingredient is prepared by nanonization, and the PDI after nanonization can be less than 0.3. The nanonization can use a conventional nanonization method in the art, such as grinding.

Wherein, the grinding can use a wet grinding method. Preferably, a stabilizer and a grinding medium are also added in the grinding process.

The grinding time may be not less than 15 min, preferably 15-250 min.

The rotational speed of the grinding may be 300-500 r•min$^{-1}$, for example, 400 r•min$^{-1}$.

The types of the stabilizer can be conventional in the art, and are preferably one or more of poloxamer 407, poloxamer 188, SDS, HPMC, Tween 80, PVP K30, PEO, TPGS and HPC, and more preferably poloxamer 188 (P188), Tween 80, poloxamer 407 (P407), SDS or HPMC, such as poloxamer 188.

The types of the grinding medium can be conventional in the art, such as zirconia beads. The particle size of the zirconia beads may be 0.1-0.3 mm, such as 0.1 mm, 0.2 mm or 0.3 mm.

After the grinding, it may further comprise freeze-drying operation.

In step (2), the step of compounding the compounded rubber and the active pharmaceutical ingredient may comprise the following steps: the compounded rubber and the active pharmaceutical ingredient are fed into an open mill for compounding, so that the compounded rubber and the active pharmaceutical ingredient are sufficiently and uniformly mixed to form a drug-loaded silicone. The drug-loaded silicone can be cut into strips.

Wherein, the mass ratio of the compounded rubber to the active pharmaceutical ingredient may be 1:(0.01-10), preferably 1:(1-5), such as 1:1.

In step (3), the drug-loaded rod core may be a matrix-type drug-loaded rod core or a hollow matrix-type drug-loaded rod core. In the present disclosure, the matrix-type drug-loaded rod core or a hollow matrix-type drug-loaded rod core may be obtained by adjusting the parameters of extrusion molding.

In step (3), the extrusion is generally carried out in a screw extruder.

In step (3), during the extrusion molding, the screw speed of the extruder can be 1-15 r min$^{-1}$, such as 4 r min$^{-1}$, 6 r min$^{-1}$ or 7 r min$^{-1}$.

In step (3), when the drug-loaded rod core is a matrix-type drug-loaded rod core, the diameter of the die orifice used in the extrusion molding may be 1-10 mm, preferably 1-6 mm, such as 2 mm, 2.96 mm, 3.5 mm, 4 mm, 4.5 mm or 5 mm.

In step (3), when the drug-loaded rod core is a matrix-type drug-loaded rod core, the diameter of the die orifice used in the extrusion molding may be 1-10 mm, preferably 1-6 mm, such as 2 mm, 2.96 mm, 3.5 mm, 4 mm, 4.5 mm or 5 mm.

In step (3), when the drug-loaded rod core is a matrix-type drug-loaded rod core, the diameter of a core mold used in the extrusion molding is preferably 0.1 mm-4.0 mm, preferably 0.4 mm-2.0 mm, such as 0.4 mm, 0.58 mm, 0.6 mm, 0.8 mm, 0.97 mm, 1.0 mm, 1.2 mm, 1.38 mm, 1.4 mm, 1.6 mm or 2.0 mm.

Preferably, when the drug-loaded rod core is a matrix-type drug-loaded rod core, during the extrusion molding, the diameter ratio of the die orifice to the core mold is (1.5-50): 1, for example, 2.14:1, 3:1, 4:1, 5:1, 8:1, 10:1, 15:1,20:1, 25:1, 30:1, 35:1 or 40:1.

In step (3), the vulcanization is generally carried out in an oven.

In step (3), the vulcanization temperature is preferably 30-120°C, such as 30°C, 50°C, 60°C, 65°C, 70°C, 80°C, 90°C, 100°C or 120°C.

In step (3), the vulcanization time is preferably 10-100min, such as 10min, 20 min, 30 min, 40 min, 50 min, 70min or 100min.

**The present disclosure further provides a reservoir-type implant,** which comprises the above drug-loaded rod core and a silicone tube.

In the present disclosure, the outer diameter of the silicone tube may be 2.0-10 mm, preferably 2.0-6.4 mm, such as 2.4 mm or 2.6 mm, 3.2 mm or 3.4 mm.

In the present disclosure, the wall thickness of the silicone tube may be preferably 0.1-1.0 mm, such as 0.2 mm, 0.3 mm, 0.4 mm, or 0.5 mm.

In the present disclosure, the length of the silicone tube may be preferably 1.5-10 cm, such as 1.9 cm or 4.4 cm.

In the present disclosure, the drug release area of the silicone tube may be 0.618-31.4cm$^2$, preferably 0.4-15.0cm$^2$, such as 0.69cm$^2$, 1.38cm$^2$, 2.07cm$^2$, 2.76cm$^2$, 3.45cm$^2$ or 3.77cm$^2$.

In the present disclosure, the diameter of the drug-loaded rod core may be preferably 1-10 mm, preferably 1-6 mm, such as 1.6mm, 2.0 mm or 3.0 mm.

In the present disclosure, the length of the drug-loaded rod core may be 1.0- 10 cm, preferably 1.0-4.0 cm, such as 1.0 cm, 1.5 cm, 2.0 cm, 3.0cm, 3.9 cm, 4.0 cm or 5.0 cm.

In the present disclosure, the raw material composition of the silicone tube may comprise the following components in parts by weight:

100 parts of R-vinyl silicone rubber;
20-80 parts of reinforcing agent;
0.3-3.0 parts of hydrogen-containing silicone oil;
$\geq$ 0.000002 part of catalyst, preferably 0.000002-0.5 part of catalyst;
wherein:

in the R-vinyl silicone rubber, R is a substituted or unsubstituted $C_1$-$C_5$ linear alkane or branched alkane, or a substituted or unsubstituted $C_6$-$C_{20}$ aromatic hydrocarbon;
the content of vinyl in the R-vinyl silicone rubber is 0.10-0.50 mol%;
the content of Si-H groups in the hydrogen-containing silicone oil is 0.18-1.6 mol%;
the molar ratio of Si-H groups in the hydrogen-containing silicone oil to vinyl in the R-vinyl silicone rubber is (0.5-20):1;
optionally, the raw material composition further comprises an inhibitor, and the inhibitor is an inhibitor capable of inhibiting the addition reaction between the R-vinyl silicone rubber and the hydrogen-containing silicone oil;
Wherein, the types and content of the R- vinyl silicone rubber may be as mentioned above: In the present disclosure, "as mentioned above" means that the selected range is the same as that mentioned above, which is independent of each other and does not affect each other.

Wherein, the reinforcing agent may be a conventional reinforcing agent in the art that can improve the hardness of the R-vinyl silicone rubber, such as one or more of white carbon black, diatomite, quartz powder, silica powder, calcium carbonate, aluminum hydroxide, magnesium oxide, titanium white powder, magnesium silicate, carbon black, zinc oxide, iron oxide, titanium dioxide, zirconium silicate, and calcium carbonate, such as white carbon black.

The white carbon black may be conventional white carbon black in the art, such as fumed white carbon black, precipitated white carbon black, gel white carbon black, or surface-treated white carbon black, preferably fumed white carbon black.

Wherein, the amount of the reinforcing agent is preferably 30-80 parts, such as 30 parts, 35 parts, 40 parts, 45 parts, 50 parts, or 60 parts.

Wherein, the types of the hydrogen-containing silicone oil, the amount of Si-H groups in the hydrogen-containing silicone oil, the molar ratio of Si-H groups in the hydrogen-containing silicone oil to vinyl in the methyl vinyl silicone rubber may be as mentioned above.

Wherein, the amount of the hydrogen-containing silicone oil is preferably 0.4-2.8 parts, such as 0.42 part, 0.67 part, 0.84 part, 1.01 parts, 1.07 parts, 1.26 parts, 1.36 parts, 1.51 parts, 1.68 parts, or 2.52 parts.

Wherein, the catalyst may be a conventional catalyst in the art capable of catalyzing the addition reaction between methyl vinyl silicone rubber and hydrogen-containing silicone oil, such as a rhodium catalyst, a palladium catalyst, or a platinum catalyst, preferably a platinum catalyst.

The concentration of platinum in the platinum catalyst may be 3000 ppm. 3000 ppm means that the mass concentration of platinum in the platinum catalyst is 3000 parts per million.

Wherein, the amount of the catalyst is preferably 0.03-0.3 part, more preferably, 0.1-0.3 part, such as 0.21 part.

Wherein, the inhibitor may be a conventional inhibitor in the art capable of inhibiting the addition reaction between methyl vinyl silicone rubber and hydrogen-containing silicone oil at room temperature, for example, an acetylenic alcohol compound, a nitrogen-containing compound, or an organic peroxide, for another example, methylbutynol, for another example, 2-methyl-3-butyn-2-ol.

Wherein, the amount of the inhibitor is as mentioned above.

In the present disclosure, the preparation method of the silicone tube comprises the following steps:

method I: when the raw material composition of the silicone material does not comprise an inhibitor, the preparation method for the silicone material comprises the following steps:

S1: mixing the R-vinyl silicone rubber and the reinforcing agent uniformly to obtain a mixture A;
S2: mixing the mixture A and the hydrogen-containing silicone oil in the presence of the catalyst to obtain a mixture B;
S3: The mixture B is formed into a tubular shape by an extrusion process, followed by catalytic addition; or, the catalytic addition process is carried out in a tubular mold to obtain the silicone tube.

method II: when the raw material composition of the silicone material further comprises an inhibitor, the preparation method for the silicone material comprises the following steps:

S1: mixing the R-vinyl silicone rubber and the reinforcing agent uniformly to obtain a mixture A;
S2: dividing the mixture A into a component A1 and a component A2, mixing the component A1 with the catalyst to obtain a component B1, and mixing the component A2 with the hydrogen-containing silicone oil and the inhibitor to obtain a component B2;
S3: mixing the component B1 and the component B2 to obtain a mixture B;
S4: The mixture B is formed into a tubular shape by an extrusion process, followed by catalytic addition; or, the catalytic addition process is carried out in a tubular mold to obtain the silicone tube.

Wherein, in the method I and method II, the reinforcing agent may be pretreated using conventional methods in the art, such as by drying at 100-210°C (e.g., 130°C) for 1-24 h (e.g., 12 h or 24 h) for later use.
Wherein, in the method I and method II, the R-vinyl silicone rubber may be pretreated using conventional methods in the art, such as by drying at 30-60°C (e.g., 40°C) for 1-24 hours (e.g., 12 hours or 24 hours) for later use.
Wherein, in the method I and method II, the step of mixing in the S1 may be: wrapping the R-vinyl silicone rubber with the reinforcing agent, then performing extruding and thin-pass process through an open mill, and sheeting.
Wherein, in the method I and method II, the mixture A may be left in a desiccator at room temperature for 24-72 hours (e.g., 24 hours) for later use.
Wherein, in the method I and method II, the step of mixing in the S1 may be: kneading the R-vinyl silicone rubber and the reinforcing agent in a kneader at 30°C for 30 minutes, then removing the mixture; performing triangular wrapping and thin-pass process through an open mill at a roll spacing of 1-10 mm (e.g., 1 mm) five times, rolling, sheeting, then being left for 24 hours to obtain the mixture A.
Wherein, in the method I, prior to the catalytic addition, the mixture B undergoes the following post-treatment: performing triangular wrapping and thin-pass process through an open mill 4-6 times (e.g., 6 times), then uniformly cutting and sheeting;
Wherein, in the method II, the step of mixing the component B1 and the component B2 may be: the component B1 and the component B2 are fed into an open mill according to the weight ratio of 1:1, then performing triangular wrapping and thin-pass process through the open mill 4-6 times (e.g., 6 times), and uniformly cutting and sheeting.
Wherein, in the method I, the step of the catalytic addition may be: subjecting the mixture B to a first heat treatment and a second heat treatment in turn; preferably, a third heat treatment is further included after the second heat treatment.
The temperature of the first heat treatment may be 250-360°C, for example, 270-300°C, for another example, 270°C, 280°C or 300°C.
The time of the first heat treatment may be about 5s.
The temperature of the second heat treatment is 120-300°C, for example, 180-280°C, for another example, 180°C, 260°C or 280°C.
The time of the second heat treatment may be about 2min.
The temperature of the third heat treatment may be 150-250°C, such as 180°C.
The time of the third heat treatment may be 24- 48h, such as 48h.
The third heat treatment may be carried out in an oven.
Wherein, in the method II, the step of the catalytic addition in S4 may be as mentioned above.
**The present disclosure provides a preparation method of the reservoir-type implant mentioned above,** which comprises the following steps: cutting the silicone tube into sections, filling the drug-loaded rod core, and sealing the two ends with silicone to obtain the reservoir-type implant.
In the present disclosure, preferably, the length of the silicone tube is 0.4 cm longer than that of the drug-loaded rod core after cutting into sections. The extra length is used for sealing, for example, 0.2cm is reserved for the sealing glue at both ends of the implant.
In the present disclosure, preferably, before the filling, the operation of cleaning and swelling the silicone tube is also included.
Wherein, the cleaning may be carried out by soaking in ethanol and repeatedly washing with ethanol. The

soaking time may be 10-60 min, for example, 15 min.

Wherein, the swelling can be achieved by soaking in petroleum ether. The soaking time may be 1-2 min. Since the swollen silicone tube rapidly shrinks back to its original shape immediately after being removed from petroleum ether, the drug-loaded rod core must be quickly inserted into the silicone tube.

In the present disclosure, a curing operation may be further included after the sealing.

Wherein, the curing may be carried out by standing at room temperature. The standing time may be 12-24 h, such as 24 h.

**The present disclosure further provides a reservoir-type implant,** which comprises a powder-type drug core and a silicone tube, the powder-type drug core comprises an active pharmaceutical ingredient, the average particle size of the active pharmaceutical ingredient is 1-1000 nm.

In the present disclosure, the size (including outer diameter, wall thickness, length and drug release area) of the silicone tube may be as mentioned above.

In the present disclosure, the diameter of the drug-loaded sections in the reservoir-type implant may be 1-10 mm, preferably 1-6 mm, such as 1.6 mm, 2.0 mm or 3.0 mm.

In the present disclosure, the length of the drug-loaded sections in the reservoir-type implant is preferably 1.0- 10 cm, more preferably 1.0-4.0 cm, such as 1.0 cm, 1.5 cm, 2.0 cm, 3.0cm, 3.9 cm, 4.0 cm or 5.0 cm.

In the present disclosure, the average particle size of the active pharmaceutical ingredient may be 10-900 nm, preferably 20 nm, 40 nm, 60 nm, 80 nm, 100 nm, 120 nm, 150 nm, 198 nm, 200 nm, 208 nm, 212 nm, 220 nm, 300 nm, 308 nm, 350 nm, 400 nm, 430 nm, 500 nm, 600 nm, 646 nm, 700 nm, 800 nm, 820 nm or 900 nm.

In the present disclosure, the powder-type drug core may further include insoluble excipient.

Wherein, the average particle size of the insoluble excipient may be 1-200 μm.

Wherein, the insoluble excipient may comprise a silicon material. The pore size of the silicon material may be less than 1 μm, such as 0 nm, 5 nm, 10 nm, 18 nm, 50 nm, or 100 nm. It can be understood by those skilled in the art that when the pore size of the silicon material is 0 nm, the silicon material is a non-porous silicon material.

In the silicon material, the content of silicon dioxide may be more than 50%, preferably 80%, 90%, 95%, 99% or 99.8%.

Wherein, the insoluble excipient may include one or more of white carbon black, AL-1FP mesoporous silica, XDP3050 mesoporous silica, and XDP3150 mesoporous silica.

The white carbon black is preferably fumed white carbon black, precipitated white carbon black, gel white carbon black, or white carbon black prepared by surface treatment.

Preferably, the insoluble excipient is one or more of white carbon black, AL-1FP mesoporous silica, XDP3050 mesoporous silica, and XDP3150 mesoporous silica.

More preferably, the insoluble excipient is one or more of white carbon black, AL-1FP mesoporous silica, and XDP3050 mesoporous silica.

In the present disclosure, the insoluble excipient may further comprise an insoluble weak acid and/or an insoluble weak base.

Wherein, the insoluble weak acid preferably includes one or more of boric acid, fumaric acid, molybdic acid, silicic acid, tungstic acid, and germanic acid, more preferably includes boric acid and/or fumaric acid.

Wherein, the insoluble weak base preferably includes one or more of magnesium hydroxide, aluminum hydroxide, zinc hydroxide, ferrous hydroxide, and magnesium oxide, more preferably includes one or more of magnesium hydroxide, aluminum hydroxide, and zinc hydroxide.

Wherein, the insoluble excipient may also be white carbon black combined with one or more of molybdic acid, silicic acid, tungstic acid, and germanic acid; for example, white carbon black and molybdic acid, white carbon black and silicic acid, white carbon black and tungstic acid, or white carbon black and germanic acid.

Alternatively, the insoluble excipient may also be white carbon black combined with ferrous hydroxide and/or magnesium oxide; for example, white carbon black and ferrous hydroxide, or white carbon black and magnesium oxide.

Alternatively, the insoluble excipient may also be AL-1FP mesoporous silica combined with one or more of molybdic acid, silicic acid, tungstic acid, and germanic acid; for example, AL-1FP mesoporous silica and molybdic acid, AL-1FP mesoporous silica and silicic acid, AL-1FP mesoporous silica and tungstic acid, or AL-1FP mesoporous silica and germanic acid.

Alternatively, the insoluble excipient may also be AL-1FP mesoporous silica combined with ferrous hydroxide and/or magnesium oxide; for example, AL-1FP mesoporous silica and ferrous hydroxide, or AL-1FP mesoporous silica and magnesium oxide.

Alternatively, the insoluble excipient may also be XDP3050 mesoporous silica combined with one or more of molybdic acid, silicic acid, tungstic acid, and germanic acid; for example, XDP3050 mesoporous silica and molybdic acid, XDP3050 mesoporous silica and silicic acid, XDP3050 mesoporous silica and tungstic acid, or XDP3050 mesoporous silica and germanic acid.

Alternatively, the insoluble excipient may also be XDP3050 mesoporous silica combined with ferrous hydroxide

and/or magnesium oxide; for example, XDP3050 mesoporous silica and ferrous hydroxide, or XDP3050 mesoporous silica and magnesium oxide.

Alternatively, the insoluble excipient may also be XDP3150 mesoporous silica combined with one or more of molybdic acid, silicic acid, tungstic acid, and germanic acid; for example, XDP3150 mesoporous silica and molybdic acid, XDP3150 mesoporous silica and silicic acid, XDP3150 mesoporous silica and tungstic acid, or XDP3150 mesoporous silica and germanic acid.

Alternatively, the insoluble excipient may also be XDP3150 mesoporous silica combined with ferrous hydroxide and/or magnesium oxide; for example, XDP3150 mesoporous silica and ferrous hydroxide, or XDP3150 mesoporous silica and magnesium oxide.

In the present disclosure, the content of the bulk drug may be 10%-99.9%, such as 50%-99.5%, or such as 95%; the percentage represents the mass percentage of the bulk drug in the pharmaceutical composition.

The content of the insoluble excipient may be 0.1%-90%, for example, 0.1%-50%, for another example, 0.5%-5%; the percentage represents the mass percentage of the insoluble excipient in the pharmaceutical composition.

In the present disclosure, when the insoluble excipient is two or three of white carbon black, AL-1FP mesoporous silica, and XDP3150 mesoporous silica, their mass ratio may be any ratio. For example, when the insoluble excipient is two of white carbon black, AL-1FP mesoporous silica, and XDP3050 mesoporous silica, their mass ratio is (0.001-1000):1.

For another example, when the insoluble excipient is XDP3150 mesoporous silica and fumaric acid, their mass ratio may be (0.01-100):1, preferably 9:1, 1.5:1, or 0.43:1.

For another example, when the insoluble excipient is fumed white carbon black and boric acid, their mass ratio may be (0.01-100):1, preferably 9:1, 1.5:1, or 0.43:1.

For another example, when the insoluble excipient is XDP3050 mesoporous silica and magnesium hydroxide, their mass ratio may be (0.01-100):1, preferably 9:1, 1.5:1, or 0.43:1.

For another example, when the insoluble excipient is AL-1FP mesoporous silica and zinc hydroxide, then the mass ratio may be (0.01-100):1, preferably 9:1, 1.5:1, or 0.43:1.

In the present disclosure, the raw material composition of the silicone tube may be as mentioned above.

**The present disclosure provides a preparation method of the reservoir-type implant mentioned above, which comprises the following steps**: cutting the silicone tube into sections, one end is sealed with silicone, the powder-type drug core is filled therein, and the other end is sealed with silicone to obtain the reservoir-type implant.

In the present disclosure, preferably, the length of the silicone tube is 0.4cm longer than that of the drug-loaded sections after cutting into sections.

In the present disclosure, preferably, before the filling, the operation of cleaning the silicone tube is also included.

Wherein, the cleaning may be carried out by soaking in ethanol and repeatedly washing with ethanol. The soaking time may be 10-60 min, for example, 15 min.

In the present disclosure, a curing operation may be further included after the sealing.

Wherein, the curing may be carried out by standing at room temperature. The standing time may be 12-24 h, such as 24 h.

**The present disclosure further provides a use of the above-mentioned raw material composition of the drug-loaded rod core, the above-mentioned drug-loaded rod core, or the above-mentioned reservoir-type implant as a release rate-regulating medium in a sustained and controlled release formulation.**

As mentioned above, implants can be divided into the matrix-type implant and the reservoir-type implant. The reservoir-type implant has the advantage of stable drug release, which can maintain zero level stable release, maintain stable blood drug concentration and achieve good therapeutic effects during the effective period (up to several years). Because drugs need to diffuse into the body through silicone tubes, the molecular weight and water solubility of drugs are very high, and only drugs with molecular weight less than 800 and solubility less than 10mg/mL can be released. Since the drug needs to diffuse into the body through the silicone tube, stringent requirements are imposed on the molecular weight and water solubility of the drug. Only drugs with a molecular weight of less than 800 and a solubility of less than 10 mg/mL can be released. This has great limitations for some high-dose drugs and cannot be applied.

The production process of the matrix-type implant is relatively simple, and it has the advantage of fast drug release. The amount of daily drug release is about several times to dozens of times as much as that of the reservoir-type implant, but its release is not stable, and the drug release rate will gradually decrease, resulting in unstable blood drug concentration and even failure to produce therapeutic effect.

In view of the defects in the prior art that the reservoir-type implant has high requirements on the molecular weight and solubility of drugs, low release dosage and cannot meet the use of high-dose drugs, as well as the defects that the matrix-type implant has unstable drug release, causes blood drug concentration fluctuation, results in poisoning phenomenon or has no therapeutic effect, the present disclosure further provides a hollow drug-loaded rod core, a preparation method and use thereof.

The present disclosure solves the aforementioned technical problem through the following technical solutions.

**The present disclosure provides a hollow matrix-type drug-loaded rod core**, the raw material composition

comprises the following components in parts by weight:

1-1000 parts of active pharmaceutical ingredient;
100 parts of R-vinyl silicone rubber;
0.45-20 parts of hydrogen-containing silicone oil;
≥ 0.000002 part of catalyst;
wherein:

in the R-vinyl silicone rubber, R is a substituted or unsubstituted $C_1$-$C_5$ linear alkane or branched alkane, or a substituted or unsubstituted $C_6$-$C_{20}$ aromatic hydrocarbon;
the content of vinyl in the R-vinyl silicone rubber is 0.1-0.5 mol%;
the content of Si-H groups in the hydrogen-containing silicone oil is 0.18-1.6 mol%;
the molar ratio of Si-H groups in the hydrogen-containing silicone oil to vinyl in the R-vinyl silicone rubber is (0.5-20):1.

In the present disclosure, the amount of the active pharmaceutical ingredient may be 1- 800, parts, such as 10 parts, 30 parts, 50 parts, 60 parts, 100 parts, 120 parts, 150 parts, 250 parts, 500 parts, or 600 parts.

In the present disclosure, in the R-vinyl silicone rubber, R may be a substituted or unsubstituted $C_1$-$C_5$ linear alkane, such as methyl.

When the R is methyl, the R-vinyl silicone rubber is methyl vinyl silicone rubber.

Herein, the methyl vinyl silicone rubber may be a conventional methyl vinyl silicone rubber in the art, such as methyl vinyl silicone rubber with a relative molecular weight of 100000-800000 g/mol.

In the present disclosure, the content of vinyl in the R-vinyl silicone rubber is preferably 0.17-0.23 mol%, such as 0.17 mol%, 0.18 mol%, 0.19mol%, 0.20mol%, 0.22mol% or 0.23mol%.

In the present disclosure, the hydrogen-containing silicone oil may be conventional hydrogen-containing silicone oil in the art, such as hydrogen-containing silicone oil purchased from Guangdong Silicon Ye New Material Technology Co., Ltd.

In the present disclosure, the amount of the hydrogen-containing silicone oil may be 0.5-20 parts, preferably, 1.2-6.3 parts, such as 1.33 parts, 2 parts, 2.67 parts, 3 parts, 3.56 parts, or 4.44 parts.

In the present disclosure, the content of Si-H groups (hydrogen amount) in the hydrogen-containing silicone oil is preferably 0.5-1.5 mol%, such as 0.75 mol%, 0.8 mol%, 1.0 mol% or 1.2 mol%.

In the present disclosure, the molar ratio of Si-H groups in the hydrogen-containing silicone oil to vinyl in the R-vinyl silicone rubber is preferably (0.8-20):1, such as 0.8:1, 1.0:1, 1.2:1, 1.5:1, 1.8:1, 2.0:1, 3.0:1, 4:1, 4.5:1, 5:1, 8:1, 10:1 or 15:1, preferably, (3-8):1 or (3-6):1.

In the present disclosure, optionally, the raw material composition further comprises an inhibitor. The inhibitor may be a conventional inhibitor in the art capable of inhibiting the addition reaction between R-vinyl silicone rubber and hydrogen-containing silicone oil at room temperature, for example, an acetylenic alcohol compound, a nitrogen-containing compound, or an organic peroxide, for another example, methylbutynol, for another example, 2-methyl-3-butyn-2-ol.

In the present disclosure, the amount of the inhibitor may be 0.000002-1 part, preferably, 0.03-5 parts, more preferably, 0.1-2.0 parts, for example, 0.3-1.0 part, for another example, 0.3 part, 0.5 part, 0.7 part, 0.9 part, or 0.98 part.

In the present disclosure, the catalyst may be a conventional catalyst in the art capable of catalyzing the addition reaction between R-vinyl silicone rubber and hydrogen-containing silicone oil, such as a rhodium catalyst, a palladium catalyst, or a platinum catalyst, preferably a platinum catalyst.

Wherein, the concentration of platinum in the platinum catalyst may be 10000 ppm. 10000 ppm means that the mass concentration of platinum in the platinum catalyst is 10000 parts per million.

In the present disclosure, the amount of the catalyst is preferably 0.000002-0.5 part, such as 0.000005 part, 0.00001 part, 0.00002 part, 0.00003 part, 0.1 part, 0.2 part, 0.21 part, 0.25 part, or 0.3 part. Preferably 0.03-0.3 part, more preferably 0.1-0.3 part.

In the present disclosure, the vulcanization principle of the two-component addition-cure silicone rubber is described as follows: Two-component addition-cure silicone rubber vulcanized at room temperature, such as using vinyl polydimethylsiloxane as the base polymer and hydrogen-containing silicone oil as the crosslinking agent, undergoes a hydrosilylation reaction between vinyl and hydrogen groups under the catalysis of a catalyst (e.g., a platinum catalyst) to form a cross-linked network structure, which can control drug release, the reaction formula is shown below.

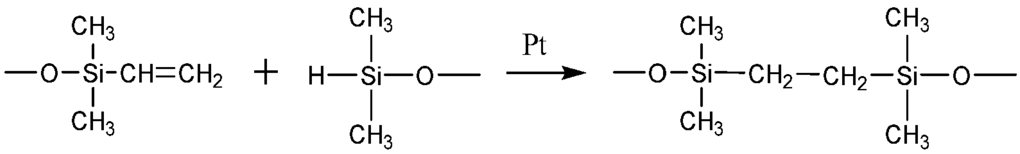


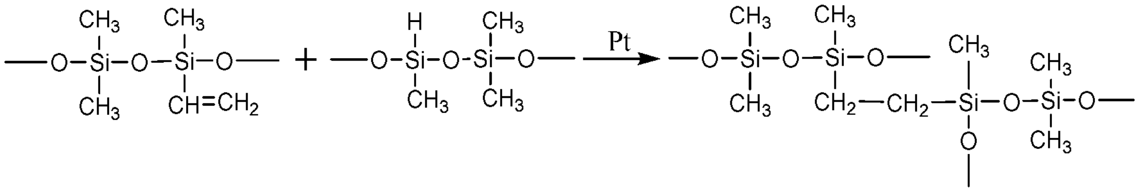


After mixing the raw rubber with crosslinking agent, and catalyst, a reaction can occur at room temperature. However, the compounding and processing of the rubber require a certain amount of time. If the reactants are prematurely cured during operation, the desired shape and properties cannot be achieved. This is especially true for addition-cure silicone rubber. Therefore, it is generally required that the catalytic reaction remains almost inactive before vulcanization (when mixed at room temperature) and reacts rapidly upon reaching the vulcanization temperature. The method of inhibiting the reaction is usually the addition of an inhibitor. The inhibitor can form a certain complex with the platinum catalyst. Effective inhibitors can be stored with the rubber for a considerable amount of time and can only be vulcanized when heated to a certain vulcanization temperature. The commonly used inhibitors with good compatibility are acetylenic alcohol compounds, nitrogen-containing compounds, organic peroxides, etc.

In the present disclosure, the active pharmaceutical ingredient is not particularly limited, and can be a conventional sustained-release pharmaceutical ingredient requiring long-term drug release, for example, drugs used in the art of contraception, schizophrenia, hormones, anti-tumor and the like.

In the present disclosure, the active pharmaceutical ingredient may be as mentioned above.

In the present disclosure, the active drug may comprise conventional drugs in the art. For example, one or more selected from the group consisting of macromolecular drugs with good solubility, micromolecule drugs with good solubility, macromolecular drugs with poor solubility and micromolecule drugs with poor solubility.

Wherein, the macromolecular drugs with good solubility may be conventional active drugs in the art with a solubility of more than 10 mg/mL (using water as the solvent) and a molecular weight of more than 800 Da, preferably active drugs with a solubility of more than 30 mg/mL (using water as the solvent) and a molecular weight of more than 800 Da, such as one or more selected from the group consisting of exenatide, semaglutide, centella asiatica total glycosides, and human growth hormone.

Wherein the micromolecule drugs with good solubility may be conventional active drugs in the art with a solubility of more than 10 mg/mL (using water as the solvent) and a molecular weight of not more than 800 Da, preferably active drugs with a solubility of more than 30 mg/mL (using water as the solvent) and a molecular weight of not more than 800 Da, such as metformin hydrochloride and/or doxorubicin hydrochloride.

Wherein, the macromolecular drugs with poor solubility may be conventional active drugs in the art with a solubility of not more than 10 mg/mL (using water as the solvent) and a molecular weight of more than 800 Da, preferably active drugs with a solubility of not more than 5 mg/mL (using water as the solvent) and a molecular weight of more than 800 Da, such as one or more selected from the group consisting of bovine insulin, paclitaxel, and levothyroxine sodium.

Wherein the micromolecule drugs with poor solubility may be conventional active drugs in the art with a solubility of not more than 10 mg/mL (using water as the solvent) and a molecular weight of not more than 800 Da, preferably an active drug with a solubility of not more than 5 mg/mL (using water as the solvent) and a molecular weight of not more than 800 Da, such as one or more selected from the group consisting of estradiol, gestodene, meloxicam, ganciclovir, puerarin and paliperidone.

In some preferred embodiments of the present disclosure, the raw material composition of the hollow matrix-type rod core may comprise the following components in parts by weight:

| Material name | Amount | | | | |
|---|---|---|---|---|---|
| | No. 1-1 | No. 1-2 | No. 1-3 | No. 1-4 | No. 1-5 |
| Paliperidone | 100 PHR | 100 PHR | 100 PHR | 100 PHR | 100 PHR |
| Content of vinyl in methyl vinyl silicone rubber | 0.18 mol% | 0.18 mol% | 0.18 mol% | 0.18 mol% | 0.18 mol% |

(continued)

| Material name | Amount | | | | |
|---|---|---|---|---|---|
| | No. 1-1 | No. 1-2 | No. 1-3 | No. 1-4 | No. 1-5 |
| Amount of methyl vinyl silicone rubber | 100 PHR | 100 PHR | 100 PHR | 100 PHR | 100 PHR |
| Amount of hydrogen-containing silicone oil | 3.56 PHR | 3.56 PHR | 3.56 PHR | 3.56 PHR | 3.56 PHR |
| Molar ratio of Si-H groups in hydrogen-containing silicone oil to vinyl in methyl vinyl silicone rubber | 4:1 | 4:1 | 4:1 | 4:1 | 4:1 |
| Amount of hydrogen in hydrogen-containing silicone oil | 0.75 mol% | 0.75 mol% | 0.75 mol% | 0.75 mol% | 0.75 mol% |
| 2-methyl-3-butyn-2-ol | 0.98 PHR | 0.98 PHR | 0.98 PHR | 0.98 PHR | 0.98 PHR |
| Platinum catalyst (10000 ppm) | 0.10 PHR | 0.20 PHR | 0.21 PHR | 0.25 PHR | 0.3 PHR |

In some preferred embodiments of the present disclosure, the raw material composition of the hollow matrix-type rod core may comprise the following components in parts by weight:

| Material name | No. 2-1 | No. 2-2 | No. 2-3 |
|---|---|---|---|
| Paliperidone | 100 PHR | 100 PHR | 100 PHR |
| Content of vinyl in methyl vinyl silicone rubber | 0.18 mol% | 0.18 mol% | 0.18 mol% |
| Amount of methyl vinyl silicone rubber | 100 PHR | 100 PHR | 100 PHR |
| Amount of hydrogen-containing silicone oil | 2.67 PHR | 3.56 PHR | 4.44 PHR |
| Molar ratio of Si-H groups in hydrogen-containing silicone oil to vinyl in methyl vinyl silicone rubber | 3:1 | 4:1 | 5:1 |
| Amount of hydrogen in hydrogen-containing silicone oil | 0.75 mol% | 0.75 mol% | 0.75 mol% |
| 2-methyl-3-butyn-2-ol | 0.98 PHR | 0.98 PHR | 0.98 PHR |
| Platinum catalyst (10000 ppm) | 0.21 PHR | 0.21 PHR | 0.21 PHR |

In the present disclosure, the ratio of the outer diameter to the inner diameter of the hollow matrix-type drug-loaded rod core may be 1.5-50: 1, for example, 2.14:1, 3:1, 4:1, 5:1, 8:1, 10:1, 15:1, 20:1, 25:1, 30:1, 35:1 or 40:1.
In the present disclosure, the outer diameter of the hollow matrix-type drug-loaded rod core may be 1 mm to 10 mm, preferably 2.0 mm, 3.0 mm, 4 mm or 5 mm.
In the present disclosure, the inner diameter of the hollow matrix-type drug-loaded rod core may be 0.1 mm-4.0 mm, preferably 0.4 mm-2.0 mm, such as 0.4 mm, 0.6 mm, 0.8 mm, 1.0 mm, 1.2 mm, 1.4 mm, 1.6 mm or 2.0 mm.
In a preferred embodiment, the outer diameter of the hollow matrix-type drug-loaded rod core may be 3 mm, the inner diameter can be 0.6-1.4 mm, 0.6mm, 1.0 mm or 1.4 mm.
In some preferred embodiments of the present disclosure, the specifications of the hollow matrix-type rod core implant are as shown in the table below, the active pharmaceutical is preferably paliperidone;

| No. | Outer diameter of rod core (mm) | Inner diameter of rod core (mm) |
|---|---|---|
| No. 5-1 | 3.00 | 0.60 |
| No. 5-2 | 3.00 | 1.00 |
| No. 5-3 | 3.00 | 1.40 |

**The present disclosure further provides a preparation method for a hollow matrix-type drug-loaded rod**

**core**, which comprises the following method:

S1: dividing the R- vinyl silicone rubber into a component A1 and a component B1;
when the raw material composition contains an inhibitor, the component A1 is mixed with the hydrogen-containing silicone oil and the inhibitor to obtain a component A2; the component B1 is mixed with the catalyst to obtain a component B2;
S2: compounding the component A2 and the component B2 to obtain a compounded rubber;
S3: compounding the compounded rubber and the active pharmaceutical ingredient to obtain a drug-loaded silicone;
S4: subjecting the drug-loaded silicone to extrusion molding and vulcanization to obtain a hollow matrix-type drug-loaded rod core.

In S1, the R-vinyl silicone rubber may be pretreated using conventional methods in the art, such as drying at 30-60°C (e.g., 40°C) for 1-24 h (e.g., 12 h) for later use.
In S1, the preparation method of the component A1 or the component B1 may be conventional in the art. Generally, the components are uniformly mixed in an open mill, then performed thin-pass process for 4-6 times, and then discharged in the form of sheets.
In S2, the step of compounding the component A2 and the component B2 may comprise the following steps: the component A2 and the component B2 are fed into an open mill according to the weight ratio of 1:1 for compounding and performing thin-pass process 4-6 times so that the components B1 and B2 are sufficiently and uniformly mixed to form a compounded rubber.
In S3, the step of compounding the compounded rubber and the active pharmaceutical ingredient may comprise the following steps: the mixed rubber and the active pharmaceutical ingredient are fed into an open mill for compounding, so that the compounded rubber and the active pharmaceutical ingredient are sufficiently and uniformly mixed to form a drug-loaded silicone. The drug-loaded silicone can be cut into strips.
In S4, the extrusion is generally carried out in a screw extruder.
In S4, during the extrusion, the screw speed of the extruder can be 1-15 r $min^{-1}$, such as 4 r $min^{-1}$or 6 r $min^{-1}$.
In S4, the diameter of the die orifice used in the extrusion is preferably 1-10 mm, such as 2 mm, 2.96 mm, 3.5 mm, 4 mm, 4.5 mm or 5 mm.
In S4, the diameter of the core mold used in the extrusion is preferably 0.1-4 mm, such as 0.58 mm, 0.8 mm, 0.97 mm, 1.2 mm or 1.38 mm.
In S4, the vulcanization is generally carried out in an oven.
In S4, the vulcanization temperature may be 25-120°C, preferably 60-90°C, such as 30°C, 50°C, 60°C, 65°C, 70°C, 80°C, 90°C, 100°C or 120°C.
In S4, the vulcanization time may be 10-200 min, preferably 10-60 min, such as 10 min, 20 min, 30 min, 40 min or 50 min.
**The present disclosure further provides a use of the hollow matrix-type drug-loaded rod core, as a release rate-modulating medium in a sustained and controlled release formulation.**
In the present disclosure, the content of vinyl in the R-vinyl silicone rubber refers to the molar percentage, which indicates the number of moles of vinyl per hundred moles of the R-vinyl silicone rubber.
In the present disclosure, the content of hydrogen content or the content of Si-H groups in the hydrogen-containing silicone oil refers to the molar percentage, which indicates the number of moles of hydrogen per hundred moles of the hydrogen-containing silicone oil.
In the present disclosure, PHR refers to the parts by mass of each specific component per 100 parts by mass of R-vinyl silicone rubber (such as methyl vinyl silicone rubber).
In the present disclosure, the room temperature refers to 25°C $\pm$ 5°C.
Based on common knowledge in the art, the above preferred conditions can be arbitrarily combined to obtain the preferred examples of the present disclosure.
The reagents and raw materials used in the present disclosure are all commercially available.
The positive and progressive effects of the present disclosure are as follows:

(1) compared with the reservoir powder-type implant, the reservoir matrix-type implant can reduce drug loss and save drug cost; reduce the phenomenon of burst release; achieve more stable drug release, and the release amount is controlled within the effective treatment window to achieve long-term stable release; at the same time, speed up production and improve production efficiency;
(2) the matrix-type implant and the reservoir matrix-type implant provided by the present disclosure can both be used as marketed preparations, such as levonorgestrel subcutaneous implant;
(3) the present disclosure adopts active pharmaceutical ingredients with an average particle size of 1-1000 nm, which can significantly improve drug solubility; and they have better compatibility with silicone rubber, and the resulting

matrix-type implant and the reservoir-type implant show significantly increased drug release rate. Taking paliperidone as an example, the release rate of the implant of the present disclosure can be increased by 3-10 times compared with the active pharmaceutical ingredient with conventional particle size;

(4) when the average particle size of paliperidone is 200-300 nm, its drug release rate can be increased by 4-8 times; when it is 300-400 nm, the drug release rate can be increased by 3-4 times; when it is 400-600 nm, the drug release rate can be increased by 2-3 times; when it is 800-900 nm, the drug release rate can be increased by 1-1.2 times;

(5) The present disclosure has a wide range of applications and is suitable for various drugs, achieving stable release at high doses. For example, the release rates of estradiol, meloxicam, and exemestane can all be increased by 4-10 times;

(6) The present disclosure adopts R-vinyl silicone rubber, hydrogen-containing silicone oil, catalyst and other components as the basic formulation of the silicone rubber. The model drug is blended and compounded with the silicone rubber by extrusion technology to prepare a hollow matrix-type drug-loaded rod core. It combines the advantages of traditional reservoir-type implant and matrix-type implant, as follows:

(1) simple production process: the production process is basically consistent with that of matrix-type implant, reducing production steps and saving costs;

(2) wide application range for drugs: applicable to all drugs, not affected by drug molecular weight, solubility, physicochemical properties, particle size, crystal form, etc. It is suitable for active ingredients with a molecular weight > 800 Da or ≤ 800 Da, as well as those with a solubility > 10 mg/mL or ≤ 10 mg/mL;

(3) stable release amount: reduces the degree of slowing down its release rate, can achieve zero-order release, stable blood drug concentration, precise therapeutic effect, and meets clinical requirements;

(4) high release amount: the drug release area is increased, enabling simultaneous drug release from both internal and external surfaces, thus elevating the drug release rate. The release dose is several times that of traditional matrix-type implant and dozens to hundreds of times that of reservoir-type implant.

## BRIEF DESCRIPTION OF THE DRAWINGS

FIG. 1 shows the drug release amount of the gestodene matrix-type rod core.

FIG. 2 shows the daily release amount of the reservoir-matrix type gestodene.

FIG. 3 shows the drug release amount at different crosslinking agent molar ratios.

FIG. 4 shows the effect of different vulcanization times of the silicone rod core on the drug release amount.

FIG. 5 shows the in vitro release of levonorgestrel silicone rod core.

FIG. 6 shows the effect of the release area of the silicone rod core on the drug release amount.

FIG. 7 shows the in vitro release results of the nanonized and non-nanonized paliperidone matrix-type rod cores prepared in Example 4-2 and Example 4-1.

FIG. 8 shows the in vitro release results of paliperidone hollow matrix-type rod cores prepared in Example 5-2 and Example 5-1 before and after nanonization.

FIG. 9 shows the in vitro release results of paliperidone matrix-type implant prepared in Example 6-2 and Example 6-1 before and after nanonization.

FIG. 10 shows the in vitro release results of paliperidone powder storage implant prepared in Example 7-2 and Example 7-1 before and after nanonization.

FIG. 11 shows the in vitro release results of paliperidone hollow matrix nano-type rod cores with different inner diameters prepared in Example 10-1 and paliperidone hollow matrix-type rod cores with different inner diameters prepared in Example 10-2.

FIG. 12 shows the comparison graph of in vitro release results between paliperidone hollow matrix nano-type rod cores with different particle sizes prepared in Example 11 and paliperidone hollow matrix-type rod cores prepared in Example 5-2.

FIG. 13 shows the in vitro release results of estradiol hollow matrix-type rod cores prepared in Example 8-2 and Example 8-1 before and after nanonization.

FIG. 14 shows the in vitro release results of meloxicam hollow matrix-type rod cores prepared in Example 9-2 and Example 9-1 before and after nanonization.

FIG. 15 is a photograph of the matrix-type rod core prepared in Example 19-4 (left in FIG. 15) and the hollow matrix-type rod core prepared in Example 15-1 (right in FIG. 15).

DETAILED DESCRIPTION OF THE PREFERRED EMBODIMENT

The present disclosure is further illustrated below by means of examples, but the present disclosure is not thereby limited to the scope of the described examples. The experimental methods for which specific conditions are not indicated in the following examples are selected according to conventional methods and conditions, or according to product instructions.

The information on the instruments and reagents used in the following examples and comparative examples is shown in Tables 1 and 2:

Table 1

| | |
|---|---|
| Precision Mini Open Mill | Dongguan Xilong Electrical Machinery Equipment Co., Ltd. |
| Φ16 Silicone Rubber Extruder | Hebei Xulang Machinery Equipment Manufacturing Co., Ltd. |
| 101-2AB Electric Blast Drying Oven | Tianjin Taist Instrument Co., Ltd. |
| 3020N Outer Diameter Measurement and Control Instrument | Shanghai Pinzhong Testing Equipment Co., Ltd. |
| V3.0 intelligent measuring system microscope | Shenzhen Zhongwei Kechuang Co., Ltd. |
| XS105 Electronic Analytical Balance | Mettler-Toledo Instruments Co., Ltd. |
| SMD200-2 Electronic Analytical Balance | Ohaus International Trade Co., Ltd. |
| QL-5E Electronic Tensile Testing Machine | Xiamen Qunlong Instrument Co., Ltd. |
| Ultima IV X-ray Diffractometer | Beijing Hongchangxin Technology Co., Ltd. |
| Zeiss Sigma 300 Scanning Electron Microscope | Carl Zeiss Shanghai Management Co., Ltd. |
| CHA-S Constant Temperature Oscillator | Changzhou Guohua Electric Appliance Co., Ltd. |
| LC-10ATVP High Performance Liquid Chromatograph | Shimadzu Corporation, Japan |
| Digital Controlled Ultrasonic Cleaner (Model KQ-250DE) | Kunshan Ultrasonic Instrument Co., Ltd. |
| Vacuum Kneader (small) | Laizhou Zhongtai Chemical Machinery Co., Ltd. |
| XPE Electronic Analytical Balance | Mettler-Toledo Instruments Co., Ltd., Switzerland |
| Vertical Hot Air Vulcanization Channel | Hebei Xulang Machinery Equipment Manufacturing Co., Ltd. |
| Horizontal Hot Air Vulcanization Channel | Hebei Xulang Machinery Equipment Manufacturing Co., Ltd. |

Table 2

| | |
|---|---|
| Gestodene (purity 98%) | Hebei Mokai Technology Development Co., Ltd. |
| levothyroxine sodium (purity 99%) | Shanghai Meilian Biotechnology Co., Ltd. |
| Levonorgestrel (purity 98.5%) | Hebei Mokai Technology Development Co., Ltd. |
| Methyl vinyl polysiloxane | Dongjue Silicone Group Co., Ltd. |
| Methyl vinyl silicone rubber, 100000-800000 g/mol | Dongjue Silicone Group Co., Ltd. |
| H-75 Hydrogen-containing silicone oil | Guangdong Silicon Ye New Material Technology Co., Ltd. |
| Hydrogen-containing silicone oil | Guangdong Silicon Ye New Material Technology Co., Ltd. |
| Platinum catalyst | Guangdong Silicon Ye New Material Technology Co., Ltd. |
| 2- methyl-3- butyn-2-ol | Jiuding Chemical Technology Co., Ltd. |

(continued)

| | |
|---|---|
| KN-300N adhesive | Kanglibang Polymer New Materials Co., Ltd. |
| Chromatographic methanol | Shandong Yuwang Chemical Reagent Co., Ltd. |
| Acetonitrile (HPLC Grade) | Shandong Yuwang Chemical Reagent Co., Ltd. |
| Ethanol (analytical grade) | Shandong Yuwang Chemical Reagent Co., Ltd. |
| HB-615 fumed silica | Yichang Huifu Silicon Materials Co., Ltd. |
| Anhydrous ethanol | Shandong Yuwang Chemical Reagent Co., Ltd. |
| Paliperidone (purity 98%), molecular weight: 426.48, solubility: 30 μg/ml. | Hubei Weideli Chemical Reagent Co., Ltd. |
| Gestodene (purity 98%), molecular weight: 310.43, insoluble in water | Hebei Mokai Technology Development Co., Ltd. |
| Estradiol (purity 99%), molecular weight: 272.39, insoluble in water | Hebei Mokai Technology Development Co., Ltd. |
| levothyroxine sodium (purity 99%), molecular weight: 800.87, solubility: 150 μg/ml | Shanghai Meilian Biotechnology Co., Ltd. |
| Metformin hydrochloride (purity 98%), molecular weight: 165.62, solubility: 346 mg/ml. | Hubei Weideli Chemical Reagent Co., Ltd. |
| Paclitaxel (purity 99%), molecular weight: 853.92, insoluble in water | Hubei Weideli Chemical Reagent Co., Ltd. |
| Centella asiatica total glycosides (purity 98%), molecular weight: | Shanghai Yuanye Bio- |
| 911.12, solubility: 120 mg/ml. | Technology Co., Ltd. |

The relevant terms are explained as follows: Matrix-type implant, also known as matrix-type rod core, means that there is an only rod core without silicone tube outside; reservoir-matrix-type implant is a rod core covered with an outer layer of silicone tube; the content of vinyl in methyl vinyl silicone rubber refers to mole percentage, the mole percentage of vinyl refers to the number of moles of vinyl per 100 moles of R-vinyl silicone rubber; the content of hydrogen of Si-H groups in hydrogen-containing silicone oil refers to mole percentage, the mole percentage of content of hydrogen of Si-H groups means the number of moles of Si-H groups per 100 moles of hydrogen-containing silicone oil.

**I. Gestodene Matrix-type Rod Core**

**1.1 Preparation of the gestodene matrix-type rod core**

Since the silicone rod core is prepared by mixing the drug with silicone rubber followed by vulcanization, the addition of the drug during vulcanization can hinder the cross-linking of silicone rubber macromolecules to a certain extent. Therefore, the vulcanization of the silicone rod core is more difficult than that of the silicone tube. Furthermore, to ensure drug stability, higher requirements are imposed on the ratio and dosage of each component in the silicone formulation.

**Example 1-1**

**Basic formulation**

The gestodene matrix-type rod core was a rod-shaped silicone rod core prepared by mixing the model drug gestodene with silicone rubber. Its formulation consisted of 20 PHR gestodene, 100 PHR methyl vinyl silicone rubber, hydrogen-containing silicone oil, 0.1 PHR platinum catalyst, and 0.98 PHR 2- methyl-3- butyn-2-ol.

Wherein, the selected gestodene had a particle size of 2-3 μm after being crushed by air flow; the selected methyl vinyl silicone rubber used vinyl as the terminal groups of the silicone rubber, and the content range of vinyl was 0.18%; the content of Si-H groups in the selected hydrogen-containing silicone oil was 0.75 mol%, the molar ratio of Si-H groups in the hydrogen-containing silicone oil to vinyl in the methyl vinyl silicone rubber was 1.5:1; the selected platinum catalyst had a platinum concentration of 10000 ppm; 2- methyl-3- butyn-2-ol was selected as the inhibitor.

Note: PHR (parts per hundreds of rubber) referred to the parts by weight of the component per 100 parts of methyl

vinyl silicone rubber; ppm (parts per million) was the parts per million notation, representing the mass concentration of platinum in the platinum catalyst.

**Preparation process**

(1) Drying pretreatment of a raw rubber: the raw methyl vinyl silicone rubber was dried at 40 °C for 12 h for later use;
(2) Preparation of component A and component B: divided the prescribed amount of the raw methyl vinyl silicone rubber into two equal parts, component A and component B, added the prescribed amounts of hydrogen-containing silicone oil and 2- methyl-3- butyn-2-ol into component A, which was uniformly mixed in the open mill, then performed thin-pass process for 5 times, and then discharged in the form of sheets, added the prescribed amount of platinum catalyst into component B, which was uniformly mixed in the open mill, then performed thin-pass process for 5 times, and then discharged in the form of sheets;
(3) Preparation of a compounded rubber: component A and component B were fed into the open mill according to the weight ratio of 1:1 for compounding and thin-pass process for 5 times so that the component A and the component B are sufficiently and uniformly mixed to form a compounded rubber;
(4) Preparation of a drug-loaded silicone: the prescribed amount of gestodene and the compounded rubber were uniformly mixed on an open mill according to the weight ratio of 1:5 to prepare drug-loaded silicone, and cut into strips;
(5) Extrusion of a silicone tube: installed a die orifice of appropriate size, set the screw speed of the extruder, and the cut drug-loaded silicone strips were fed into the extruder. The drug-loaded silicone was continuously extruded through the die orifice to form rod-shaped silicone rod core;
(6) Vulcanization of the silicone rod core: after extrusion, the silicone rod core was placed in an oven and vulcanized into an elastomer at a vulcanization temperature of 90 °C and vulcanization time of 1 h;
(7) Preparation of silicone rod core with different diameters: silicone rod core was prepared by the die orifice with a diameter of 1.9 mm;
(8) Size measurement and appearance inspection of silicone rod core: the diameter of the extruded silicone rod core was measured by an infrared diameter gauge, and the surface appearance was observed using an electron microscope. The gestodene matrix rod core had a diameter of 2 mm, a length of 1.5 cm, and a drug release area of 1.0048 $cm^2$.

**Example 1-2 to Example 1-5 (Investigation of Catalyst amount)**

Except that the amount of catalyst was adjusted to the following values (as shown in Table 3), the other operations and conditions were the same as those in Example 1-1.

Table 3

| | The amount of Platinum catalyst (PHR) | Elongation at break (%) | Tensile strength (MPa) | Tear strength (KN/m) |
|---|---|---|---|---|
| Example 1-1 | 0.10 | 41.7 ± 3.6 | 1.02 ± 0.08 | 0.71 ± 0.06 |
| Example 1-2 | 0.20 | 75.3 ± 4.3 | 1.70 ± 0.13 | 1.18 ± 0.09 |
| Example 1-3 | 0.21 | 87.1 ± 3.5 | 2.06 ± 0.12 | 1.43 ± 0.08 |
| Example 1-4 | 0.25 | 68.4 ± 6.2 | 1.50 ± 0.09 | 1.04 ± 0.14 |
| Example 1-5 | 0.30 | 54.5 ± 3.8 | 1.21 ± 0.14 | 0.90 ± 0.06 |

The results in Table 3 showed that: as the amount of platinum catalyst increased, the mechanical properties of the silicone rod core first increased significantly and then showed a slight downward trend. When the amount of platinum catalyst was 0.1 PHR, due to the insufficient amount of catalyst, complete vulcanization could not be achieved; when the amount of the platinum catalyst was 0.21 PHR, optimal performance was obtained. During the experiment, it was found that when the amount of the platinum catalyst was increased to 0.25 PHR, due to the excessive amount, the rubber material became slightly hard during the compounding of drug-rubber and the extrusion of the silicone rod core, resulting in rough extrusion surfaces and decreased mechanical properties of the silicone rod core. When the amount of the platinum catalyst was increased to 0.30 PHR, due to the excessive amount, the silicone rod core became overly hard, resulting in relatively poor mechanical properties.

**Example 1-6 and Example 1-7 (Investigation on the molar ratio of hydrogen-containing silicone oil)**

Except that the molar ratio of hydrogen-containing silicone oil was adjusted to the following values (as shown in Table 4), the other operations and conditions were the same as those in Example 1-3. The molar ratio of the hydrogen-containing silicone oil referred to the molar ratio of Si-H groups in the hydrogen-containing silicone oil to vinyl in the methyl vinyl silicone rubber.

Table 4

| | Molar ratio of hydrogen-containing silicone oil | Elongation at break (%) | Tensile strength (MPa) | Tear strength (KN/m) |
|---|---|---|---|---|
| Example 1-6 | 3.0:1 | 112.3 ± 5.8 | 2.30 ± 0.22 | 1.59 ± 0.15 |
| Example 1-7 | 4.5:1 | 136.7 ± 7.6 | 2.57 ± 0.20 | 1.78 ± 0.14 |

The results in Table 4 showed that: with the increase in the amount of hydrogen-containing silicone oil, the elongation at break, tensile strength, and tear strength of the silicone rod core gradually increased. Generally, in the addition reaction of addition-type silicone rubber, the molar ratio of hydrogen-containing silicone oil to vinyl in the silicone rubber was generally sufficient in the range of 1.2-1.8. However, for the silicone rod core mixed with drug-rubber, the large amount of drug presented in the silicone rubber hindered the contact between Si-H groups in the hydrogen-containing silicone oil and vinyl, leading to incomplete reaction. Therefore, it was necessary to further increase the molar ratio to increase the excess of hydrogen-containing silicone oil, so that more effective hydrogen-containing silicone oil could participate in the addition reaction, thereby achieving complete reaction and optimal vulcanization state of the silicone rod core. When the input of hydrogen-containing silicone oil reached a certain amount, the internal molecular bonding and crosslinking degree of the silicone tube were appropriate, and the mechanical properties reached the peak. When the amount continued to increase, there were too many molecular bonding and crosslinking points in the silicone tube, which increased the binding force of particles around it, reduced the elasticity, made the silicone tube brittle, deteriorated the toughness and reduced the elongation at break.

**Example 1-8 and Example 1-9 (Investigation of vulcanization temperature)**

Except that the vulcanization temperature was adjusted to the following values (as shown in Table 5), the other operations and conditions were the same as those in Example 1-7.

Table 5

| | Vulcanization temperature | Elongation at break (%) | Tensile strength (MPa) | Tear strength (KN/m) |
|---|---|---|---|---|
| Example 1-8 | 70°C | 70.1 ± 6.5 | 1.51 ± 0.16 | 1.04 ± 0.11 |
| Example 1-9 | 110°C | 139.8 ± 7.9 | 2.59 ± 0.19 | 1.79 ± 0.13 |

The results in Table 5 showed that: when the vulcanization temperature was 70 °C, the tensile elongation, tensile strength and tear strength of the silicone rod core were not high, and the vulcanization effect was not good. When the vulcanization temperature was 90 °C and 110 °C, the mechanical properties of the silicone rod core were all good and had little difference. During the experiment, it was found that the color of the silicone rod core became dark due to the high temperature when the curing temperature was between 110 °C and 150 °C. Therefore, considering both the mechanical properties of the silicone rod core and the thermal stability of the drug, a suitable range of vulcanization temperature was 70-110 °C.

**1.2 In vitro release of gestodene matrix-type rod core:**

**Content determination and *in vitro* release testing method for gestodene matrix-type rod core**

(1) Chromatographic conditions

Chromatographic column: Diamonsil® C18 column (250 mm × 4.60 mm, 5 μm);
Mobile phase: methanol-water (80:20, v/v);
Column temperature: 30°C;
Detection wavelength: 239 nm;
Flow rate: 1 mL·min$^{-1}$;
Injection volume: 20 μL;

*(2) In vitro* release testing method

A horizontal shaking method was employed. One implant was taken, and both ends were fixed to the bottom and wall of a 50 mL stoppered conical flask using silicone adhesive (to prevent the implant from floating on the liquid surface, which would cause inaccurate release results). After adhesion, it was left standing for 12 h to allow complete curing of the silicone adhesive. 50 mL of distilled water was exactly measured as the release medium and injected into a conical flask. The conical flask was then placed in a constant temperature air shaker for shaking. The temperature was set to 37 °C and the amplitude was 100 r·min$^{-1}$. The medium was replaced with an equal volume every 24 hours. The release solution was filtered by 0.22 μm microporous membrane and detected according to chromatographic conditions. The injection volume was 20 μL.

According to relevant literature and patent reports, a daily release of 10-20 μg of gestodene was sufficient to achieve therapeutic effect. In this experiment, the silicone rod core with a diameter of 2.0 mm, a length of 1.5 cm, a vulcanization temperature of 90 °C, and a vulcanization time of 1 h (the formulation of the rod core is the same as Example 1-7) was selected for in vitro release investigation for 35 days.

According to FIG. 1 and Table 6, the results showed that: the release of gestodene matrix-type core could be maintained within the effective treatment window of 10-20μg/d for 35 days, but the release was unstable and the daily release tends to decrease. However, there were issues with unstable release and a decreasing daily release rate.

Table 6

| Release time (d) | 1 | 5 | 10 | 15 | 20 | 25 | 30 | 35 |
|---|---|---|---|---|---|---|---|---|
| Release amount (μg/d) | 26.28 | 17.25 | 19.62 | 14.28 | 11.35 | 16.34 | 13.94 | 9.88 |

**1.3 Preparation of silicone tube:**

Table 7 Formulation, vulcanization process and size of silicone tube

| Formulation, vulcanization process and size | Silicone tube |
|---|---|
| Methyl vinyl silicone rubber | 100 PHR |
| Hydrogen-containing silicone oil | / |
| Fumed white carbon black | 40 PHR |
| 2-methyl-3-butyn-2-ol | 0.69 PHR |
| Platinum catalyst | 0.01 PHR |
| First vulcanization temperature | 300°C |
| First vulcanization time | 15s |
| Second vulcanization temperature | 240°C |
| Second vulcanization time | 2min |
| Outer diameter | 2.4 mm |
| Wall thickness | 0.2 mm |
| Length | 1.9 cm |

Note: the content of vinyl in the methyl vinyl silicone rubber was 0.18%, the content of Si-H groups in the hydrogen-containing silicone oil was 0.75 %, the molar ratio of Si-H groups in the hydrogen-containing silicone oil to vinyl in the methyl vinyl silicone rubber was 1.5:1, the selected platinum catalyst had a platinum concentration of 3000 ppm.

The specific process was as follows:

(1) Drying pre-treatment of rubber material: the reinforcing agent, fumed white carbon black, was placed in an oven at 130°C and dried for 18h hours for later use. The raw methyl vinyl silicone rubber was dried at 40°C for 18h hours for later use;

(2) Preparation of compounded rubber: the prescribed amount of fumed white carbon black and the raw methyl vinyl silicone rubber were fed into a kneader, ensuring uniform mixing of the fumed white carbon black and the raw rubber to obtain the compounded rubber. The compounded rubber was then transferred to an open mill and processed by extruding and thin-pass process (the roll distance is 1mm). By utilizing the shearing action of the two rollers of the open mill, the rubber material was further sufficiently mixed. The rubber material was then removed from the open mill in the form of sheets, wrapped in cling film, sealed in a ziplock bag, and parked in a desiccator for 18 hours for later use;

(3) Preparation of component A and component B: the compounded rubber was divided into two equal components, component A and component B, the prescribed amounts of hydrogen-containing silicone oil and 2- methyl-3- butyn-2-ol were added into component A, which was uniformly mixed in the open mill, then performed thin-pass process for several times, and then discharged in the form of sheets, the prescribed amount of platinum catalyst was added into component B, which was uniformly mixed in the open mill, then performed thin-pass process for several times, and then discharged in the form of sheets. The prepared component A and component B were individually wrapped in cling film, sealed in a ziplock bag, and parked in a desiccator for 18 hours for later use;

(4) Mixing of the component A and the component B: the parked the component A and the component B were fed into an open mill at a ratio of 1:1, and processed by performing triangular wrapping and thin-pass process through the open mill 6 times to make the component A and the component B mix sufficiently and uniformly. The mixture was discharged from the open mill in the form of sheets and then cut into strips;

(5) Extrusion of silicone tube: the appropriate-sized mold was installed, and the screw speed of the extruder was set. The cut silicone strips were fed into the extruder, and the rubber material was continuously extruded through the die orifice driven by the rotation of the screw to form a silicone tube;

(6) First vulcanization treatment: the silicone tube extruded from the die orifice was first passed through a front drying tunnel (vertical hot air vulcanization channel) for rapid high-temperature vulcanization, known as the first vulcanization treatment. This process quickly transformed the silicone tube from a sticky and soft state to an elastic state, achieving preliminary shaping;

(7) Second vulcanization treatment: after the first vulcanization, the silicone tube entered a rear drying tunnel (horizontal hot air vulcanization channel) for continued vulcanization, known as the second vulcanization treatment. This process ensured a more complete vulcanization reaction of the silicone tube, achieving optimal cross-linking. The finished silicone tube was then continuously transported out by the conveyor belt of the rear drying tunnel.

**1.4 Preparation of reservoir-matrix type gestodene implant:**

Example 1-10

(1) Treatment of the silicone tube: prepared silicone tube according to the above-mentioned section 1.3, took an addition-type silicone tube with an outer diameter of 2.4 cm that met the required appearance and performance standards. Cut it to the desired length, which was 0.4 cm longer than the silicone rod core. Reserved 0.2 cm for the sealing adhesive at both ends of the implant. Placed the cut silicone tube in a beaker and soak it in 75% ethanol for 15 minutes, then rinsed it repeatedly with 75% ethanol and let it air dry naturally;

(2) Assembly of silicone rod core and silicone tube:

Silicone rod core: except that the weight ratio of gestodene to the compounded rubber in step (4) was adjusted to 1:1, the formulation and preparation process were the same as those in Example 1-7, the rod core had a diameter of 0.2 cm and a length of 1.5 cm.

The cut silicone tube was soaked in petroleum ether for 2 min to swell. After swelling, the tube was removed from the petroleum ether, and the residual petroleum ether on the surface was quickly shaken off by hand. The swollen silicone tube shrinked rapidly back to its original shape after removal; at this point, the cut silicone rod core was immediately inserted into the middle of the tube using tweezers, leaving equal lengths of space at both ends for sealing. After assembly, the implant was air-dried naturally for 20 h to completely volatilize the residual petroleum ether;

(3) Sealing of the implant: both ends of the air-dried implant were sealed with sealing adhesive, and the implant was allowed to stand at room temperature for 20 h to cure the adhesive, thereby obtaining the reservoir-matrix type gestodene implant.

**1.5 Investigation on factors affecting *in vitro* release rate of the reservoir-matrix type gestodene implant:**

The in vitro test method of the reservoir-matrix type gestodene implant was the same as "1.2 In vitro release of gestodene matrix-type rod core".

(1) Investigation on vulcanization time of silicone rod core

Example 1-11 and Example 1-12

Except that the vulcanization time was adjusted to the following values (as shown in Table 8), the other operations and conditions were the same as those in Example 1-10.

Table 8

| | Vulcanization time |
|---|---|
| Example 1-10 | 1h |
| Example 1-11 | 2h |
| Example 1-12 | 3h |

The reservoir-matrix type gestodene implant prepared in Examples 1-10, 1-11 and 1-12 as described above were used to conduct a 30-day in vitro release test, so as to investigate the effect of vulcanization time of the silicone rod core on the release behavior of the reservoir-matrix type gestodene implant.

The results in Table 9 showed that: the mean daily release amounts of the three groups of the implants within 30 days were similar. The drug release stability at vulcanization time of 1 h and 2 h was slightly better than that at vulcanization time of 3 h. Taking the thermal stability of the drug into account, when the vulcanization time was 1 h, the silicone rod core could already reach the optimal vulcanization state and the drug release stability was also good.

Table 9 Drug release amount at different vulcanization time $\mu$g/d

| Release time (d) | 1 | 5 | 10 | 15 | 20 | 25 | 30 |
|---|---|---|---|---|---|---|---|
| Vulcanization for 1h | 33.72 | 32.05 | 28.87 | 27.19 | 25.82 | 25.44 | 24.06 |
| Vulcanization for 2h | 31.49 | 29.90 | 29.43 | 27.95 | 25.67 | 24.70 | 23.53 |
| Vulcanization for 3h | 33.94 | 30.56 | 25.48 | 24.23 | 24.31 | 24.42 | 23.70 |

(2) Investigation of drug release area of the silicone rod core

During the drug release process of the implant, the medium entered the surface of the silicone rod core through the silicone tube, dissolved the drug in the silicone rod core, and then diffused out of the silicone tube. Therefore, the contact area between the medium and the silicone rod core was the drug release area of the silicone rod core, which directly affected the drug release of the implant. The drug release area of the silicone rod core was calculated using the lateral surface area formula of a cylinder as follows:

$$S=\pi d \times L,$$

S: drug release area of the silicone rod core, d: diameter of the silicone rod core, L: length of the silicone rod core

It could be seen from the formula that when the diameter of the silicone rod core was fixed, the drug release area could be changed by adjusting the length of the silicone rod core. Therefore, in this test, as shown in Table 10, the outer diameter of the silicone tube was fixed at 2.4 mm, the wall thickness was 0.2 mm, and the diameter of the silicone rod core was fixed at 0.2 cm. The silicone rod core was cut to lengths of 0.5, 1.5, 2.5, and 3.5 cm, respectively, and assembled with the silicone tube to form the implant for 30-day of in vitro release, and the variation of in vitro release amount with the release area of the silicone rod core was investigated.

Table 10 Effect of drug release area on in vitro release of the reservoir-matrix type gestodene implant

| Drug-loaded silicone rod core (Diameter * Length) | Drug release area of the drug-loaded silicone rod core | Silicone tube (Outer diameter * Wall thickness * Length) |
|---|---|---|
| 0.2 cm*0.5 cm | 0.3141 $cm^2$ | 2.4 mm*0.2 mm*0.9 cm |
| 0.2 cm*1.5 cm | 0.9424 $cm^2$ | 2.4 mm*0.2 mm*1.9 cm |
| 0.2 cm*2.5 cm | 1.5707 $cm^2$ | 2.4 mm*0.2 mm*2.9 cm |
| 0.2 cm*3.5 cm | 2.1991 $cm^2$ | 2.4 mm*0.2 mm*3.9 cm |
| Note: except that the drug release area mentioned above was used, the preparation of the reservoir-matrix type gestodene implant was the same as those in Example 1-10. | | |

According to the results in Table 11, it could be seen that as the release area of the silicone rod core increased, the daily release amount of the reservoir-matrix type gestodene implant increased accordingly. There is a linear relationship between the drug release amount and the drug release area of the silicone rod core.

Table 11 Drug release amount in different drug release areas μg/d

| Release time (d) | 1 | 5 | 10 | 15 | 20 | 25 | 30 |
|---|---|---|---|---|---|---|---|
| 0.3141 $cm^2$ | 13.34 | 11.41 | 20.86 | 19.39 | 9.73 | 6.19 | 7.37 |
| 0.9424 $cm^2$ | 33.72 | 32.05 | 28.87 | 27.19 | 25.82 | 25.44 | 24.06 |
| 1.5707 $cm^2$ | 65.89 | 45.71 | 42.69 | 36.73 | 37.06 | 26.01 | 34.27 |
| 2.1991 $cm^2$ | 86.28 | 80.55 | 73.67 | 64.00 | 55.58 | 55.03 | 55.37 |

**1.6 In vitro release of the reservoir-matrix type gestodene implant**

Except that the size in Table 12 was used, the formula and preparation process of the rod core were the same as those in Example 1-10, and the preparation of the reservoir-matrix type gestodene implant was the same as that in Section 1.4 above.

Table 12

| Drug-loaded silicone rod core (Diameter * Length) | Drug release area of the drug-loaded silicone rod core | Silicone tube (Outer diameter * Wall thickness * Length) |
|---|---|---|
| 0.2 cm*1.5 cm | 0.9424 $cm^2$ | 2.4 mm*0.2 mm*1.9 cm |

A 150-day long-term in vitro release experiment was conducted on the reservoir-matrix type gestodene implant. The results were shown in FIG. 2 and Table 13. The results showed that: the average daily release of the final implant for 150-day was about 19.5μg, and the release stability was good, with no obvious sudden release behavior. It showed that the drug release behavior of the implant within 150-day met the requirements, and the implant could achieve controlled release for at least 5 months.

Table 13

| Release time (d) | Release amount (μg) | Release time (d) | Release amount (μg) |
|---|---|---|---|
| 1 | 33.72 | 80 | 16.78 |
| 5 | 32.05 | 85 | 18.57 |
| 10 | 28.87 | 90 | 18.43 |
| 15 | 27.19 | 95 | 17.83 |
| 20 | 25.82 | 100 | 17.85 |
| 25 | 25.44 | 105 | 15.66 |
| 30 | 24.06 | 110 | 15.45 |
| 35 | 22.83 | 115 | 15.88 |

(continued)

| Release time (d) | Release amount (μg) | Release time (d) | Release amount (μg) |
|---|---|---|---|
| 40 | 21.58 | 120 | 12.23 |
| 45 | 21.32 | 125 | 15.56 |
| 50 | 19.48 | 130 | 17.04 |
| 55 | 19.30 | 135 | 18.31 |
| 60 | 17.26 | 140 | 11.81 |
| 65 | 17.41 | 145 | 15.71 |
| 70 | 17.87 | 150 | 13.90 |
| 75 | 17.82 | | |

## II. Levothyroxine sodium Matrix-type Rod Core

### 2.1 Preparation of the levothyroxine sodium matrix-type rod core

Example 2-1

The formulation consisted of 100 PHR levothyroxine sodium, 100 PHR methyl vinyl silicone rubber, 0.3 PHR hydrogen-containing silicone oil, 0.27 PHR platinum catalyst, and 0.49 PHR 2- methyl-3- butyn-2-ol.

Wherein, the selected levothyroxine sodium had a particle size of 80 μm; the selected methyl vinyl silicone rubber used vinyl as the terminal groups of the silicone rubber, and the content range of vinyl was 0.17%; the content of Si-H groups in the selected hydrogen-containing silicone oil was 0.75 mol%, the molar ratio of Si-H groups in the hydrogen-containing silicone oil to vinyl in the methyl vinyl silicone rubber was 2:1; the selected platinum catalyst had a platinum concentration of 10000 ppm; 2- methyl-3- butyn-2-ol was selected as the inhibitor.

Except that the above formulation was used, and the vulcanization temperature was adjusted to 50 °C and the vulcanization time was adjusted to 0.5 h, the preparation of the levothyroxine sodium matrix-type rod core was the same as that in Example 1-1; the levothyroxine sodium matrix-type rod core had a diameter of 3 mm, a length of 4 cm, and a drug release area of 3.9003 $cm^2$.

Example 2-2 to Example 2-4

Except that the following amount of catalyst was used (as shown in Table 14), the other operations and conditions were the same as those in Example 2-1.

Table 14 Formulation and vulcanization of silicone rod core with different amounts of catalyst

| Formulation | Platinum catalyst (PHR) | Vulcanization time (h) | Vulcanization degree |
|---|---|---|---|
| Example 2-2 | 0.20 | 24 | Incomplete vulcanization |
| Example 2-3 | 0.25 | 5 | Complete vulcanization |
| Example 2-1 | 0.27 | 0.5 | Complete vulcanization |
| Example 2-4 | 0.30 | 0.1 | Overvulcanization |

The amount of platinum catalyst added had a significant influence on the moldability of the rod core. An insufficient amount of catalyst resulted in incomplete crosslinking of the drug core, leading to easy deformation or fracture and poor mechanical properties. Excessive amount of catalyst caused over-vulcanization, resulting in a hard texture that affects rubber processing and also led to fracture.

The effect of the amount of catalyst on the crosslinking degree of the implant was shown in Table 14. The vulcanization and molding behavior of the implant was investigated with the amount of catalyst ranging from 0.20 PHR - 0.30 PHR. It was found that when the input amount of the catalyst was less than 0.20 PHR, the implant remained in an under-vulcanized state no matter how long the vulcanization time was. When the input amount of the catalyst was 0.20 PHR, the implant could not be completely vulcanized and molded. When the input amount of the catalyst was in the range of 0.25 PHR, the implant could be molded after vulcanization for 5 h. And when the input amount of the catalyst was in the range of 0.27 PHR, the implant exhibited good moldability after only 0.5 h of vulcanization. And when the input amount of

the catalyst reached 0.30 PHR, the implant became hard and could not be extruded before compression molding, indicating an "over-vulcanized" state.

**2.2 Content determination and in vitro release testing method for levothyroxine sodium matrix-type rod core**

(1) Chromatographic conditions

Chromatographic column: SinoDetectHCL-C18 column (4.6×250 mm, 5 μm)
Mobile phase: acetonitrile - 0.1% TFA (50:50, v/v)
Column temperature: 30°C
Detection wavelength: 225 nm
Flow rate: 1 mL·$min^{-1}$
Injection volume: 20 μL

(2) *In vitro* release testing method

A horizontal shaking method was employed. One piece/one set of subcutaneous implant was taken and fixed in a 20 mL sample vial using silicone adhesive. An appropriate amount of 0.1% BSA release medium was exactly measured and added into the sample vial, such that the preparation was constantly submerged under the surface of the release medium. The vial was placed in a constant-temperature shaker with the amplitude of 100 r•$min^{-1}$ and the temperature of 37 °C for release. Samples were taken at an interval of 24 h, and the release medium was replaced. The solution was filtered through a 0.22 μm membrane filter, then injected under the chromatographic conditions for detection and recording.

**2.3 Investigation on factors affecting *in vitro* release rate of the levothyroxine sodium matrix-type implant:**

Example 2-5 to Example 2-7 (molar ratio of crosslinking agent)
Except that the following molar ratio of crosslinking agent was used, the other operations and conditions were the same as those in Example 2-1.
The molar ratio of crosslinking agent amount affected the vulcanization crosslinking degree of the implant, while the crosslinking network degree affected the later release diffusion rate. See Table 15 for the specific formulation.

Table 15 Formulations of silicone rod core with different crosslinking agent ratios

| Formulation | Molar ratio of crosslinking agent |
|---|---|
| Example 2-1 | 2:1 |
| Example 2-5 | 3:1 |
| Example 2-6 | 3.5:1 |
| Example 2-7 | 4:1 |

The results of different crosslinking ratios were shown in FIG. 3 and Table 16. When the molar ratio of crosslinking agent was 2:1, the addition amount was too small, resulting in fewer formed channels. The internal branches of the implant were disorderly arranged, which hindered the release of the mixed drug and led to a low release amount. When the molar ratio was 4:1, an excessive amount of crosslinking agent caused an unstable internal crosslinking structure and side reactions were easy to occur, resulting in changes in the internal structure and hindering drug release. When the ratios were 3:1 and 3.5:1, the internal network channels were more suitable for drug diffusion, the release amount was closer to the release requirements, and the release was more stable.

Table 16 Effect of molar ratio of different crosslinking agents on drug release μg/d

| Release time (d) | 1d | 5d | 10d | 15d | 20d | 25d | 30d |
|---|---|---|---|---|---|---|---|
| Molar ratio of crosslinking agent is 2:1. | 34.85 | 39.61 | 39.65 | 22.36 | 16.48 | 21.22 | 12.43 |
| Molar ratio of crosslinking agent is 3:1. | 63.53 | 69.15 | 54.99 | 59.56 | 54.99 | 57.43 | 48.79 |
| Molar ratio of crosslinking agent is 3.5:1. | 58.34 | 49.93 | 45.21 | 42.71 | 42.51 | 48.07 | 37.54 |

(continued)

| Release time (d) | 1d | 5d | 10d | 15d | 20d | 25d | 30d |
|---|---|---|---|---|---|---|---|
| Molar ratio of crosslinking agent is 4:1. | 44.42 | 46.66 | 44.61 | 31.58 | 26.24 | 31.32 | 20.44 |
| Note: the test object in Table 16 was a levothyroxine sodium matrix-type implant. | | | | | | | |

Example 2-8 to Example 2-10 (drug release area)

Except that the rod core with the following size was prepared, the other operations and conditions (components and amount of the rod core) were the same as those in Example 2-5.

The matrix-type implant was a cylinder, and the drug was released through the side and two bottoms of the cylinder. The drug release area was related to the diameter and length of the implants. Considering the patient's compliance, implants with diameters of 2 mm and 3 mm at various lengths were investigated.

$$S_{\text{drug release area}} = 2S_{\text{bottom}} + S_{\text{side}} = 2\pi r^2 + \pi dL$$

r: implant bottom radius d: implant bottom diameter L: implant length

The drug release areas of matrix-type implants with different diameters and lengths were shown in Table 17. The effect of different drug release areas on the in vitro release of matrix-type implants was investigated.

Table 17 Release area table of the silicone rod cores with different sizes

| Formulation | *Diameter of the silicone rod core (cm)* | Length of the silicone rod core (cm) | Drug release area ($cm^2$) |
|---|---|---|---|
| Example 2-8 | 0.3 | 2.0 | 2.0253 |
| Example 2-9 | 0.3 | 3.0 | 2.9673 |
| Example 2-10 | 0.3 | 4.0 | 3.9093 |

The results of drug release area were shown in Table 18. When the diameter was 3 mm and the length was increased to 4 cm, it was found that the daily release of the preparation was obviously increased and the release was more stable.

Table 18 Drug release amount in different drug release areas μg/d

| Release time (d) | 1 | 5 | 10 | 15 | 20 | 25 | 30 |
|---|---|---|---|---|---|---|---|
| 2.02503 $cm^2$ | 33.25 | 30.04 | 33.94 | 28.45 | 26.55 | 25.98 | 26.46 |
| 2.9673 $cm^2$ | 43.36 | 39.51 | 41.79 | 41.58 | 42.24 | 40.35 | 36.70 |
| 3.9003 $cm^2$ | 59.22 | 61.43 | 61.72 | 59.43 | 56.96 | 58.23 | 54.66 |
| Note: the test object in Table 18 was a levothyroxine sodium matrix-type implant. **2.4 In vitro release of the levothyroxine sodium matrix-type implant:** | | | | | | | |

In vitro release test was performed on the levothyroxine sodium matrix-type implant prepared in Example 2-10.

One set consisting of two levothyroxine sodium matrix-type implants was subjected to long-term release for one month. In vitro release profile was shown in Table 19. The drug release exhibited a slow downward trend, which was attributed to the gradual dissolution and release of the drug in the outer layer, resulting in an increase in the blank annular carrier region. The path for solvent molecules to enter the interior of the implant and the diffusion path for the dissolved drug to migrate outward became longer, leading to a decrease in the diffusion rate and a gradual reduction in the daily release amount. The daily release amount was maintained at 100-120 μg/d within one month. The results demonstrated that the prepared matrix-type implants could effectively release the drug for one month.

Table 19

| Release time (d) | 1 | 5 | 10 | 15 | 20 | 25 | 30 |
|---|---|---|---|---|---|---|---|
| Release amount (μg/d) | 119.46 | 119.48 | 119.79 | 120.23 | 113.92 | 115.91 | 105.96 |

**III. A levonorgestrel matrix-type silicone rod core**

**3.1 Preparation of the levonorgestrel matrix-type silicone rod core**

Example 3-1

Basic Formulation

10 PHR levonorgestrel; 100 PHR of methyl vinyl silicone rubber; 0.667 PHR hydrogen-containing silicone oil; 0.49 PHR 2- methyl -3- butyne -2-ol (inhibitor); 0.25 PHR platinum catalyst;

Wherein, the selected methyl vinyl silicone rubber had vinyl as the terminal groups, and the content of vinyl was 0.18%; the content of Si-H groups in the selected hydrogen-containing silicone oil was 0.75mol%, the molar ratio of Si-H groups in the hydrogen-containing silicone oil to vinyl in the methyl vinyl silicone rubber was 1.5:1; the selected platinum catalyst had a platinum concentration of 10000 ppm; the process conditions were as follows: vulcanization temperature was 110 °C, vulcanization time was 1 h.

Note: PHR (parts per hundreds of rubber) referred to the parts by weight of the corresponding component per 100 parts by weight of the base polymer.

Preparation Method

Except that the above formulation was used, the preparation of the levonorgestrel matrix-type rod core was the same as that in Example 1-1; the levonorgestrel matrix-type rod core had a diameter of 2 mm, a length of 4 cm, and a drug release area of 2.5748 $cm^2$.

**3.2. In vitro release testing methods for levonorgestrel matrix-type implant**

Chromatographic conditions
Chromatographic column: Diamonsil® C18 column (250 mm × 4.60 mm)
Mobile phase: acetonitrile-water (70:30, v/v)
Column temperature: 30°C
Detection wavelength: 240 nm
Flow rate: 1 $mL\cdot min^{-1}$
Injection volume: 20 μL

(2) *In vitro* release testing method

The release rate was determined by horizontal shaking method. One set of implants was taken and fixed alternately in a 100 mL stoppered conical flask using an adhesive. A 100 mL aliquot of distilled water was accurately measured and added as the release medium. The flask was placed in a constant-temperature shaker with an amplitude of 100 $r\cdot min^{-1}$ and a temperature of 37 °C, ensuring the implants were always submerged under the liquid surface. Samples were taken every 24 h, and an equal volume of fresh release medium was replaced. The sample solution was filtered through a 0.22 μm microporous membrane filter and then determined according to the "chromatographic conditions".

**3.3 Investigation on the process of levonorgestrel matrix-type silicone rod core:**

The most important process factors affecting the vulcanization and molding of the silicone rod core were vulcanization temperature and vulcanization time. Vulcanization temperature was related to whether the silicone rod core could be vulcanized and molded. Too high temperature might lead to drug decomposition and affect drug release. If the temperature was too low, the silicone rod core might not be completely vulcanized. Vulcanization time also faced the same problem. Too long or too short time might affect the drug release behavior.

Example 3-2 and Example 3-3 (Effect of vulcanization temperature)

Except that the following vulcanization temperature was used (as shown in Table 20), the other operations and conditions were the same as those in Example 3-1.

Table 20

| | Example 3-2 | Example 3-1 | Example 3-3 |
|---|---|---|---|
| Vulcanization temperature | 90°C | 110°C | 130°C |

The effect of different vulcanization temperatures of silicone rod core on in vitro release was shown in Table 21. When the vulcanization temperature of the drug core was 90 °C and 110 °C, the daily release amount was similar, but the release amount significantly decreased at 130 °C. At 110°C, the daily release and mechanical properties were the best.

Table 21 Drug release amount at different vulcanization temperature μg/d

| Release time (d) | 1 | 5 | 10 | 15 | 20 | 25 | 30 | 35 |
|---|---|---|---|---|---|---|---|---|
| 90°C | 21.47 | 21.64 | 21.27 | 21.81 | 21.84 | 20.09 | 22.26 | 19.70 |
| 110°C | 24.14 | 21.38 | 20.39 | 19.83 | 21.27 | 17.01 | 18.67 | 21.65 |
| 130°C | 29.82 | 18.27 | 15.52 | 15.41 | 15.23 | 14.88 | 13.70 | 12.57 |
| Note: the test object in Table 21 was a levonorgestrel matrix-type implant. | | | | | | | | |

Example 3-4 and Example 3-5 (Effect of vulcanization time)

Except that the following vulcanization temperature was used (as shown in Table 22), the other operations and conditions were the same as those in Example 3-1.

Table 22

| | Example 3-1 | Example 3-4 | Example 3-5 |
|---|---|---|---|
| Vulcanization time | 1h | 0.5h | 2h |

The effects of different vulcanization time of the silicone rod cores on in vitro release were as shown in FIG. 4 and Table 23. The silicone rod core with a vulcanization time of 0.5 h was prepared as a matrix-type implant, and the daily release amount was relatively high. However, the difference in daily release amount between the silicone rod cores with vulcanization times of 1 h and 2 h was not significant. When the vulcanization time was 1h, the daily release and mechanical properties were better, and the time cost of preparation process was lower.

Table 23 Drug release amount at different vulcanization time μg/d

| Release time (d) | 1 | 5 | 10 | 15 | 20 | 25 | 30 | 35 |
|---|---|---|---|---|---|---|---|---|
| 0.5h | 29.16 | 27.97 | 32.26 | 27.14 | 32.62 | 27.71 | 31.08 | 26.59 |
| 1h | 24.14 | 21.38 | 20.39 | 19.83 | 21.27 | 17.01 | 18.67 | 21.65 |
| 2h | 18.67 | 23.21 | 26.05 | 24.79 | 27.46 | 24.50 | 26.31 | 22.40 |
| Note: the test object in Table 23 was a levonorgestrel matrix-type implant. | | | | | | | | |

**3.4 In vitro release of the matrix-type silicone rod core:**

The levonorgestrel silicone rod core prepared in Example 3-1 was subjected to a 35-day release.
As shown in the release curve (FIG. 5 and Tables 21-22), the drug release could be maintained within the effective therapeutic window, but the release was unstable and the daily release amount tended to decrease.

**3.5 Preparation of reservoir-matrix type levonorgestrel implant**

Example 3-6

Preparation of reservoir-matrix type levonorgestrel implant:

The reservoir-matrix levonorgestrel implants were prepared according to the aforementioned preparation method of "1.4 Preparation of reservoir-matrix type gestodene implant". The formulations, vulcanization process and size information of the silicone tube and the rod core were shown in Table 24 and Table 25, respectively. Except for these, the other steps were the same as those for the preparation of the reservoir-matrix gestodene implant.

Table 24 Final formulation, vulcanization process and size of silicone tube

| Final formulation, vulcanization process and size | Silicone tube |
|---|---|
| Methyl vinyl silicone rubber | 100 PHR |
| Hydrogen-containing silicone oil | 0.533 PHR |
| Fumed white carbon black | 50 PHR |
| 2-methyl-3-butyn-2-ol | 0.74 PHR |
| Platinum catalyst | 0.01 PHR |
| First vulcanization temperature | 300°C |
| First vulcanization time | 15s |
| Second vulcanization temperature | 240°C |
| Second vulcanization time | 2min |
| Outer diameter | 2.4 mm |
| Wall thickness | 0.2 mm |
| Length | 4.4 cm |

Note: the content of vinyl in the methyl vinyl silicone rubber was 0.18%, the content of Si-H groups in the hydrogen-containing silicone oil was 0.75 %, the molar ratio of Si-H groups in the hydrogen-containing silicone oil to vinyl in the methyl vinyl silicone rubber was 1.2:1, the platinum catalyst had a platinum concentration of 3000 ppm.

Table 25 Final formulation, vulcanization process and size of drug-loaded silicone rod core

| Final formulation, vulcanization process and size | Silicone rod core |
|---|---|
| Methyl vinyl silicone rubber | 100 PHR |
| Levonorgestrel | 50 PHR |
| Hydrogen-containing silicone oil | / |
| 2-methyl-3-butyn-2-ol | 0.49 PHR |
| Platinum catalyst | 0.25 PHR |
| Vulcanization temperature | 110°C |
| Vulcanization time | 1 hours |
| Diameter | 0.24 cm |
| Length | 4 cm |

Note: the content of vinyl in the methyl vinyl silicone rubber was 0.18%, the content of Si-H groups in the hydrogen-containing silicone oil was 0.75 %, the molar ratio of Si-H groups in the hydrogen-containing silicone oil to vinyl in the methyl vinyl silicone rubber was 1.5:1, the platinum catalyst had a platinum concentration of 10000 ppm.

**3.6 Investigation on factors affecting *in vitro* release rate of the levonorgestrel reservoir-matrix type levonorgestrel implant:**

The in vitro release testing methods of reservoir-matrix type levonorgestrel implant was the same as "3.2. In vitro release testing methods for levonorgestrel matrix-type implant".

**Investigation of drug release area**

Except that the size in Table 26 was used, the other operations and conditions were the same as those in Example

3-6.

The implant was cylindrical in appearance, with both ends sealed by end-capping adhesive. The drug was released entirely through the lateral surface of the preparation. The drug release area directly determined the contact extent between the drug and body fluid, as well as the diffusion pathway for drug release, and thus played a crucial role in drug release. The drug release area of the implant was S=π•d•L, where d was the diameter of the silicone rod core, L was the length of the silicone rod core. The reservoir-matrix levonorgestrel implant of different specifications were prepared according to Table 27 to investigate the effect of drug release area on the in vitro release test.

Table 26 Effect of drug release area on daily release

| *Outer diameter of rod core (mm)* | *Length of the rod core* (mm) | Drug release area ($mm^2$) | Outer diameter of silicone tube (mm) | Wall thickness of silicone tube (mm) | Length of silicone tube (mm) |
|---|---|---|---|---|---|
| 2.0 | 10 | 62.8 | 2.4 | 0.2 | 14 |
| 2.0 | 20 | 125.6 | 2.4 | 0.2 | 24 |
| 2.0 | 30 | 188.4 | 2.4 | 0.2 | 34 |
| 2.0 | 40 | 251.2 | 2.4 | 0.2 | 44 |
| 2.0 | 50 | 314.0 | 2.4 | 0.2 | 54 |

The effect of different drug release areas on in vitro release was shown in FIG. 6 and Table 27. As the drug release area increased, the daily release amount gradually increased. A positive correlation was observed between the in vitro daily release amount and the drug release area of the implant, indicating that the drug release area exerted a significant effect on the daily release amount of the reservoir-matrix levonorgestrel implant.

Table 27 Drug release amount in different drug release areas μg/d

| Release time (d) | 1 | 5 | 10 | 15 | 20 | 25 | 30 | 35 |
|---|---|---|---|---|---|---|---|---|
| 62.8 $mm^2$ | 5.21 | 8.37 | 9.23 | 10.32 | 10.38 | 6.70 | 7.46 | 6.75 |
| 125.6 $mm^2$ | 13.85 | 14.13 | 14.50 | 14.88 | 15.55 | 10.91 | 13.70 | 14.23 |
| 188.4 $mm^2$ | 15.77 | 19.36 | 22.61 | 25.18 | 18.99 | 18.65 | 17.82 | 19.11 |
| 251.2 $mm^2$ | 22.66 | 22.83 | 23.91 | 20.33 | 27.31 | 18.79 | 24.50 | 27.89 |
| 314.0 $mm^2$ | 34.15 | 37.46 | 39.09 | 34.40 | 36.97 | 36.30 | 33.49 | 36.49 |
| Note: the test object in Table 27 was a reservoir-matrix type levonorgestrel implant. | | | | | | | | |

An increase in the drug release area of the implant led to a larger contact area with the release medium, as well as a greater diffusion area for the dissolved drug. As the drug release pathways increased, the daily release amount showed a significant upward trend.

Compared with the levonorgestrel matrix-type implant, the release amount of the reservoir-matrix type levonorgestrel implant with a controlled release membrane was more stable. The implant wrapped in controlled release membranes increased the diffusion path of drugs under the action of controlled release membranes, and the diffusion rate of drugs in the membrane was lower than that in the matrix, resulting in a relatively low release amount; in addition, the controlled release membrane also slowed down the formation of blank areas in the silicone rod core, maintaining a certain drug concentration inside the membrane, allowing the implant to be released smoothly.

Table 28

| Release time (d) | Release amount (μg) | Release time (d) | Release amount (μg) |
|---|---|---|---|
| 1 | 48.28 | | |
| 5 | 42.76 | 95 | 45.04 |
| 10 | 40.78 | 100 | 44.57 |
| 15 | 39.65 | 105 | 39.23 |
| 20 | 42.53 | 110 | 38.77 |
| 25 | 34.07 | 115 | 36.28 |

(continued)

| Release time (d) | Release amount (μg) | Release time (d) | Release amount (μg) |
|---|---|---|---|
| 30 | 40.28 | 120 | 36.40 |
| 35 | 43.29 | 125 | 35.83 |
| 40 | 38.94 | 130 | 37.48 |
| 45 | 39.88 | 135 | 34.94 |
| 50 | 39.95 | 140 | 42.52 |
| 55 | 35.79 | 145 | 44.36 |
| 60 | 35.65 | 150 | 41.21 |
| 65 | 42.83 | 155 | 40.82 |
| 70 | 39.90 | 160 | 36.56 |
| 75 | 39.65 | 165 | 37.67 |
| 80 | 44.44 | 170 | 41.20 |
| 85 | 42.63 | 175 | 46.09 |
| 90 | 39.99 | 180 | 43.11 |
| Note: the test object in Table 28 was two reservoir-matrix type levonorgestrel implants. | | | |

In vitro release tests were performed on the reservoir-matrix type levonorgestrel implants with a silicone rod core drug release area of 251.2 $mm^2$. The results in Table 28 showed that the mean daily release amount of the final implants over 180 days was approximately 40 μg, with good drug release stability and no obvious burst release. It showed that the drug release behavior of the implant within 180-day met the requirements, and the implant could achieve controlled release for at least 6 months.

**Example 4-1 paliperidone matrix nano-type rod core**

**A. Basic formulation**

(1) nano paliperidone, which an average particle size was 220nm, and the addition amount was 100 PHR;
(2) methyl vinyl silicone rubber, which the content of vinyl was 0.18mol%, and an addition amount was 100 PHR;
(3) hydrogen-containing silicone oil, which the content of Si-H group was 0.75mol%, and an addition amount was 4.44 PHR;
(4) platinum catalyst, which the concentration of platinum was 10000ppm, and an addition amount was 0.21 PHR;
(5) 2- methyl -3- butyne -2-ol, which an addition amount was 0.98 PHR;

Wherein, the molar ratio of Si-H groups in hydrogen-containing silicone oil to vinyl in R-vinyl silicone rubber was 5:1.

Note: PHR (parts per hundreds of rubber) referred to the parts by weight of the component per 100 parts of methyl vinyl silicone rubber; ppm (parts per million) was the parts per million notation, representing the mass concentration of platinum in the platinum catalyst.

**B. Preparation process:**

(1) Drying pretreatment of a raw rubber: the raw methyl vinyl silicone rubber was dried at 40 °C for 12 h for later use;
(2) Preparation of component rubbers A and B: the raw rubber with the prescribed amount was evenly divided into components A and B. The hydrogen-containing silicone oil and inhibitor were added to component A, mixed uniformly in an open mill, and then subjected to thin-pass process through the open mill several times before being removed in the form of sheets. The amount of platinum catalyst was added to component B, mixed uniformly in an open mill, and

subjected to thin-pass process through the open mill several times before being removed in the form of sheets;

(3) Preparation of a compounded rubber: component A and component B were fed into the open mill according to the weight ratio of 1:1 for compounding and thin-pass process for 6 times so that the component rubbers A and B were sufficiently and uniformly mixed to form a compounded rubber;

(4) Preparation of a drug-loaded silicone: nano paliperidone and the compoundedd rubber were uniformly mixed on an open mill according to the weight ratio of 1:1 to prepare drug-loaded silicone, and cut into strips;

Nano paliperidone was obtained by nanonizing raw paliperidone. Specifically: wet grinding was carried out using a planetary ball mill. 0.4 g of poloxamer 188 as stabilizer was added to 2 g of raw paliperidone. Zirconia beads with a diameter of 0.1 mm were used as grinding medium. The milling time was controlled at 50 min and the rotation speed at 400 $r \cdot min^{-1}$. After milling, freeze-drying was carried out, and the nanonized lyophilized powder was obtained.

(5) Extrusion of a rod core: installed a die orifice with a diameter of 2.96mm, set the screw speed of the extruder to 7$r \cdot min^{-1}$, and the cut drug-loaded silicone strips were fed into the extruder. The drug-loaded silicone was continuously extruded through the die orifice to form rod core;

(6) Vulcanization of rod core: the extruded rod core was placed in an oven and vulcanized at 80 °C for 50 min to form an elastomer.

The diameter of paliperidone matrix nano-type rod core was determined by the diameter of the die orifice, and the outer diameter of the rod core made in this example was 3 mm.

### Example 4-2 paliperidone matrix-type rod core

#### A. Basic formulation

(1) The addition amount of paliperidone was 100 PHR, and its average particle size was 5 μm;

(2)-(5) were the same as the formulations (2)-(5) of Example 4-1.

#### B. Preparation process

Compared with Example 4-1, the difference of Example 4-2 was that paliperidone was directly used as the raw material in step (4), and other operations were the same as those in Example 4-1.

### Example 5-1 Paliperidone hollow matrix nano-type rod core

#### A. Basic Formulation

The basic formulation was the same as Example 4-1.

#### B. Preparation process

The preparation processes of (1)-(4) and (6) were the same as those of Example 4-1.

(5) Extrusion of hollow matrix-type rod core: a die orifice with a diameter of 2.96 mm and a core mold with a diameter of 1.4 mm were installed. The screw speed of the extruder was set at 7 $r \cdot min^{-1}$. The cut drug-loaded silicone strips were fed into the extruder, and the drug-loaded silicone was continuously extruded through the die orifice under the push of the screw to form hollow matrix-type rod core.

The outer diameter of paliperidone hollow matrix nano-type rod core was determined by the diameter of die orifice, and the inner diameter was determined by the diameter of core mold. The rod core made in this example had an outer diameter of 3mm and an inner diameter of 1.4 mm.

### Example 5-2 Paliperidone hollow matrix-type rod core

#### A. Basic Formulation

The basic formulation was the same as Example 4-2.

**B. Preparation process**

Compared with Example 5-1, the difference of Example 5-2 was that paliperidone was directly used as the raw material in step (4), and other operations were the same as those in Example 5-1.

**Example 6-1 Paliperidone matrix reservoir nano-type implant**

**A. Basic Formulation:**

It consisted of a silicone tube and paliperidone matrix nano-type rod core with an average particle size of 220 nm prepared by the preparation method of Example 4-1.

**a. Silicone tube basic formulation**

The basic formulation for the silicone tube consisted of a base polymer, a reinforcing agent, a crosslinking agent, a catalyst, and an inhibitor.

(1) The selected basic polymer was methyl vinyl polysiloxane, and the vinyl was the capping group of polysiloxane. The content of vinyl was 0.18 mol%, and the addition amount was 100 PHR;
(2) The selected reinforcing agent was fumed white carbon black, and the addition amount was 40 PHR;
(3) The selected crosslinking agent was hydrogen containing silicone oil, which the content of the Si-H groups was 0.75 mol% and an addition amount was 1.07 PHR.
(4) The selected platinum catalyst, which the concentration of platinum was 3000ppm, and an addition amount was 0.21 PHR;
(5) The selected inhibitor was 2- methyl -3- butyne -2-ol, which an addition amount was 0.98 PHR;
wherein, the molar ratio of Si-H groups in hydrogen-containing silicone oil to vinyl in methyl vinyl polysiloxane was 1.2:1.

**b. Basic formulation of paliperidone matrix nano-type rod core**

The basic formulation of the paliperidone matrix nano-type rod core was the same as that prepared in Example 4-1; wherein, the average particle size of paliperidone was 220 nm.

**B. Preparation process**

(1) Preparation of silicone tube:

(i) drying pre-treatment of rubber material: the reinforcing agent, fumed white carbon black, was placed in an oven at 130°C and dried for 12 hours for later use. The methyl vinyl silicone rubber raw rubber was dried at 40°C for 12 hours for later use.
(ii) preparation of compounded rubber: the prescribed amount of fumed white carbon black and methyl vinyl silicone rubber raw rubber were fed into a kneader, and kneaded at 30 °C for 30 min, ensuring uniform mixing of the fumed white carbon black and raw rubber to obtain the compounded rubber. The mixed rubber was then transferred to an open mill and subjected to extruding and thin-pass process through the open mill. Triangular packs were formed and performed thin-pass process for 5 times at a roll distance of 1 mm to further homogenize the rubber material via shearing action between the two rolls. The rubber material was then removed from the open mill in the form of sheets, wrapped in cling film, sealed in a ziplock bag, and stored in a desiccator for 24 hours for later use.
(iii) preparation of components A and B: the compounded rubber was evenly divided into components A and B. The prescribed amount of hydrogen-containing silicone oil and inhibitor were added to component A, mixed uniformly in an open mill, and then subjected to thin-pass process through the open mill several times before being removed in the form of sheets. The prescribed amount of platinum catalyst was added to component B, mixed uniformly in an open mill, and then subjected to thin-pass process through the open mill several times before being removed in the form of sheets. The prepared components A and B were individually wrapped in cling film, sealed in a ziplock bag, and packed in a desiccator for 24 hours for later use.
(iv) Mixing of the component A and the component B: the packed the component A and the component B were fed

into an open mill in a 1:1 ratio, and processed by performing triangular wrapping and thin-pass process through the open mill for 6 times, ensuring thorough and uniform mixing of the component A and the component B. The mixture was then removed from the open mill in the form of sheets and cut into strips.

(v) Extrusion of silicone tube: a die orifice with a diameter of 2.96 mm was installed, and the screw speed of the extruder was set at 7 $r \cdot min^{-1}$. The cut silicone strips were fed into the extruder, and the rubber material was continuously extruded through the mold opening driven by the rotation of the screw to form a silicone tube.

(vi) First vulcanization treatment: the silicone tube extruded from the die orifice was first passed through a front drying tunnel (vertical hot air vulcanization channel) for rapid high-temperature vulcanization, known as the first vulcanization treatment (the temperature is 300°C, and the vulcanization time is about 5s). This process quickly transformed the silicone tube from a sticky and soft state to an elastic state, achieving preliminary shaping.

(vii) Second vulcanization treatment: after the first vulcanization, the silicone tube entered a rear drying tunnel (horizontal hot air vulcanization channel) for continued vulcanization, known as the second vulcanization treatment (the temperature is 280°C, and the vulcanization time is about 2min). This process ensured a more complete vulcanization reaction of the silicone tube, achieving optimal cross-linking. The finished silicone tube was then continuously transported out by the conveyor belt of the rear drying tunnel. A silicone tube with an outer diameter of 3.4 mm and a wall thickness of 0.2 mm was prepared.

(2) Preparation of paliperidone matrix nano-type rod core: the preparation method of paliperidone matrix nano-type rod core was the same as that in Example 4-1.

(3) Treatment of the silicone tube: an addition-type silicone tube with qualified appearance and properties was taken, and cut to a length of 4.4 cm, which was 0.4 cm longer than the paliperidone matrix nano-type rod core, reserving 0.2 cm for sealing adhesive at each end of the implant. Placed the cut silicone tube in a beaker and soaked it in 75% ethanol for 15 minutes, then rinsed it repeatedly with 75% ethanol and let it air dry naturally.

(4) Assembly of paliperidone matrix nano-type rod core and silicone tube: the above silicone tube was soaked and swelled in petroleum ether for 1-2 min. After swelling, the silicone tube was taken out and quickly shaken to remove residual petroleum ether on the surface. The swelled silicone tube would rapidly shrink back to their original shape after being removed from petroleum ether. At this point, the cut paliperidone matrix nano-type rod core (4 cm in length, 3 mm in outer diameter) was immediately and rapidly inserted into the middle of the silicone tube using tweezers, leaving equal lengths at both ends of the silicone tube for sealing. After assembly, the implant was air-dried naturally for 24 h to volatilize residual petroleum ether.

(5) Sealing of the implant: both ends of the air-dried implant were sealed with sealing adhesive, and the implant was allowed to stand at room temperature for 24 h to cure the adhesive, thereby obtaining the paliperidone implant.

The paliperidone matrix reservoir nano-type implant prepared by the above method, wherein, the drug-loaded rod core was matrix-type drug-loaded rod core with a diameter of 3.0 mm and a length of 4.0 cm. The silicone tube had an outer diameter of 3.4 mm, a wall thickness of 0.2 mm, and a length of 4.4 cm, with a drug release area of 3.77 $cm^2$.

### Example 6-2 Paliperidone matrix reservoir-type implant

#### A. Basic formulation

It consisted of a silicone tube and matrix-type rod core prepared by Example 4-2.

##### a. Silicone tube basic formulation

The basic formulation of the silicone tube was the same as that prepared in Example 6-1.

##### b. Basic formulation of matrix-type rod core

The basic formulation of the matrix-type rod core was the same as that prepared in Example 4-2.

#### B. Preparation process

Compared with Example 6-1, the difference of Example 6-2 was that the rod core was different; specifically, except that the paliperidone matrix nano-type rod core prepared in step (2) was replaced with the paliperidone matrix-type rod core prepared in Example 4-2; all other operations were the same as in Example 6-1, other operations were the same as those in Example 6-1.

**Example 7-1 Paliperidone powder reservoir nano-type implant**

**A. Basic formulation:**

It consisted of a silicone tube and a nano powder-type drug core. Silicone tube had the same basic formulation as prepared by Example 6-1. The nano-powder-type core was paliperidone with an average particle size of 220nm.

**B. Preparation process:**

Compared with Example 6-1, the difference in Example 7-1 was that the rod core was different; Specifically, on the one hand, the paliperidone matrix nano-type rod core prepared in step (2) was replaced with a nano-powder-type drug core; on the other hand, in step (4), after the silicone tube was cleaned with ethanol, one end of the silicone tube was sealed with silicone sealing adhesive first, and the nano-powder-type drug core was filled through a powder-filling funnel. In step (5), the other end was sealed with silicone.

The paliperidone powder reservoir nano-type implant prepared by the above method, wherein, the silicone tube had an outer diameter of 3.4 mm, a wall thickness of 0.2 mm, a length of 4.4 cm, the drug release area of 3.77 $cm^2$; the diameter of the drug-loaded sections was 3.0 mm and the length was 4.0 cm

**Example 7-2 Paliperidone powder reservoir-type implant**

**A. Basic formulation**

It consisted of silicone tube and powder-type drug core. Silicone tube had the same basic formulation as prepared by Example 6-1. The powder-type drug core was paliperidone with an average particle size of 5μm.

**B. Preparation process**

Compared with Example 7-1, the difference of Example 7-2 was that the rod core was different; specifically, except that nano-powder-type drug core was replaced with powder-type drug core, other operations were the same as those in Example 7-1.

**Example 8-1 Estradiol hollow matrix nano-type rod core**

On the basis of Example 5-1, only the active pharmaceutical ingredient was changed from nano paliperidone to nano estradiol, the average particle size of nano estradiol was 198nm.

**Example 8-2 Estradiol hollow matrix-type rod core**

On the basis of Example 8-1, only the active pharmaceutical ingredient was changed from nano estradiol to estradiol, and the average particle size of estradiol was 66 μm.

**Example 9-1 Meloxicam hollow matrix nano-type rod core**

On the basis of Example 5-1, only the active pharmaceutical ingredient was changed from nano paliperidone to nano meloxicam, the average particle size of nano meloxicam was 308nm.

**Example 9-2 Meloxicam hollow matrix-type rod core**

On the basis of Example 9-1, only the active pharmaceutical ingredient was changed from nano meloxicam to meloxicam, and the average particle size of estradiol was 80 μm.

**Example 10-1 Paliperidone hollow matrix nano-type rod cores with different inner diameters**

On the basis of Example 5-1, only the size of core mold during extrusion of the hollow matrix-type rod core was changed to 0.58mm, 0.97mm, and 1.38mm, respectively. Paliperidone hollow matrix nano-type rod cores with the outer diameter of 3 mm and inner diameters of 0.6 mm, 1.0 mm, and 1.4 mm were obtained.

**Example 10-2 Paliperidone hollow matrix-type rod cores with different inner diameters**

On the basis of Example 5-2, only the size of core mold during extrusion of the hollow matrix-type rod core was changed to 0.58mm, 0.97mm, and 1.38mm, respectively. Paliperidone hollow matrix nano-type rod cores with the outer diameter of 3 mm and inner diameters of 0.6 mm, 1.0 mm, and 1.4 mm were obtained. In other words, compared with Example 10-1, the difference of Example 10-2 was that paliperidone was directly used as the raw material in step (4), and other operations were the same as those in Example 10-1.

**Example 11-1 Paliperidone hollow matrix nano-type rod cores with different particle sizes**

On the basis of Example 5-1, only the grinding time during the nanonization of raw material paliperidone was changed, thereby preparing paliperidone hollow matrix nano-type rod cores with average particle sizes of 820 nm, 646 nm, 430 nm, and 220 nm, respectively.

Examples 12-14 were based on Example 4-1, investigating the types of stabilizers, types of grinding media, and grinding time during the grinding process.

**Example 12 Investigation on types of stabilizers**

Active pharmaceutical ingredient: stabilizer = 5:1. The ball mill speed was 400 r·min$^{-1}$, the grinding media was 0.2 mm of zirconia beads with a volume of 20 mL, and the grinding time was 50 min. The effects of stabilizer types Poloxamer 188 (P188), Tween 80, Poloxamer 407 (P407), SDS, HPMC on the average particle size and PDI (PDI is a dimensionless value reflecting the width of particle size distribution, ranging from 0 to 1, a smaller value indicates a more uniform and narrower particle size distribution) of the active pharmaceutical ingredient after grinding are investigated.

Table 29 Effects of different stabilizers on average particle size and PDI

| Paliperidone/g | Zirconia beads/mL | Zirconia beads/mm | Grinding time /min | Stabilizer | Stabilizer/g | PDI | Average particle size /nm |
|---|---|---|---|---|---|---|---|
| 2.0 | 20 | 0.2 | 50 | P407 | 0.4 | 0.26 | 417 |
| 2.0 | 20 | 0.2 | 50 | P188 | 0.4 | 0.15 | 240 |
| 2.0 | 20 | 0.2 | 50 | Tween | 0.4 | 0.07 | 250 |
| 2.0 | 20 | 0.2 | 50 | SDS | 0.4 | 0.21 | 320 |
| 2.0 | 20 | 0.2 | 50 | HPMC | 0.4 | 0.18 | 330 |

The results showed that the effects of the above five stabilizers met the experimental requirements, and the PDI was less than 0.3.

**Example 13 Investigation on the types of grinding media**

Active pharmaceutical ingredient: stabilizer = 5:1. The ball mill speed was 400 r·min$^{-1}$, Poloxamer 188 was used as the stabilizer, the grinding media was 20 mL, and the grinding time was 50 min. The effects of 0.1 mm, 0.2 mm, and 0.3 mm zirconia beads on the average particle size and PDI of the active pharmaceutical ingredient after grinding were investigated.

Table 30 Effects of different types of grinding media on average particle size and PDI

| Paliperidone/g | Zirconia beads/mL | Zirconia beads/mm | Grinding time /min | Stabilizer | Stabilizer/g | PDI | Average particle size /nm |
|---|---|---|---|---|---|---|---|
| 2.0 | 20 | 0.1 | 50 | P188 | 0.4 | 0.06 | 220 |
| 2.0 | 20 | 0.2 | 50 | P188 | 0.4 | 0.15 | 240 |
| 2.0 | 20 | 0.3 | 50 | P188 | 0.4 | 0.25 | 420 |

The results showed that with the increasing diameter of zirconia beads, the average particle size and PDI were increasing, and the PDI of 0.1-0.3mm zirconia beads was less than 0.3, which could meet the experimental requirements.

Using 0.1mm zirconia beads as grinding media, the final grinding PDI and average particle size were the smallest in the same time.

**Example 14 Investigation on grinding time**

Active pharmaceutical ingredient: stabilizer = 5:1. The ball mill speed was 400 r·min$^{-1}$, Poloxamer 188 was used as the stabilizer, the grinding media was 0.1 mm zirconia beads. The effects of grinding time for 15 min, 25 min, 35 min, 45 min, 50 min, 60 min on the average particle size and PDI of the active pharmaceutical ingredient after grinding were investigated.

Table 31 Effects of different grinding time on average particle size and PDI

| Paliperidone/g | Zirconia beads/mL | Zirconia beads/mm | Grinding time /min | Stabilize r | Stabilizer /g | PDI | Average particle size /nm |
|---|---|---|---|---|---|---|---|
| 2.0 | 20 | 0.1 | 15 | P188 | 0.4 | 0.13 | 820 |
| 2.0 | 20 | 0.1 | 25 | P188 | 0.4 | 0.14 | 646 |
| 2.0 | 20 | 0.1 | 35 | P188 | 0.4 | 0.09 | 430 |
| 2.0 | 20 | 0.1 | 45 | P188 | 0.4 | 0.08 | 246 |
| 2.0 | 20 | 0.1 | 50 | P188 | 0.4 | 0.06 | 220 |
| 2.0 | 20 | 0.1 | 60 | P188 | 0.4 | 0.12 | 253 |

The results show that paliperidone with different particle sizes could be obtained by using 0.1 mm zirconia beads in the grinding process for 15-60 min.

According to Examples 12-14, it could be seen that when the ball mill speed was 400 r•min$^{-1}$, P188 was used as the stabilizer, 0.1 mm zirconia beads were used as the grinding media, the process parameters were grinded for 50 min, and then combined with freeze-drying process after grinding, the freeze-drying was carried out for 24 h. When the obtained nano freeze-dried powder was applied to the implant, the drug release rate could be significantly improved.

**Effect Example 1 Investigation of in vitro release**

**1. Test conditions:**

**(1) Chromatographic conditions of paliperidone in vitro release**

Chromatographic column: Agilent® C18 column (4.6 × 100 mm, 2.7 μm);
Mobile phase: methanol: water (45:55, v/v) (pH was adjusted to 3 with phosphoric acid);
Column temperature: 35°C;
Detection wavelength: 280 nm;
Flow rate: 1.0 mL·min$^{-1}$;
Injection volume: 20 μL.

**(2) Chromatographic conditions for in vitro release of estradiol**

Chromatographic column: Diamonsil® C18 column (250 mm × 4 mm, 5 μm)
Mobile phase: acetonitrile-water (55:45, v/v)
Column temperature: 25°C
Detection wavelength: 202 nm
Flow rate: 0.8 mL·min$^{-1}$
Injection volume: 20 μL

**(3) Chromatographic conditions for in vitro release of meloxicam**

Chromatographic column: Agilent® C18 column (4.6 mm × 150 mm, 5 μm);
Mobile phase: methanol: 0.5% phosphoric acid aqueous solution (70:30, v/v);

Column temperature: room temperature;
Detection wavelength: 270 nm;
Flow rate: 1.0 mL·min$^{-1}$;
Injection volume: 20 μL.

### 2. *In vitro* release rate testing method

A horizontal shaking method was used. One rod core was taken, and both ends of the rod core were fixed to the bottom and wall of a 100 mL conical flask using a silicone adhesive (to prevent the rod core from floating on the liquid surface, which could lead to inaccurate release results). After adhesion, the rod core was left to stand for 12 hours to allow the silicone adhesive to fully cure. Exactly 100 mL of distilled water was measured as the release medium and injected into a conical flask. The conical flask was then placed in a thermostatic air shaker and shake it. The temperature was set to 37°C and the amplitude was 100 r min$^{-1}$. The medium was replaced with an equal volume of fresh medium every 1 day.

### 3. Release results

### (1) Paliperidone matrix-type rod core

Table 32 In vitro release results of the paliperidone matrix-type rod core prepared in Example 4-2

| Time (d) | Daily release amount (μg·d$^{-1}$) | Time (d) | Daily release amount (μg·d$^{-1}$) | Time (d) | Daily release amount (μg·d$^{-1}$) |
|---|---|---|---|---|---|
| 1 | 592.46 | | | | |
| 5 | 190.97 | 55 | 169.66 | 105 | 133.14 |
| 10 | 184.81 | 60 | 167.50 | 110 | 139.08 |
| 15 | 181.08 | 65 | 168.83 | 115 | 136.30 |
| 20 | 177.99 | 70 | 169.61 | 120 | 130.87 |
| 25 | 180.59 | 75 | 142.20 | 125 | 131.85 |
| 30 | 171.15 | 80 | 140.69 | 130 | 120.68 |
| 35 | 181.69 | 85 | 145.08 | 135 | 133.47 |
| 40 | 171.50 | 90 | 148.45 | 140 | 133.41 |
| 45 | 170.98 | 95 | 147.92 | 145 | 134.65 |
| 50 | 181.29 | 100 | 140.83 | 150 | 121.31 |

Table 33 In vitro release results of the paliperidone matrix nano-type rod core prepared in Example 4-1

| Time (d) | Daily release amount (μg·d$^{-1}$) | Time (d) | Daily release amount (μg·d$^{-1}$) | Time (d) | Daily release amount (μg·d$^{-1}$) |
|---|---|---|---|---|---|
| 1 | 2226.99 | | | | |
| 5 | 754.55 | 55 | 667.82 | 105 | 562.40 |
| 10 | 761.78 | 60 | 733.03 | 110 | 546.04 |
| 15 | 813.12 | 65 | 599.53 | 115 | 578.77 |
| 20 | 792.96 | 70 | 626.45 | 120 | 495.43 |
| 25 | 780.36 | 75 | 625.76 | 125 | 495.58 |
| 30 | 730.88 | 80 | 583.31 | 130 | 523.46 |
| 35 | 733.52 | 85 | 609.36 | 135 | 421.97 |
| 40 | 673.61 | 90 | 535.37 | 140 | 446.53 |
| 45 | 726.30 | 95 | 512.87 | 145 | 426.28 |
| 50 | 720.52 | 100 | 519.48 | 150 | 424.97 |

The results showed that the release rate of paliperidone matrix nano-type rod core was about 4 times faster than before after the drug was nanonized. (As shown in FIG. 7)

**(2) Paliperidone hollow matrix-type rod core**

Table 34 In vitro release results of the paliperidone hollow matrix-type rod core prepared in Example 5-2

| Time (d) | Daily release amount ($\mu g \cdot d^{-1}$) | Time (d) | Daily release amount ($\mu g \cdot d^{-1}$) | Time (d) | Daily release amount ($\mu g \cdot d^{-1}$) |
|---|---|---|---|---|---|
| 1 | 902.12 | | | | |
| 5 | 408.90 | 25 | 379.27 | 45 | 358.34 |
| 10 | 434.13 | 30 | 375.74 | 50 | 332.66 |
| 15 | 304.62 | 35 | 358.23 | 55 | 322.29 |
| 20 | 375.07 | 40 | 375.58 | 60 | 395.87 |

Table 35 In vitro release results of the paliperidone hollow matrix nano-type rod core prepared in Example 5-1

| Time (d) | Daily release amount ($\mu g \cdot d^{-1}$) | Time (d) | Daily release amount ($\mu$g·d-1) | Time (d) | Daily release amount ($\mu$g·d-1) |
|---|---|---|---|---|---|
| 1 | 3400.28 | | | | |
| 5 | 2553.75 | 25 | 1370.39 | 45 | 1324.36 |
| 10 | 1377.64 | 30 | 1388.57 | 50 | 1298.16 |
| 15 | 1395.32 | 35 | 1357.02 | 55 | 1263.29 |
| 20 | 1363.09 | 40 | 1374.33 | 60 | 1278.48 |

The results showed that: the release rate of paliperidone hollow matrix nano-type rod core was about 4 times faster than before after the drug was nanonized. (As shown in FIG. 8)

**(3) Paliperidone matrix reservoir-type implant**

Table 36 In vitro release results of the paliperidone matrix reservoir-type rod core prepared in Example 6-2

| Time (d) | Daily release amount ($\mu g \cdot d^{-1}$) | Time (d) | Daily release amount ($\mu g \cdot d^{-1}$) | Time (d) | Daily release amount ($\mu g \cdot d^{-1}$) |
|---|---|---|---|---|---|
| 1 | 13.08 | | | | |
| 5 | 9.50 | 55 | 8.66 | 105 | 7.81 |
| 10 | 8.17 | 60 | 7.55 | 110 | 8.13 |
| 15 | 7.24 | 65 | 7.36 | 115 | 7.11 |
| 20 | 8.02 | 70 | 9.53 | 120 | 8.50 |
| 25 | 8.10 | 75 | 7.87 | 125 | 8.24 |
| 30 | 9.82 | 80 | 7.92 | 130 | 7.73 |
| 35 | 9.97 | 85 | 8.18 | 135 | 8.89 |
| 40 | 9.95 | 90 | 8.04 | 140 | 7.23 |
| 45 | 8.55 | 95 | 8.35 | 145 | 7.68 |
| 50 | 7.03 | 100 | 8.57 | 150 | 7.43 |

Table 37 In vitro release results of the paliperidone matrix reservoir nano-type implant prepared in Example 6-1

| Time (d) | Daily release amount ($\mu g \cdot d^{-1}$) | Time (d) | Daily release amount ($\mu g \cdot d^{-1}$) | Time (d) | Daily release amount ($\mu g \cdot d^{-1}$) |
|---|---|---|---|---|---|
| 1 | 41.86 | | | | |
| 5 | 30.40 | 55 | 31.94 | 105 | 27.81 |
| 10 | 24.99 | 60 | 27.12 | 110 | 26.49 |

(continued)

| Time (d) | Daily release amount ($\mu g \cdot d^{-1}$) | Time (d) | Daily release amount ($\mu g \cdot d^{-1}$) | Time (d) | Daily release amount ($\mu g \cdot d^{-1}$) |
|---|---|---|---|---|---|
| 15 | 23.27 | 65 | 27.53 | 115 | 26.63 |
| 20 | 29.88 | 70 | 28.38 | 120 | 26.80 |
| 25 | 23.06 | 75 | 26.43 | 125 | 26.46 |
| 30 | 29.41 | 80 | 26.99 | 130 | 27.88 |
| 35 | 28.53 | 85 | 22.85 | 135 | 25.13 |
| 40 | 28.02 | 90 | 25.34 | 140 | 26.04 |
| 45 | 25.58 | 95 | 28.84 | 145 | 25.39 |
| 50 | 30.81 | 100 | 26.87 | 150 | 26.58 |

The results show that: the release rate of paliperidone matrix reservoir-type implant is about 3.5 times faster than before after the drug is nanonized. (As shown in FIG. 9)

**(4) Paliperidone powder reservoir-type implant**

Table 38 In vitro release results of paliperidone powder reservoir-type implant prepared in Example 7-2

| Time (d) | Daily release amount ($\mu g \cdot d^{-1}$) | Time (d) | Daily release amount ($\mu g \cdot d^{-1}$) | Time (d) | Daily release amount ($\mu g \cdot d^{-1}$) |
|---|---|---|---|---|---|
| 1 | 25.21 | | | | |
| 5 | 11.94 | 55 | 9.98 | 105 | 8.66 |
| 10 | 11.35 | 60 | 9.72 | 110 | 9.01 |
| 15 | 11.97 | 65 | 9.51 | 115 | 8.92 |
| 20 | 11.50 | 70 | 9.50 | 120 | 9.07 |
| 25 | 11.43 | 75 | 9.62 | 125 | 8.73 |
| 30 | 11.57 | 80 | 9.46 | 130 | 8.61 |
| 35 | 10.85 | 85 | 9.53 | 135 | 8.96 |
| 40 | 10.32 | 90 | 9.16 | 140 | 8.50 |
| 45 | 10.43 | 95 | 9.57 | 145 | 8.94 |
| 50 | 10.84 | 100 | 9.42 | 150 | 8.86 |

Table 39 In vitro release results of the paliperidone powder reservoir nano-type implant prepared in Example 7-1

| Time (d) | Daily release amount ($\mu g \cdot d^{-1}$) | Time (d) | Daily release amount ($\mu g \cdot d^{-1}$) | Time (d) | Daily release amount ($\mu g \cdot d^{-1}$) |
|---|---|---|---|---|---|
| 1 | 72.01 | | | | |
| 5 | 54.34 | 55 | 50.97 | 105 | 48.28 |
| 10 | 52.88 | 60 | 50.88 | 110 | 48.03 |
| 15 | 54.27 | 65 | 50.70 | 115 | 48.20 |
| 20 | 52.51 | 70 | 50.66 | 120 | 48.54 |
| 25 | 54.76 | 75 | 50.96 | 125 | 48.30 |
| 30 | 51.49 | 80 | 50.71 | 130 | 48.71 |
| 35 | 51.15 | 85 | 49.69 | 135 | 47.68 |
| 40 | 51.51 | 90 | 49.25 | 140 | 47.93 |
| 45 | 50.04 | 95 | 48.37 | 145 | 47.34 |
| 50 | 51.96 | 100 | 49.34 | 150 | 47.82 |

The results showed that: the release rate of paliperidone powder reservoir nano-type implant was about 6 times faster than before after the drug was nanonized. (As shown in FIG. 10)

**(5) Comparison of in vitro release results of paliperidone hollow matrix nano-type rod cores with different inner diameters**

In vitro release results of paliperidone hollow matrix nano-type rod cores with different inner diameters prepared in Example 10-1 and paliperidone hollow matrix-type rod cores with different inner diameters prepared in Example 10-2 were shown in FIG. 11.

Table 40 In vitro release results of paliperidone hollow matrix-type rod cores with different inner diameters

| Inner diameter 0.6 mm | | Inner diameter 1.0 mm | | Inner diameter 1.4 mm | |
|---|---|---|---|---|---|
| Time (d) | Daily release dose ($\mu g \cdot d^{-1}$) | Time (d) | Daily release dose ($\mu g \cdot d^{-1}$) | Time (d) | Daily release dose ($\mu g \cdot d^{-1}$) |
| 1 | 431.70 | 1 | 587.21 | 1 | 902.12 |
| 5 | 241.65 | 5 | 340.08 | 5 | 408.90 |
| 10 | 211.94 | 10 | 301.52 | 10 | 434.13 |
| 15 | 198.09 | 15 | 283.63 | 15 | 304.62 |
| 20 | 220.43 | 20 | 312.56 | 20 | 375.07 |
| 25 | 223.12 | 25 | 316.09 | 25 | 379.27 |
| 30 | 205.27 | 30 | 292.78 | 30 | 375.74 |

Table 41 In vitro release results of paliperidone hollow matrix nano-type rod cores with different inner diameters

| Inner diameter 0.6 mm | | Inner diameter 1.0mm | | Inner diameter 1.4mm | |
|---|---|---|---|---|---|
| Time (d) | Daily release dose ($\mu g \cdot d^{-1}$) | Time (d) | Daily release dose ($\mu g \cdot d^{-1}$) | Time (d) | Daily release dose ($\mu g \cdot d^{-1}$) |
| 1 | 1561.12 | 1 | 2013.90 | 1 | 3400.28 |
| 5 | 963.52 | 5 | 1397.26 | 5 | 2553.75 |
| 10 | 864.33 | 10 | 1139.23 | 10 | 1377.64 |
| 15 | 848.90 | 15 | 1148.20 | 15 | 1395.32 |
| 20 | 859.35 | 20 | 1144.09 | 20 | 1363.09 |
| 25 | 820.51 | 25 | 1189.03 | 25 | 1370.39 |
| 30 | 837.30 | 30 | 1153.44 | 30 | 1388.57 |

The results showed that: as the inner diameter of paliperidone hollow matrix nano-type rod core increased, in vitro release dose also increased significantly.

**(6) Comparison of in vitro release results of paliperidone hollow matrix nano-type rod cores with different particle sizes**

In vitro release results of paliperidone hollow matrix nano-type rod cores with average particle sizes of 820 nm, 646 nm, 430 nm, and 220 nm prepared in Example 11, and the paliperidone hollow matrix-type rod core prepared in Example 5-2 were shown in FIG. 12.

Table 42 In vitro release results of paliperidone hollow matrix nano-type rod cores with different particle sizes

| Time (d) | Daily release amount ($\mu g \cdot d^{-1}$) | | | | |
|---|---|---|---|---|---|
| | 820nm | 646nm | 430nm | 220nm | 5$\mu$m |
| 1 | 872.12 | 1213.57 | 2031.08 | 3400.28 | 902.12 |
| 5 | 597.95 | 889.21 | 1048.52 | 2553.75 | 408.90 |
| 10 | 636.50 | 778.20 | 1032.84 | 1377.64 | 434.13 |
| 15 | 441.23 | 755.99 | 1009.36 | 1395.32 | 304.62 |
| 20 | 477.02 | 756.41 | 1032.62 | 1363.09 | 375.07 |
| 25 | 465.43 | 768.13 | 1033.51 | 1370.39 | 379.27 |
| 30 | 466.76 | 758.01 | 1037.05 | 1388.57 | 375.74 |

The results showed that: the release rate of paliperidone rod cores with different particle sizes was different, and the smaller the particle size was, the faster the release rate was. In the above paliperidone hollow matrix nano-type rod cores with different particle sizes, the release rate of paliperidone of 220 nm was about 4 times faster than that before nanonization, the release rate of paliperidone of 430 nm was about 3 times faster than that before nanonization, the release rate of paliperidone of 646 nm was about 2 times faster than that before nanonization, the release rate of paliperidone of 820 nm was about 1.5 times faster than that before nanonization.

**(7) Estradiol hollow matrix-type rod core**

Table 43 In vitro release results of the estradiol hollow matrix-type rod core prepared in Example 8-2

| Time (d) | Daily release amount ($\mu g \cdot d^{-1}$) | Time (d) | Daily release amount ($\mu g \cdot d^{-1}$) | Time (d) | Daily release amount ($\mu g \cdot d^{-1}$) |
|---|---|---|---|---|---|
| 1 | 562.39 | | | | |
| 5 | 542.63 | 25 | 559.38 | 45 | 519.88 |
| 10 | 589.75 | 30 | 520.41 | 50 | 510.35 |
| 15 | 639.17 | 35 | 559.29 | 55 | 510.69 |
| 20 | 598.06 | 40 | 520.06 | 60 | 523.32 |

Table 44 In vitro release results of the estradiol hollow matrix nano-type rod core prepared in Example 8-1

| Time (d) | Daily release amount ($\mu g \cdot d^{-1}$) | Time (d) | Daily release amount ($\mu g \cdot d^{-1}$) | Time (d) | Daily release amount ($\mu g \cdot d^{-1}$) |
|---|---|---|---|---|---|
| 1 | 2472.03 | | | | |
| 5 | 2182.67 | 25 | 2144.61 | 45 | 2123.75 |
| 10 | 2145.86 | 30 | 2156.93 | 50 | 2102.52 |
| 15 | 2119.47 | 35 | 2117.64 | 55 | 2101.94 |
| 20 | 2156.16 | 40 | 2121.38 | 60 | 2155.21 |

(8) Meloxicam hollow matrix-type rod core

Table 45 In vitro release results of the meloxicam hollow matrix-type rod core prepared in Example 9-2

| Time (d) | Daily release amount ($\mu g \cdot d^{-1}$) | Time (d) | Daily release amount ($\mu g \cdot d^{-1}$) | Time (d) | Daily release amount ($\mu g \cdot d^{-1}$) |
|---|---|---|---|---|---|
| 1 | 421.35 | | | | |
| 5 | 429.09 | 25 | 438.58 | 45 | 430.24 |
| 10 | 449.78 | 30 | 448.77 | 50 | 425.56 |
| 15 | 439.65 | 35 | 428.61 | 55 | 424.81 |
| 20 | 438.34 | 40 | 447.43 | 60 | 435.35 |

Table 46 In vitro release results of the meloxicam hollow matrix nano-type rod core prepared in Example 9-1

| Time (d) | Daily release amount ($\mu g \cdot d^{-1}$) | Time (d) | Daily release amount ($\mu g \cdot d^{-1}$) | Time (d) | Daily release amount ($\mu g \cdot d^{-1}$) |
|---|---|---|---|---|---|
| 1 | 2105.82 | | | | |
| 5 | 2145.93 | 25 | 2175.17 | 45 | 2153.73 |
| 10 | 2225.57 | 30 | 2190.48 | 50 | 2153.35 |
| 15 | 2145.44 | 35 | 2143.37 | 55 | 2070.87 |
| 20 | 2191.52 | 40 | 2135.19 | 60 | 2225.70 |

The results showed that: the release rate of estradiol of 198nm was about four times faster than that before grinding (FIG. 13), and the release rate of meloxicam of 308nm was about five times faster than that before grinding (FIG. 14).

Conclusion of the full text: the nanonized drug could be applied to matrix-type implant, hollow matrix-type implant, matrix reservoir-type implant, and drug powder reservoir-type implant. Compared to non-nanonized drugs, the release rate could be increased by 2-8 times, and the nanonized drugs had better compatibility with silicone, which was more conducive to the production of implants.

**Example 15-18 Paliperidone hollow matrix-type rod core**

**Formulation and process investigation of hollow matrix-type rod core**

(1) Using tensile strength, elongation at break and tear strength as evaluation indexes, the effects of catalyst amount, hydrogen-containing silicone oil amount, vulcanization temperature and vulcanization time on the mechanical properties of the matrix-type rod core were investigated.

Tensile strength (Ts) was the maximum tensile stress endured by a specimen until fracture. It represented the resistance of a material to maximum uniform plastic deformation and could indicate the ultimate limit of the rod core against external damage. Elongation at break (Eb) was the ratio of the elongated portion to the original length when the specimen broke. It represented the deformation range of the specimen and could indicate the acceptable deformation range of the rod core before fracture. Tear strength (T) was the strength required to tear the specimen, representing the tear resistance of the rod core.

(2) Method for determining mechanical properties:

The prepared matrix-type rod cores were symmetrically placed on the upper and lower grippers of a servo system tensile testing machine. The gripper distance was set at 50 mm. A device to measure elongation was prepared, the tensile rate was set at 200 mm·min$^{-1}$, and the tensile testing machine was started. The maximum tensile stress during the stretching process, the length of the sample when stretched to break, and the force required to tear the sample were recorded. The elongation at break, tensile strength, and tear strength of the samples were calculated according to the following formulas:

$$\text{Elongation at break (\%)}: E_b = 100\,(L_b - L_0) / L_0$$

$L_b$ - the gauge length at break, mm, $L_0$ - the initial gauge length, mm.

$$\text{Tensile strength (Mpa) } Ts = Fm / Wt$$

*F*m - recorded maximum force, *W* - width of the narrow part of the cutter, mm, t - thickness of the length part of the sample.

$$\text{Tear strength (Kn/m) } T = F/t$$

F - maximum force required to tear the sample, t - thickness of the sample (mm).

**Example 15 Investigation on amount of catalyst**

1. The basic formulation and mechanical properties data of the hollow matrix-type rod core were shown in Table 47.

Table 47

| Material name | Amount | | | | |
|---|---|---|---|---|---|
| | Example 15-1 | Example 15-2 | Example 15-3 | Example 15-4 | Example 15-5 |
| Paliperidone | 100 PHR | 100 PHR | 100 PHR | 100 PHR | 100 PHR |
| Content of vinyl in methyl vinyl silicone rubber | 0.18 mol% | 0.18 mol% | 0.18 mol% | 0.18 mol% | 0.18 mol% |
| Amount of methyl vinyl silicone rubber | 100 PHR | 100 PHR | 100 PHR | 100 PHR | 100 PHR |
| Amount of hydrogen-containing silicone oil | 3.56PHR | 3.56PHR | 3.56PHR | 3.56PHR | 3.56PHR |
| Molar ratio of Si-H groups in hydrogen-containing silicone oil to vinyl in methyl vinyl silicone rubber | 4:1 | 4:1 | 4:1 | 4:1 | 4:1 |
| Amount of hydrogen in hydrogen-containing silicone oil | 0.75 mol% | 0.75 mol% | 0.75 mol% | 0.75 mol% | 0.75 mol% |
| 2-methyl-3-butyn-2-ol | 0.98 PHR | 0.98 PHR | 0.98 PHR | 0.98 PHR | 0.98 PHR |
| Platinum catalyst (10000 ppm) | 0.10PHR | 0.20PHR | 0.21PHR | 0.25 PHR | 0.3 PHR |
| Tensile strength (MPa) | 0.90 | 2.08 | 2.13 | 2.10 | 2.05 |
| Elongation at break (%) | 58 | 118 | 125 | 121 | 113 |
| Tear strength (KN/m) | 0.53 | 1.29 | 1.33 | 1.31 | 1.21 |
| Note: PHR (parts per hundreds of rubber) referred to the parts by weight of the component per 100 parts of methyl vinyl silicone rubber; ppm (parts per million) was the parts per million notation, representing the mass concentration of platinum in the platinum catalyst. | | | | | |

2. Preparation process of the hollow matrix-type rod core:

(1) Drying pretreatment of a raw rubber: the raw methyl vinyl silicone rubber was dried at 40 °C for 12 h for later use.
(2) Preparation of component rubbers A and B: R- vinyl silicone rubber with the prescribed amount was evenly divided into components A1 and B1. The hydrogen-containing silicone oil and inhibitor were added to component A1, mixed uniformly in an open mill, and then subjected to thin-pass process through the open mill several times before being removed in the form of sheets to prepare component A2. The platinum catalyst added in the component B1 was mixed uniformly in an open mill, and then performed thin-pass process for several times before being removed in the form of sheets to prepare component B2.
(3) Preparation of the compounded rubber: the prepared sheets of component A2 and component B2 were fed into an open mill at a mass ratio of 1:1 for compounding, and then performed thin-pass process for 4 to 6 times until component A2 and component B2 were sufficiently and uniformly mixed into a compounded rubber.
(4) Preparation of a drug-loaded silicone: paliperidone and the compounded rubber were uniformly mixed on an open mill to prepare drug-loaded silicone, and cut into strips.
(5) Extrusion of hollow matrix-type rod core: a die orifice with a diameter of 2.96 mm and a core mold with a diameter of 1.38 mm were installed. The screw speed of the extruder was set at 4 $r \cdot min^{-1}$. The cut drug-loaded silicone strips were fed into the extruder, and the drug-loaded silicone was continuously extruded through the die orifice under the push of the screw to form hollow matrix-type rod core.
Wherein, the size of hollow matrix-type rod core: the outer diameter of hollow matrix-type rod core was determined by the diameter of die orifice, and the inner diameter was determined by the diameter of core mold.
(6) Vulcanization of hollow matrix-type rod core: the extruded rod core was placed in an oven and vulcanized at 80 °C for 30 min to form an elastomer.

3. The results showed that with the increase of amount of catalyst, the mechanical indexes of the rod core first significantly increased and then slightly decreased, reaching the best when the amount of the catalyst was 0.21 PHR. During the experiment, it was found that when the amount of the catalyst was increased to 0.25 PHR and 0.3 PHR, due to excessive amount, the rubber material became slightly hard during the compounding and extrusion of the drug rubber,

resulting in rough extrusion of the rod core and a decrease in mechanical properties. Therefore, the amount of the catalyst for 10000 ppm could be selected as 0.1-0.3 PHR, most preferably 0.21PHR.

**Example 16 Investigation on amount of hydrogen-containing silicone oil**

1. The basic formulation and mechanical properties data of the hollow matrix-type rod core were shown in Table 48.
2. Preparation process of fixing hollow matrix-type rod core was as described in Example 15.

Table 48

| Material name | Example 16-1 | Example 16-2 | Example 16-3 |
|---|---|---|---|
| Paliperidone | 100 PHR | 100 PHR | 100 PHR |
| Amount of vinyl in methyl vinyl silicone rubber | 0.18 mol% | 0.18 mol% | 0.18 mol% |
| Content of methyl vinyl silicone rubber | 100 PHR | 100 PHR | 100 PHR |
| Amount of hydrogen-containing silicone oil | 2.67PHR | 3.56PHR | 4.44PHR |
| Molar ratio of Si-H groups in hydrogen-containing silicone oil to vinyl in methyl vinyl silicone rubber | 3:1 | 4:1 | 5:1 |
| Amount of hydrogen in hydrogen-containing silicone oil | 0.75 mol% | 0.75 mol% | 0.75 mol% |
| 2-methyl-3-butyn-2-ol | 0.98 PHR | 0.98 PHR | 0.98 PHR |
| Platinum catalyst (10000 ppm) | 0.21PHR | 0.21PHR | 0.21PHR |
| Tensile strength (MPa) | 1.86 | 2.13 | 2.34 |
| Elongation at break (%) | 92 | 125 | 158 |
| Tear strength (KN/m) | 1.12 | 1.33 | 1.48 |

3. The results showed that: with the increase in the amount of hydrogen-containing silicone oil, the mechanical properties of the rod core gradually increased. Generally, in the addition reaction of addition-type silicone rubber, the molar ratio of hydrogen-containing silicone oil to vinyl in the silicone rubber was generally sufficient in the range of 1.2-1.5. However, for the rod core mixed with drug-rubber, the large amount of drug present in the silicone rubber hindered the contact between Si-H groups in the hydrogen-containing silicone oil and vinyl, leading to incomplete reaction. Therefore, it was necessary to further increase the molar ratio to increase the excess of hydrogen-containing silicone oil, so that more effective hydrogen-containing silicone oil could participate in the addition reaction, thereby achieving complete reaction and optimal vulcanization state of the drug core. In this experiment, the molar ratio of hydrogen-containing silicone oil used was selected to be 2.0-6.0.

**Example 17 Investigation on vulcanization temperature**

1. The basic formulation for fixing the hollow matrix-type rod core was Example 16-3.
2. Preparation process of the hollow matrix-type rod core was the same as Example 15, wherein the differences were that:

(5) Extrusion of a rod core: a die orifice with a diameter of 2.96 mm and a core mold with an inner diameter of 1.38 mm were installed, the screw speed of the extruder was set to $6r \cdot min^{-1}$, and the cut drug-loaded silicone strips were fed into the extruder. The drug-loaded silicone was continuously extruded through the die orifice to form rod core.

(6) Vulcanization of rod core: the extruded rod core was placed in an oven and vulcanized (vulcanization process and mechanical properties data were shown in Table 49) to form an elastomer.

Table 49

| Material name | Example 17-1 | Example 17-2 | Example 17-3 | Example 17-4 | Example 17-5 |
|---|---|---|---|---|---|
| Vulcanization time (min) | 20 | 20 | 20 | 20 | 20 |
| Vulcanization temperature (°C) | 30 | 60 | 80 | 100 | 120 |
| Tensile strength (MPa) | 1.43 | 1.74 | 2.01 | 1.92 | 1.68 |
| Elongation at break (%) | 53 | 112 | 143 | 138 | 105 |
| Tear strength (KN/m) | 0.92 | 1.25 | 1.46 | 1.43 | 1.12 |

3. The results show that when the vulcanization temperature was 30-60°C, the mechanical properties were poor and the vulcanization effect was not good. When the vulcanization temperature was 60 °C to 90 °C, the mechanical properties of the drug core first improved and then deteriorated, and they decreased continuously from 90 °C to 120 °C. Therefore, considering both the mechanical properties of the rod core and the thermal stability of the drug, the vulcanization temperature was selected as 60°C to 90 °C, preferably 65 °C to 90 °C.

**Example 18 Investigation on vulcanization time**

1. The basic formulation for fixing the hollow matrix-type rod core was Example 16-3.
2. Preparation process of the hollow matrix-type rod core was the same as Example 15, wherein the differences were that:

(6) Vulcanization of rod core: the extruded rod core was placed in an oven and vulcanized (vulcanization process and mechanical properties data were shown in Table 50) to form an elastomer.

Table 50

| Name | Example 18-1 | Example 18-2 | Example 18-3 | Example 18-4 | Example 18-5 |
|---|---|---|---|---|---|
| Vulcanization time (min) | 10 | 20 | 30 | 40 | 50 |
| Vulcanization temperature (°C) | 80 | 80 | 80 | 80 | 80 |
| Tensile strength (MPa) | 1.88 | 2.01 | 2.34 | 1.93 | 1.88 |
| Elongation at break (%) | 125 | 143 | 158 | 137 | 132 |
| Tear strength (KN/m) | 1.32 | 1.45 | 1.48 | 1.41 | 1.38 |

3. The results showed that: the rod core achieved the optimal mechanical indexes at a vulcanization time of 30 min. When the vulcanization time was 40 min and 50 min, respectively, the mechanical properties of the rod core did not increase with prolonged vulcanization time, indicating that the drug core had reached the optimum vulcanization state at a vulcanization temperature of 80 °C for 30 min. The vulcanization time of the present disclosure could be 10-60 min.

In summary, the final formulation and process of the hollow matrix-type rod core were as follows: 100 PHR paliperidone; 100 PHR methyl vinyl silicone rubber with 0.18% content of vinyl; 4.4 PHR hydrogen-containing silicone oil with 0.75 mol% content of hydrogen; the molar ratio of Si-H groups in hydrogen-containing silicone oil to vinyl in methyl vinyl silicone rubber was 5:1; the content of hydrogen in hydrogen-containing silicone oil was 0.75 mol%; 0.21 PHR platinum catalyst with platinum concentration of 10000 ppm; 0.98 PHR 2-methylbut-3-yn-2-ol; vulcanization temperature was 80 °C; vulcanization time was 30 min. Unless otherwise specified, the basic formulation and process of the following hollow matrix-type rod cores were performed in accordance with the above conditions.

**Example 19 Paliperidone hollow matrix-type rod core**

1. Basic formulation and preparation process of the hollow matrix-type rod core Except for the size of the hollow skeleton core, the basic formulation and preparation process were the same as those obtained in Examples 15-18, except for the size of the hollow matrix-type rod core. Wherein, the screw speed of the extruder was set at 6 $r \cdot min^{-1}$.

2. Investigation on extrusion size of the hollow matrix-type rod core

2.1 Effects of the diameter of die orifice and the diameter of core mold on the diameter of the hollow matrix-type rod core

The effects of die orifice and core mold with different diameters on diameter of the hollow matrix-type rod core were investigated. The die orifice determined the outer diameter of the hollow matrix-type rod core, the core mold determined the inner diameter of the hollow matrix-type rod core. Different sizes of the hollow matrix-type rod cores were prepared by different die orifices and core molds, as shown in Table 51.

Table 51 The hollow matrix-type rod cores with different sizes

| | Die orifice (mm) | Core mold (mm) | Outer diameter of rod core (mm) | Inner diameter of rod core (mm) | Tensile strength (MPa) | Elongation at break (%) | Tear strength (KN/m) |
|---|---|---|---|---|---|---|---|
| Example 19-1 | 2.96 | 0.58 | 3.00 | 0.60 | 2.24 | 146 | 1.37 |
| Example 19-2 | 2.96 | 0.97 | 3.00 | 1.00 | 2.28 | 151 | 1.42 |
| Example 19-3 | 2.96 | 1.38 | 3.00 | 1.40 | 2.34 | 158 | 1.48 |

The experimental results: the actual diameter of the drug-loaded rod core was slightly larger than that of the mold after passing through the die orifice and the core mold. According to the actual needs, the hollow matrix-type rod core with outer diameter of 1.8-6mm and inner diameter of 0.4-2mm could be prepared by matching the die orifice and the core mold with different diameters.

Unless otherwise specified, a die orifice of 2.96 mm and a core mold of 1.38 mm were selected in the following examples, and hollow matrix-type rod core with an outer diameter of 3 mm and an inner diameter of 1.40 mm were finally prepared.

**Example 19-4 Paliperidone matrix-type rod core (solid matrix-type rod core)**

1. The basic formulation was the same as that of the hollow matrix-type rod core in Example 19-3.
2. Preparation process of matrix-type rod core

Steps (1)-(4) were the same as those of Example 19-3;
(5) Extrusion of a rod core: a die orifice with a diameter of 2.96mm was installed, the screw speed of the extruder was set to 6r·min$^{-1}$, and the cut drug-loaded silicone strips were fed into the extruder. The drug-loaded silicone was continuously extruded through the die orifice to form rod core. The diameter of the matrix-type rod core was 3 mm. Wherein, the size of the rod core: the diameter of the matrix-type rod core was determined by the diameter of die orifice.

(6) Vulcanization of rod core: the extruded rod core was placed in an oven and vulcanized at 80 °C for 30 min to form an elastomer.

FIG. 15 was a photograph of the matrix-type rod core prepared in Example 19-4 (left in FIG. 15) and the hollow matrix-type rod core prepared in Example 15-1 (right in FIG. 15).

**Example 20-1 Estradiol hollow matrix-type rod core**

1. Basic formulation: the same as the basic formulation in Example 19-3, except that the drug was replaced with an equivalent amount of estradiol.
2. Except that the vulcanization temperature is 70 °C and the vulcanization time was 50 min, the rest of the preparation process was the same as that of Example 19-3.

**Example 20-2 Estradiol hollow matrix-type rod core**

1. The basic formulation was the same as that of Example 20-1.
2. Except that the vulcanization temperature was 70 °C and the vulcanization time was 50 min, the rest of the preparation process was the same as that of Example 19-4.

**Example 21-1 Gestodene hollow matrix-type rod core**

1. Basic formulation: except that the drug was replaced with an equivalent amount of gestodene, the molar ratio of hydrogen-containing silicone oil to vinyl in methyl vinyl silicone rubber was 4.5:1, the rest was the same as that of Example 19-3.
2. Except that the vulcanization temperature was 90 °C and the vulcanization time was 50 min, the rest of the preparation process was the same as that of Example 19-3.

**Example 21-2 Gestodene matrix-type rod core**

1. The basic formulation was the same as that of Example 21-1.
2. Except that the vulcanization temperature was 90 °C and the vulcanization time was 50 min, the rest of the preparation process was the same as that of Example 19-4.

**Example 22-1 Levothyroxine sodium hollow matrix-type rod core**

1. Basic formulation: except that the drug was replaced with an equivalent amount of levothyroxine sodium, the molar ratio of hydrogen-containing silicone oil to vinyl in methyl vinyl silicone rubber was 3:1, the rest was the same as that of Example 19-3.
2. Except that the vulcanization temperature was 50 °C, the rest of the preparation process was the same as that of Example 19-3.

**Example 22-2 Levothyroxine sodium matrix-type rod core**

1. The basic formulation was the same as that of Example 22-1.
2. Except that the vulcanization temperature was 50 °C, the rest of the preparation process was the same as that of Example 19-4.

**Example 23-1 Metformin hydrochloride hollow matrix-type rod core**

1. The addition of metformin hydrochloride in the basic formulation was adjusted to 30 PHR, and the rest of the basic formulation was the same as that of Example 19-3.
2. Preparation process was the same as that of Example 19-3.

**Example 23-2 Metformin hydrochloride matrix-type rod core**

1. The basic formulation was the same as that of Example 23-1.
2. Preparation process was the same as that of Example 19-4.

**Example 24-1 Paclitaxel hollow matrix-type rod core**

1. The addition of paclitaxel in the basic formulation was adjusted to 80 PHR, and the rest of the basic formulation was the same as that of Example 19-3.
2. Except that the vulcanization temperature was 75 °C, the rest of the preparation process was the same as that of Example 19-3.

**Example 24-2 Paclitaxel matrix-type rod core**

1. The basic formulation was the same as that of Example 24-1.
2. Except that the vulcanization temperature was 75 °C, the rest of the preparation process was the same as that of Example 19-4.

**Example 25-1 Centella asiatica total glycosides hollow matrix-type rod core**

1. The addition of centella asiatica total glycosides in the basic formulation was adjusted to 40 PHR, and the rest of the basic formulation was the same as that of Example 19-3.
2. Preparation process was the same as that of Example 15.

**Example 25-2 Centella asiatica total glycosides matrix-type rod core**

1. The basic formulation was the same as that of Example 25-1.
2. Preparation process was the same as that of Example 19-4.

In vitro release investigation on different preparations in the effect examples

1. Chromatographic conditions for in vitro release of paliperidone: Column: Agilent® C18 column (4.6 × 100 mm, 2.7 μm); Mobile phase: methanol: water (45:55, v/v) (pH adjusted to 3 with phosphoric acid); Column temperature: 35 °C; Detection wavelength: 280 nm; Flow rate: 1.0 mL·min$^{-1}$; Injection volume: 20 μL.
2. Chromatographic conditions for in vitro release of estradiol: Column: Diamonsil® C18 column (250 mm × 4 mm, 5 μm); Mobile phase: acetonitrile: water (55:45, v/v); Column temperature: 25 °C; Detection wavelength: 202 nm; Flow rate: 0.8 mL·min$^{-1}$; Injection volume: 20 μL.
3. Chromatographic conditions for in vitro release of gestodene: Column: Diamonsil® C18 column (4.6 mm × 250 mm, 5 μm); Mobile phase: methanol: water (80:20, v/v); Column temperature: 30 °C; Detection wavelength: 239 nm; Flow rate: 1.0 mL·min$^{-1}$; Injection volume: 20 μL.
4. Chromatographic conditions for in vitro release of levothyroxine sodium: Column: SinoDetect C18 column (4.6* 250 mm, 5μm); Mobile phase: acetonitrile-0.1%TFA (50:50, v/v); Column temperature: 30 °C; Detection wavelength: 225 nm; Flow rate: 0.8mL·min$^{-1}$; Injection volume: 20 μL.
5. Chromatographic conditions for in vitro release of metformin hydrochloride: Column: Agilent® C18 column (4.6 × 100 mm, 2.7 μm); Mobile phase: methanol: water (60:40, v/v) (pH adjusted to 3 with phosphoric acid); Column temperature: 40 °C; Detection wavelength: 218 nm; Flow rate: 1.0 mL·min$^{-1}$; Injection volume: 20 μL.
6. Chromatographic conditions for in vitro release of paclitaxel: Column: Diamonsil® C18 column (4.6 mm × 250 mm, 5 μm); Mobile phase: acetonitrile: water (45:55, v/v); Column temperature: 35 °C; Detection wavelength: 227 nm; Flow rate: 1.0 mL·min$^{-1}$; Injection volume: 20 μL.
7. Chromatographic conditions for in vitro release of centella asiatica total glycosides: Column: Diamonsil® C18 column (4.6 mm × 250 mm, 5 μm) methanol: water (60:40, v/v); Column temperature: 35 °C; Detection wavelength: 276 nm; Flow rate: 1.0 mL·min$^{-1}$; Injection volume: 20 μL.
8. *In vitro* release testing method

A horizontal shaking method was used. One rod core was taken, and both ends of the drug core are fixed to the bottom and wall of a 100 mL conical flask using a silicone adhesive (to prevent the rod core from floating on the liquid surface, which could lead to inaccurate release results). After adhesion, the rod core was left to stand for 12 hours to allow the silicone adhesive to fully cure. Exactly 100 mL of distilled water was measured as the release medium and injected into a conical flask. The conical flask was then placed in a thermostatic air shaker and shook it. The temperature was set to 37°C

and the amplitude was 100 r $min^{-1}$. The medium was replaced with an equal volume of fresh medium every 1 d.

**9 In vitro release results**

**9.1 In vitro release results of paliperidone hollow matrix-type rod cores with different ratios of outer diameter to inner diameter**

Table 52 showed the in vitro release data of paliperidone hollow matrix-type rod cores in Examples (19-1) to (19-3).

Table 52 In vitro release results of paliperidone hollow matrix-type rod cores with different ratios of outer diameter to inner diameter

| Example 19-1 Outer diameter 3 mm, inner diameter 0.6 mm, time (d) | Daily release dose ($\mu g \cdot d^{-1}$) | Example 19-2 Outer diameter 3 mm, inner diameter 1.0 mm, time (d) | Daily release dose ($\mu g \cdot d^{-1}$) | Example 19-3 Outer diameter 3 mm, inner diameter 1.4 mm, time (d) | Daily release dose ($\mu g \cdot d^{-1}$) |
|---|---|---|---|---|---|
| 1 | 431.70 | 1 | 587.21 | 1 | 902.14 |
| 5 | 241.65 | 5 | 340.08 | 5 | 408.40 |
| 10 | 211.94 | 10 | 301.52 | 10 | 434.31 |
| 15 | 198.09 | 15 | 283.63 | 15 | 304.28 |
| 20 | 220.43 | 20 | 312.56 | 20 | 375.07 |
| 25 | 223.12 | 25 | 316.09 | 25 | 379.74 |
| 30 | 205.27 | 30 | 292.78 | 30 | 375.33 |

As could be seen from the above table, different ratios of outer diameter to inner diameter had different release speeds. When the outer diameter was constant, the smaller the inner diameter was, the less water permeated, and the slower the release speed was. In the case of ensuring good mechanical properties and faster release speed, the size of the hollow matrix-type rod core in the examples of the present disclosure was selected as outer diameter of 3 mm and inner diameter of 1.40mm.

**9.2 Comparison of in vitro release results of paliperidone**

Table 53 and Table 54 showed 150-day in vitro release data of paliperidone matrix-type rod core of Example 19-4 and paliperidone hollow matrix-type rod core of Example 19-3, respectively. It could be seen that after preparing the paliperidone matrix-type rod core into a hollow matrix-type rod core, the release rate of the hollow matrix-type rod core was about 3-4 times faster than before.

Table 53 150-day in vitro release data of paliperidone hollow matrix-type rod core

| Time (d) | Daily release dose ($\mu g \cdot d^{-1}$) | Time (d) | Daily release dose ($\mu g \cdot d^{-1}$) | Time (d) | Daily release dose ($\mu g \cdot d^{-1}$) |
|---|---|---|---|---|---|
| 1 | 592.46 | | | | |
| 5 | 190.97 | 55 | 169.66 | 105 | 133.14 |
| 10 | 184.81 | 60 | 167.05 | 110 | 139.08 |
| 15 | 181.08 | 65 | 168.83 | 115 | 136.30 |
| 20 | 177.99 | 70 | 169.61 | 120 | 130.87 |
| 25 | 180.56 | 75 | 142.20 | 125 | 131.85 |
| 30 | 171.10 | 80 | 140.69 | 130 | 120.68 |
| 35 | 181.69 | 85 | 145.08 | 135 | 133.47 |
| 40 | 171.50 | 90 | 148.45 | 140 | 133.34 |

(continued)

| Time (d) | Daily release dose ($\mu g \cdot d^{-1}$) | Time (d) | Daily release dose ($\mu g \cdot d^{-1}$) | Time (d) | Daily release dose ($\mu g \cdot d^{-1}$) |
|---|---|---|---|---|---|
| 45 | 170.98 | 95 | 147.92 | 145 | 134.65 |
| 50 | 181.29 | 100 | 140.83 | 150 | 121.31 |

Table 54 150-day in vitro release data of paliperidone hollow matrix-type rod core

| Time (d) | Daily release dose ($\mu g \cdot d^{-1}$) | Time (d) | Daily release dose ($\mu g \cdot d^{-1}$) | Time (d) | Daily release dose ($\mu g \cdot d^{-1}$) |
|---|---|---|---|---|---|
| 1 | 902.12 | | | | |
| 5 | 408.70 | 55 | 322.29 | 105 | 367.16 |
| 10 | 434.63 | 60 | 395.07 | 110 | 341.03 |
| 15 | 304.26 | 65 | 352.03 | 115 | 384.26 |
| 20 | 375.07 | 70 | 342.29 | 120 | 341.32 |
| 25 | 379.45 | 75 | 351.61 | 125 | 364.11 |
| 30 | 375.38 | 80 | 380.19 | 130 | 320.55 |
| 35 | 358.23 | 85 | 351.83 | 135 | 375.02 |
| 40 | 375.28 | 90 | 364.30 | 140 | 346.17 |
| 45 | 358.71 | 95 | 328.76 | 145 | 334.24 |
| 50 | 332.26 | 100 | 340.21 | 150 | 333.30 |

### 9.3 Comparison of in vitro release results of estradiol

Table 55 and Table 56 showed 150-day in vitro release data of estradiol matrix-type rod core of Example 20-2 and estradiol hollow matrix-type rod core of Example 20-1, respectively. It could be seen from Table 55 and Table 56 that after preparing the estradiol matrix-type rod core into an estradiol hollow matrix-type rod core, the release rate of the hollow matrix-type rod core was about 4 times faster than before.

Table 55 150-day in vitro release data of estradiol matrix-type rod core

| Time (d) | Daily release dose ($\mu g \cdot d^{-1}$) | Time (d) | Daily release dose ($\mu g \cdot d^{-1}$) | Time (d) | Daily release dose ($\mu g \cdot d^{-1}$) |
|---|---|---|---|---|---|
| 1 | 482.36 | | | | |
| 5 | 207.22 | 55 | 168.73 | 105 | 132.36 |
| 10 | 180.97 | 60 | 166.21 | 110 | 134.61 |
| 15 | 182.34 | 65 | 168.74 | 115 | 132.09 |
| 20 | 185.23 | 70 | 157.01 | 120 | 131.77 |
| 25 | 176.50 | 75 | 158.65 | 125 | 121.10 |
| 30 | 175.06 | 80 | 155.04 | 130 | 126.52 |
| 35 | 175.86 | 85 | 158.29 | 135 | 126.01 |
| 40 | 179.67 | 90 | 146.58 | 140 | 123.66 |
| 45 | 179.29 | 95 | 142.06 | 145 | 129.08 |
| 50 | 172.34 | 100 | 142.62 | 150 | 129.89 |

Table 56 150-day in vitro release data of estradiol hollow matrix-type rod core

| Time (d) | Daily release dose ($\mu g \cdot d^{-1}$) | Time (d) | Daily release dose ($\mu g \cdot d^{-1}$) | Time (d) | Daily release dose ($\mu g \cdot d^{-1}$) |
|---|---|---|---|---|---|
| 1 | 562.39 | | | | |
| 5 | 542.23 | 55 | 502.19 | 105 | 517.04 |
| 10 | 589.35 | 60 | 533.03 | 110 | 533.51 |
| 15 | 639.17 | 65 | 517.28 | 115 | 517.25 |
| 20 | 598.36 | 70 | 526.14 | 120 | 526.12 |
| 25 | 559.31 | 75 | 523.19 | 125 | 523.23 |
| 30 | 520.29 | 80 | 526.26 | 130 | 526.60 |
| 35 | 559.83 | 85 | 521.76 | 135 | 516.36 |
| 40 | 520.47 | 90 | 518.30 | 140 | 515.15 |
| 45 | 519.18 | 95 | 517.25 | 145 | 518.35 |
| 50 | 510.30 | 100 | 510.47 | 150 | 513.24 |

### 9.4 Comparison of in vitro release results of gestodene

Table 57 and Table 58 showed 150-day in vitro release data of gestodene matrix-type rod core of Example 21-2 and gestodene hollow matrix-type rod core of Example 21-1, respectively. It could be seen from Table 57 and Table 58 that after preparing the gestodene matrix-type rod core into a gestodene hollow matrix-type rod core, the release rate of the hollow matrix-type rod core was about 3- 4 times faster than before.

Table 57 150-day in vitro release data of gestodene matrix-type rod core

| Time (d) | Daily release dose ($\mu g \cdot d^{-1}$) | Time (d) | Daily release dose ($\mu g \cdot d^{-1}$) | Time (d) | Daily release dose ($\mu g \cdot d^{-1}$) |
|---|---|---|---|---|---|
| 1 | 228.56 | | | | |
| 5 | 134.81 | 55 | 119.08 | 105 | 74.19 |
| 10 | 130.59 | 60 | 115.54 | 110 | 74.72 |
| 15 | 138.35 | 65 | 118.83 | 115 | 78.88 |
| 20 | 140.07 | 70 | 82.10 | 120 | 72.53 |
| 25 | 134.25 | 75 | 101.31 | 125 | 71.57 |
| 30 | 124.58 | 80 | 107.56 | 130 | 77.93 |
| 35 | 121.20 | 85 | 105.94 | 135 | 77.57 |
| 40 | 120.96 | 90 | 84.37 | 140 | 65.16 |
| 45 | 118.38 | 95 | 85.09 | 145 | 69.73 |
| 50 | 117.81 | 100 | 81.57 | 150 | 68.19 |

Table 58 150-day in vitro release data of gestodene hollow matrix-type rod core

| Time (d) | Daily release dose ($\mu g \cdot d^{-1}$) | Time (d) | Daily release dose ($\mu g \cdot d^{-1}$) | Time (d) | Daily release dose ($\mu g \cdot d^{-1}$) |
|---|---|---|---|---|---|
| 1 | 660.15 | | | | |
| 5 | 356.47 | 55 | 243.98 | 105 | 252.35 |
| 10 | 274.83 | 60 | 247.29 | 110 | 242.96 |
| 15 | 269.22 | 65 | 242.46 | 115 | 246.55 |

(continued)

| Time (d) | Daily release dose ($\mu g \cdot d^{-1}$) | Time (d) | Daily release dose ($\mu g \cdot d^{-1}$) | Time (d) | Daily release dose ($\mu g \cdot d^{-1}$) |
|---|---|---|---|---|---|
| 20 | 288.35 | 70 | 261.33 | 120 | 242.67 |
| 25 | 285.64 | 75 | 250.52 | 125 | 246.32 |
| 30 | 246.91 | 80 | 250.84 | 130 | 242.87 |
| 35 | 263.32 | 85 | 262.63 | 135 | 247.13 |
| 40 | 253.01 | 90 | 255.41 | 140 | 234.49 |
| 45 | 253.34 | 95 | 246.22 | 145 | 243.18 |
| 50 | 242.29 | 100 | 242.54 | 150 | 232.56 |

**9.5 Comparation of in vitro release of the levothyroxine sodium:**

Table 59 and Table 60 showed 150-day in vitro release data of levothyroxine sodium matrix-type rod core of Example 22-2 and levothyroxine sodium hollow matrix-type rod core of Example 22-1, respectively. It could be seen from Table 59 and Table 60 that after preparing the levothyroxine sodium matrix-type rod core into levothyroxine sodium hollow matrix-type rod core, the release rate of the hollow matrix-type rod core was about 3- 4 times faster than before.

Table 59 150-day in vitro release data of levothyroxine sodium matrix-type rod core

| Time (d) | Daily release dose ($\mu g \cdot d^{-1}$) | Time (d) | Daily release dose ($\mu g \cdot d^{-1}$) | Time (d) | Daily release dose ($\mu g \cdot d^{-1}$) |
|---|---|---|---|---|---|
| 1 | 301.29 | | | | |
| 5 | 142.44 | 55 | 122.77 | 105 | 113.54 |
| 10 | 148.73 | 60 | 123.68 | 110 | 113.78 |
| 15 | 144.30 | 65 | 120.55 | 115 | 113.64 |
| 20 | 147.63 | 70 | 122.42 | 120 | 101.90 |
| 25 | 148.15 | 75 | 119.65 | 125 | 100.42 |
| 30 | 140.98 | 80 | 116.81 | 130 | 100.51 |
| 35 | 140.16 | 85 | 116.37 | 135 | 104.82 |
| 40 | 134.85 | 90 | 119.59 | 140 | 104.16 |
| 45 | 138.20 | 95 | 115.14 | 145 | 104.55 |
| 50 | 137.92 | 100 | 117.80 | 150 | 101.29 |

Table 60 150-day in vitro release data of levothyroxine sodium hollow matrix-type rod core

| Time (d) | Daily release dose ($\mu g \cdot d^{-1}$) | Time (d) | Daily release dose ($\mu g \cdot d^{-1}$) | Time (d) | Daily release dose ($\mu g \cdot d^{-1}$) |
|---|---|---|---|---|---|
| 1 | 666.37 | | | | |
| 5 | 429.06 | 55 | 330.86 | 105 | 330.62 |
| 10 | 339.92 | 60 | 334.13 | 110 | 364.76 |
| 15 | 382.20 | 65 | 324.19 | 115 | 324.37 |
| 20 | 384.72 | 70 | 318.78 | 120 | 328.30 |
| 25 | 364.11 | 75 | 326.07 | 125 | 326.91 |
| 30 | 359.29 | 80 | 335.54 | 130 | 345.26 |
| 35 | 333.85 | 85 | 336.08 | 135 | 376.53 |

(continued)

| Time (d) | Daily release dose ($\mu g \cdot d^{-1}$) | Time (d) | Daily release dose ($\mu g \cdot d^{-1}$) | Time (d) | Daily release dose ($\mu g \cdot d^{-1}$) |
|---|---|---|---|---|---|
| 40 | 341.15 | 90 | 339.76 | 140 | 339.28 |
| 45 | 354.31 | 95 | 330.27 | 145 | 382.49 |
| 50 | 322.26 | 100 | 334.01 | 150 | 320.17 |

**9.6 Comparison of in vitro release results of metformin hydrochloride**

Table 61 showed in vitro release data of metformin hydrochloride matrix-type rod core of Example 23-2 and metformin hydrochloride hollow matrix-type rod core of Example 23-1, respectively. It could be seen from the following table that the hollow matrix-type rod core could improve the drug release rate by about 4 times and keep the drug release stable.

Table 61 In vitro release data of metformin hydrochloride matrix-type rod core (Example 23-2) and hollow matrix-type rod core (Example 23-1)

| Time (d) | Example 23-2 Daily release dose ($\mu g \cdot d^{-1}$) | Time (d) | Example 23-1 Daily release dose ($\mu g \cdot d^{-1}$) |
|---|---|---|---|
| 1 | 1.31 | 1 | 2.19 |
| 5 | 0.65 | 5 | 1.08 |
| 10 | 0.43 | 10 | 1.38 |
| 15 | 0.19 | 15 | 0.61 |
| 20 | 0.23 | 20 | 0.64 |
| 25 | 0.15 | 25 | 0.68 |
| 30 | 0.14 | 30 | 0.57 |

**9.7 Comparison of in vitro release results of paclitaxel**

Table 62 and Table 63 showed in vitro release data of paclitaxel matrix-type rod core of Example 24-2 and paclitaxel hollow matrix-type rod core of Example 24-1, respectively. It could be seen from Table 62 and Table 63 that after preparing the paclitaxel matrix-type rod core into a paclitaxel hollow matrix-type rod core, the release rate of the hollow matrix-type rod core was about 3- 4 times faster than before.

Table 62 150-day in vitro release data of paclitaxel matrix-type rod core

| Time (d) | Daily release dose ($\mu g \cdot d^{-1}$) | Time (d) | Daily release dose ($\mu g \cdot d^{-1}$) | Time (d) | Daily release dose ($\mu g \cdot d^{-1}$) |
|---|---|---|---|---|---|
| 1 | 355.30 | | | | |
| 5 | 108.52 | 55 | 95.18 | 105 | 69.84 |
| 10 | 99.41 | 60 | 91.80 | 110 | 69.01 |
| 15 | 103.69 | 65 | 85.07 | 115 | 69.44 |
| 20 | 103.42 | 70 | 89.45 | 120 | 69.68 |
| 25 | 100.86 | 75 | 85.61 | 125 | 67.62 |
| 30 | 94.53 | 80 | 91.81 | 130 | 68.77 |
| 35 | 94.31 | 85 | 86.64 | 135 | 67.81 |
| 40 | 97.85 | 90 | 86.08 | 140 | 68.14 |
| 45 | 96.50 | 95 | 83.73 | 145 | 68.36 |
| 50 | 97.77 | 100 | 82.06 | 150 | 67.18 |

Table 63 150-day in vitro release data of paclitaxel hollow matrix-type rod core

| Time (d) | Daily release dose (μg·d$^{-1}$) | Time (d) | Daily release dose (μg·d$^{-1}$) | Time (d) | Daily release dose (μg·d$^{-1}$) |
|---|---|---|---|---|---|
| 1 | 435.79 | | | | |
| 5 | 235.48 | 55 | 248.96 | 105 | 239.07 |
| 10 | 259.55 | 60 | 249.71 | 110 | 237.75 |
| 15 | 236.34 | 65 | 249.65 | 115 | 237.41 |
| 20 | 228.42 | 70 | 249.20 | 120 | 229.53 |
| 25 | 242.07 | 75 | 247.25 | 125 | 228.60 |
| 30 | 233.21 | 80 | 248.71 | 130 | 226.67 |
| 35 | 249.88 | 85 | 237.54 | 135 | 231.51 |
| 40 | 247.19 | 90 | 237.38 | 140 | 227.85 |
| 45 | 245.66 | 95 | 238.06 | 145 | 225.96 |
| 50 | 247.04 | 100 | 239.82 | 150 | 225.37 |

**9.8 Comparison of in vitro release results of centella asiatica total glycosides**

Table 64 showed in vitro release data of centella asiatica total glycosides matrix-type rod core of Example 25-2 and centella asiatica total glycosides hollow matrix-type rod core of Example 25-1, respectively. It could be seen from the following table that the hollow matrix-type rod core was suitable for active drugs with a solubility > 30 mg/mL (water as solvent) and a molecular weight > 800 Da, which could increase the drug release rate by about 3-4 times while maintaining stable drug release.

Table 64 In vitro release data of centella asiatica total glycosides matrix-type rod core (Example 25-2) and centella asiatica total glycosides hollow matrix-type rod core (Example 25-1)

| Time (d) | Example 25-2 Daily release dose (μg·d$^{-1}$) | Time (d) | Example 25-1 Daily release dose (μg·d$^{-1}$) |
|---|---|---|---|
| 1 | 2.31 | 1 | 3.62 |
| 5 | 0.83 | 5 | 2.53 |
| 6 | 0.86 | 6 | 2.48 |
| 10 | 0.85 | 10 | 2.51 |
| 15 | 0.69 | 15 | 2.40 |
| 20 | 0.63 | 20 | 2.43 |
| 25 | 0.49 | 25 | 2.29 |
| 30 | 0.52 | 30 | 2.38 |

Although the specific embodiments of the present disclosure have been described above, those skilled in the art should understand that these are merely illustrative examples. Various changes or modifications can be made to these embodiments without departing from the principles and essence of the present disclosure. Therefore, the scope of protection of the present disclosure is defined by the appended claims.

## Claims

**1.** A pharmaceutical composition, which comprises the following raw material components in parts by weight:

1-1000 parts of active pharmaceutical ingredient;
100 parts of R-vinyl silicone rubber;
hydrogen-containing silicone oil;

0.000002-1 part of catalyst;
wherein:

in the R-vinyl silicone rubber, R is a substituted or unsubstituted $C_1$-$C_5$ linear alkane or branched alkane, or a substituted or unsubstituted $C_6$-$C_{20}$ aromatic hydrocarbon;
the content of vinyl in the R-vinyl silicone rubber is 0.10%-0.50 mol%;
the content of Si-H groups in the hydrogen-containing silicone oil is 0.15-1.6 mol%;
the molar ratio of Si-H groups in the hydrogen-containing silicone oil to vinyl in the R-vinyl silicone rubber is (0.5-20):1.

**2.** The pharmaceutical composition according to claim 1, wherein, the active pharmaceutical ingredient is an active drug with a solubility of ≤100 mg/mL (using water as a solvent), preferably comprising the active pharmaceutical ingredient acting on the reproductive system, or, the active pharmaceutical ingredient acting on the urinary system, or, the active pharmaceutical ingredient acting on chronic diseases related to metabolism and nutrition, or, the active pharmaceutical ingredient for treating chronic diseases related to connective tissue and rheumatism, or, the active pharmaceutical ingredient for treating hyperlipidemia, tumors, neuropsychiatric disorders, chronic dental diseases, simple obesity, chronic low back pain, or leukemia, or, the active pharmaceutical ingredient acting on the circulatory system, or, the active pharmaceutical ingredient acting on the respiratory system, or, the active pharmaceutical ingredient acting on the digestive system, or, the active pharmaceutical ingredient acting on the blood system, or, the active pharmaceutical ingredient acting on the endocrine system;

wherein,
the active pharmaceutical ingredient acting on the reproductive system is preferably a contraceptive active pharmaceutical ingredient; the contraceptive active pharmaceutical ingredient is preferably levonorgestrel, gestodene, gestrinone or steroidal estrogen;
the active pharmaceutical ingredient acting on the urinary system is preferably an active pharmaceutical ingredient for treating chronic nephritis, chronic renal failure, or chronic prostatitis;
the active pharmaceutical ingredient acting on chronic diseases related to metabolism and nutrition is preferably an active pharmaceutical ingredient for anti-diabetes, treating nutritional deficiency diseases, anti-gout, or anti-osteoporosis;
the active pharmaceutical ingredient for treating chronic diseases related to connective tissue and rheumatism is preferably an active pharmaceutical ingredient for treating rheumatoid arthritis, systemic lupus erythematosus, ankylosing spondylitis, Sjogren's syndrome, vasculitis, idiopathic inflammatory myopathies, systemic sclerosis, or osteoarthritis;
the active pharmaceutical ingredient for treating hyperlipidemia is preferably a statin drug (e.g., simvastatin, atorvastatin, pravastatin), a fibrate drug (e.g., fenofibrate, bezafibrate, gemfibrozil), or a niacin (e.g., nicotinic acid);
the active pharmaceutical ingredient for treating tumors is preferably an anti-breast cancer drug (e.g., azacitidine, docetaxel, buserelin, tamoxifen, mitoxantrone, adriamycin, paclitaxel, capecitabine, goserelin, cyclophosphamide, megestrol, cetuximab, or leuprorelin), an anti-prostate cancer drug (e.g., degarelix, leuprorelin, histrelin, flutamide, estramustine, cyproterone), an anti-ovarian cancer drug (e.g., carboplatin, topotecan, methotrexate), an anti-rectal cancer drug (e.g., panitumumab), an anti-colon cancer drug (e.g., bevacizumab, oxaliplatin), an anti-liver cancer drug (e.g., sorafenib), an anti-lung cancer drug (e.g., erlotinib, gefitinib, decitabine), an anti-kidney cancer drug (e.g., pazopanib, everolimus, temsirolimus), an anti-gastric cancer drug (e.g., fluorouracil, mitomycin, cisplatin, adriamycin, etoposide), an anti-pancreatic cancer drug (e.g., nimotuzumab), an anti-esophageal cancer drug (e.g., docetaxel, paclitaxel, cisplatin, gemcitabine, tegafur, irinotecan, oxaliplatin, gefitinib, trastuzumab, anlotinib), an anti-skin cancer drug (e.g., fluorouracil), an anti-lymphoma drug (e.g., vincristine, bexarotene, dacarbazine, etoposide), an anti-myeloma drug (e.g., bortezomib), an anti-cervical cancer drug (e.g., bleomycin), or an anti-bladder cancer drug (e.g., epirubicin, BCG vaccine);
the active pharmaceutical ingredient for treating chronic dental diseases is preferably nitroimidazole drug (e.g., metronidazole, tinidazole, ornidazole), penicillin drug, minocycline or chlorhexidine;
the active pharmaceutical ingredient for treating chronic low back pain is preferably a non-steroidal analgesic, such as ibuprofen, celecoxib, tramadol, oxycodone, meloxicam, loxoprofen, acetaminophen-codeine, or voltaren, more preferably ibuprofen or meloxicam;
the active pharmaceutical ingredient acting on the circulatory system preferably is preferably an active pharmaceutical ingredient for treating chronic heart failure, coronary heart disease, congenital heart disease, chronic infective endocarditis, or chronic pericarditis;
the active pharmaceutical ingredient acting on the respiratory system is preferably an active pharmaceutical

ingredient for treating chronic obstructive pulmonary emphysema, asthma, chronic pulmonary heart disease, chronic respiratory failure, silicosis, or pulmonary fibrosis;
the active pharmaceutical ingredient acting on the digestive system is preferably an active pharmaceutical ingredient for treating chronic gastritis, peptic ulcers, intestinal tuberculosis, chronic enteritis, chronic diarrhea, chronic hepatitis, liver cirrhosis, chronic pancreatitis, or chronic cholecystitis;
the active pharmaceutical ingredient acting on the blood system is preferably an active pharmaceutical ingredient for treating chronic anemia, chronic myeloid leukemia, or chronic lymphocytic leukemia;
the active pharmaceutical ingredient acting on the endocrine system is preferably an active pharmaceutical ingredient for treating chronic lymphocytic thyroiditis, hyperthyroidism, or hypothyroidism; the active pharmaceutical ingredient for treating hypothyroidism is preferably thyroid hormones, more preferably levothyroxine, levothyroxine sodium or thyroxine;
and/or,
the pharmaceutical composition meets one or more of the following conditions:

(1) the amount of the active pharmaceutical ingredient is 5-500 parts, preferably, 5-100 parts, such as 10 parts, 20 parts, 50 parts, or 100 parts;
(2) the R-vinyl silicone rubber is methyl vinyl silicone rubber;
(3) the content of vinyl in the R-vinyl silicone rubber is 0.17-0.23mol%, such as 0.17 mol%, 0.18 mol%, 0.19 mol%, 0.20 mol%, 0.21 mol%, 0.22 mol% or 0.23 mol%;
(4) the amount of the hydrogen-containing silicone oil is 0.3-20 parts, preferably 0.3-10 parts, such as 0.3 part, 0.667 part or 1 part;
(5) the content of Si-H groups in the hydrogen-containing silicone oil is 0.5-1.0 mol%, such as 0.75 mol%;
(6) the molar ratio of Si-H groups in the hydrogen-containing silicone oil to vinyl in the R-vinyl silicone rubber is (1-10):1, such as 1.5:1, 2:1, 3:1, 3.5:1, 4:1, 4.5:1 or 6:1;
(7) the catalyst is a catalyst capable of catalyzing the addition reaction between the R-vinyl silicone rubber and the hydrogen-containing silicone oil, such as a platinum catalyst, a platinum-containing catalyst, a rhodium catalyst, a palladium catalyst, or a nickel catalyst, preferably a platinum catalyst and/or a platinum-containing catalyst;
(8) the amount of the catalyst is 0.000002-0.5 part, preferably, 0.05-0.4 part, such as 0.1 part, 0.20 part, 0.21 part, 0.25 part, 0.27 part, or 0.30 part; and,
(9) preferably, the pharmaceutical composition further comprises an inhibitor, which refers to an inhibitor capable of inhibiting the addition reaction between the R-vinyl silicone rubber and the hydrogen-containing silicone oil; the amount of the inhibitor is preferably 0.03-2.0 parts, such as 0.49 part or 0.98 part.

**3.** The pharmaceutical composition according to claim 2, wherein, when the active pharmaceutical ingredient is the pharmaceutical active ingredient acting on the reproductive system, the pharmaceutical composition satisfies one or more of the following conditions:

(1) the particle size of the active pharmaceutical ingredient is 0.001-200 μm, preferably 2-80 μm, such as 2-3 μm;
(2) the amount of the active pharmaceutical ingredient is 5-500 parts, such as 10 parts, 20 parts, 50 parts, 80 parts, or 100 parts;
(3) the amount of the hydrogen-containing silicone oil is 0.3-20 parts, preferably 0.3-5 parts, such as 0.3 part, 0.5 part, 0.667 part, or 1 part;
(4) the amount of the catalyst is 0.10-0.30 part, such as 0.10 part, 0.20 part, 0.21 part, 0.25 part or 0.30 part, preferably 0.2-0.25 part;
(5) the content of vinyl in the R-vinyl silicone rubber is 0.10% -0.50 mol%, preferably 0.10%-0.30mol%, such as 0.18 mol%;
(6) the content of Si-H groups in the hydrogen-containing silicone oil is 0.15-1.6 mol%, preferably 0.15-1.2mol%, such as 0.75 mol%;
(7) the molar ratio of Si-H groups in the hydrogen-containing silicone oil to vinyl in the R-vinyl silicone rubber is (1.5-10):1, such as 1.5:1, 3.0:1, or 4.5:1, more preferably (3.0-4.5):1;
or,
when the active pharmaceutical ingredient is the active pharmaceutical ingredient acting on the endocrine system, the pharmaceutical composition satisfies one or more of the following conditions:
(8) the particle size of the active pharmaceutical ingredient is 0.001-180 μm, such as 0.01 μm, 0.1 μm, 0.5 μm, 1 μm, 5 μm, 20 μm, 60 μm, 80 μm or 100 μm;
(9) the amount of the active pharmaceutical ingredient is 10-500 parts, such as 100 parts, 150 parts, or 200 parts;
(10) the amount of the hydrogen-containing silicone oil is 0.3-20 parts, preferably 0.3-5 parts, such as 0.3 part, 0.5

part or 1 part;
(11) the amount of the catalyst is 0.20-0.30 part, such as 0.20 part, 0.25 part, 0.27 part, or 0.27 part, preferably 0.25-0.27 part;
(12) the content of vinyl in the R-vinyl silicone rubber is 0.10%-0.30 mol%, such as 0.17 mol%;
(13) the content of Si-H groups in the hydrogen-containing silicone oil is 0.15-1.2 mol%, such as 0.75 mol%;
(14) the molar ratio of Si-H groups in the hydrogen-containing silicone oil to vinyl in the R-vinyl silicone rubber is (1-6):1, preferably (2-6):1, such as 2: 1, 3:1, 3.5:1, or 4:1, more preferably (3.0-3.5):1.

**4.** A rod core, wherein its raw material components comprise the pharmaceutical composition according to any one of claims 1 to 3;
optionally, the preparation method of the rod core comprises the following steps:

S1: dividing the R-vinyl silicone rubber into component A1 and component A2; mixing the component A1 with the catalyst to obtain component B1, mixing the component A2 with the hydrogen-containing silicone oil to obtain component B2;
S2: compounding the component B1 and the component B2 to obtain a compounded rubber;
S3: compounding the compounded rubber and the active pharmaceutical ingredient to obtain a drug-loaded silicone;
S4: subjecting the drug-loaded silicone to extrusion molding and vulcanization to obtain the rod core.

**5.** An implant, which comprises the rod core according to claim 4 and a silicone tube;
optionally, preparation method of the implant comprises the following steps: filling the rod core according to claim 4 into the silicone tube.

**6.** A raw material composition of a drug-loaded rod core, which comprises the following components in parts by weight:

1-1000 parts of active pharmaceutical ingredient;
100 parts of R-vinyl silicone rubber;
0.45-20 parts of hydrogen-containing silicone oil;
≥ 0.000002 part of catalyst, preferably 0.000002-1 part of catalyst;
wherein:

the average particle size of the active pharmaceutical ingredient is 1-1000 nm;
in the R-vinyl silicone rubber, R is a substituted or unsubstituted $C_1$-$C_5$ linear alkane or branched alkane, or a substituted or unsubstituted $C_6$-$C_{20}$ aromatic hydrocarbon;
the content of vinyl in the R-vinyl silicone rubber is 0.1-0.5 mol%;
the content of Si-H groups in the hydrogen-containing silicone oil is 0.18-1.6 mol%;
the molar ratio of Si-H groups in the hydrogen-containing silicone oil to vinyl in the R-vinyl silicone rubber is (0.5-20):1;
optionally, the raw material composition further comprises an inhibitor, and the inhibitor is an inhibitor capable of inhibiting the addition reaction between the R-vinyl silicone rubber and the hydrogen-containing silicone oil;
optionally, the raw material composition of the drug-loaded rod core meets one or more of the following conditions:

(1) the average particle size of the active pharmaceutical ingredient is 10-900nm, preferably 20nm, 40nm, 60nm, 80nm, 100nm, 120nm, 150nm, 198nm, 200nm, 208nm, 212nm, 220nm, 300nm, 308nm, 350nm, 400nm, 430nm, 500nm, 600nm, 646nm, 700nm, 800nm, 820nm or 900nm;
(2) the active pharmaceutical ingredient is an active drug with solubility I ≤ 100 mg/mL;
(3) the active pharmaceutical ingredient comprises a contraceptive active pharmaceutical ingredient; the contraceptive active pharmaceutical ingredient comprises such as levonorgestrel, gestodene or gestrinone; the active pharmaceutical ingredient can comprise steroid estrogen, such as estradiol; or, the active pharmaceutical ingredient comprises the active pharmaceutical ingredient for treating hyperlipidemia, tumors, neuropsychiatric diseases, chronic dental diseases, simple obesity, chronic low back pain, or leukemia, the active pharmaceutical ingredient for treating neuropsychiatric diseases is preferably a drug for treating schizophrenia, such as paliperidone;
(4) the amount of the active pharmaceutical ingredient is 1-800 parts;
(5) the R-vinyl silicone rubber is methyl vinyl silicone rubber; the methyl vinyl silicone rubber is preferably

methyl vinyl silicone rubber with a relative molecular weight of 100000-800000 g/mol;
(6) the content of vinyl in the R-vinyl silicone rubber is 0.17-0.23 mol%;
(7) the amount of the hydrogen-containing silicone oil is 0.5-20 parts, preferably 1.2-6.3 parts;
(8) the content of Si-H groups in the hydrogen-containing silicone oil is 0.36-1.6 mol%, preferably 0.5-1 mol%;
(9) the molar ratio of Si-H groups in the hydrogen-containing silicone oil to vinyl in the R-vinyl silicone rubber is (0.8-20):1;
(10) the catalyst is a rhodium catalyst, a palladium catalyst, or a platinum catalyst, preferably a platinum catalyst;
wherein, the concentration of platinum in the platinum catalyst is preferably $1\times10^3$-$1\times10^5$ppm;
(11) the amount of the catalyst is $2\times10^{-6}$-1 part, preferably, 0.03-0.3 part, more preferably 0.1-0.3 part;
(12) the inhibitor is an acetylenic alcohol compound, a nitrogen-containing compound, or an organic peroxide, for another example, methylbutynol, for another example, 2-methyl-3-butyn-2-ol;
(13) the amount of the inhibitor is 0.03-2.5 parts, preferably 0.1-2.0 parts, more preferably 0.3-1.0 part.

**7.** A drug-loaded rod core, which is prepared by the raw material composition of the drug-loaded rod core according to claim 6;

preferably, the drug-loaded rod core is a matrix-type drug-loaded rod core or a hollow matrix-type drug-loaded rod core;
preferably, when the drug-loaded rod core is a matrix-type drug-loaded rod core, the outer diameter of the drug-loaded rod core is 1-10 mm, preferably 1- 6 mm;
preferably, when the drug-loaded rod core is a hollow matrix-type drug-loaded rod core, the outer diameter of the drug-loaded rod core is 1-10 mm, preferably 1- 6 mm;
preferably, when the drug-loaded rod core is a hollow matrix-type drug-loaded rod core, the inner diameter of the drug-loaded rod core is 0.1 mm-4.0 mm, preferably 0.4 mm- 2.0 mm;
preferably, when the drug-loaded rod core is a hollow matrix-type drug-loaded rod core, the diameter ratio of the outer diameter to the inner diameter of the drug-loaded rod core is (1.5-50):1;
optionally, the preparation method of the drug-loaded rod core comprises the following steps:

(1) dividing the R-vinyl silicone rubber into component A1 and component B1, mixing the component A1 with the hydrogen-containing silicone oil and the inhibitor to obtain component A2, mixing the component B1 with the catalyst to obtain component B2; mixing the component A2 with the component B2 to obtain a mixed rubber;
(2) compounding the mixed rubber with the active pharmaceutical ingredient to obtain a drug-loaded silicone;
(3) subjecting the drug-loaded silicone to extrusion molding and vulcanization to obtain a drug-loaded rod core;

optionally, the preparation method of the drug-loaded rod core meets one or more of the following conditions:

(1) in step (1), the R-vinyl silicone rubber is dried at 30-60°C for 1-24 hours for later use;
(2) in step (1), the preparation method of the component B1 or the component B2 is: mixing the respective components uniformly in an open mill, then performing thin-pass process for 4-6 times, and discharging in the form of sheets;
(3) in step (1), the step of compounding the component A2 and the component B2 comprises the following steps: the component A2 and the component B2 are fed into an open mill according to the weight ratio of 1:1 for compounding and thin-pass process for 4-6 times so that the component A2 and the component B2 are sufficiently and uniformly mixed to form a compounded rubber;
(4) in step (2), the active pharmaceutical ingredient is prepared by nanonization, and PDI after nanonization is less than 0.3; preferably, the nanonization adopts a wet grinding method;
(5) a stabilizer and a grinding medium are also added in the grinding process;

the types of the stabilizer are preferably one or more selected from the group consisting of poloxamer 407, poloxamer 188, SDS, HPMC, Tween 80, PVP K30, PEO, TPGS and HPC, more preferably poloxamer 188, Tween 80, poloxamer 407, SDS or HPMC, such as poloxamer 188;
the grinding medium is preferably zirconia beads; the particle size of the zirconia beads is 0.1-0.3 mm, such as 0.1 mm, 0.2 mm or 0.3 mm;
the grinding time may be not less than 15 min, preferably 15-250 min; the grinding speed may be 300-500

r•min$^{-1}$;
after the grinding, it may further comprise freeze-drying operation;

(6) in step (2), the step of compounding the compounded rubber and the active pharmaceutical ingredient comprises the following steps: the compounded rubber and the active pharmaceutical ingredient are fed into an open mill for compounding, so that the compounded rubber and the active pharmaceutical ingredient are sufficiently and uniformly mixed to form a drug-loaded silicone; the drug-loaded silicone may be cut into strips; wherein, the mass ratio of the compounded rubber to the active pharmaceutical ingredient is 1:(0.01-10), preferably 1:(1-5);
(7) in step (3), the drug-loaded rod core is a matrix-type drug-loaded rod core or a hollow matrix-type drug-loaded rod core;
(8) in step (3), during the extrusion molding, the screw speed of the extruder is 1-15 r min$^{-1}$;
(9) in step (3), when the drug-loaded rod core is a matrix-type drug-loaded rod core, the diameter of a die orifice used in the extrusion molding is 1-10 mm, preferably 1-6 mm;
(10) in step (3), when the drug-loaded rod core is a hollow matrix-type drug-loaded rod core, the diameter of a die orifice used in the extrusion molding is 1-10 mm, preferably 1-6 mm;
(11) in step (3), when the drug-loaded rod core is a hollow matrix-type drug-loaded rod core, the diameter of a core mold used in the extrusion molding is 0.1 mm- 4.0 mm;
preferably, when the drug-loaded rod core is a hollow matrix-type drug-loaded rod core, the diameter ratio of the die orifice to the core mold is (1.5-50):1;
(12) in step (3), the vulcanization temperature is 30-120°C;
(13) in step (3), the vulcanization time is 10-100 min.

**8.** A reservoir-type implant, which comprises the drug-loaded rod core according to claim 7 and a silicone tube;

preferably, the outer diameter of the silicone tube is 2.0-10 mm, preferably 2.0-6.4 mm;
preferably, the wall thickness of the silicone tube is 0.1-1.0 mm;
preferably, the length of the silicone tube is 1.5-10 cm;
preferably, the drug release area of the silicone tube is 0.628-31.4cm$^2$, preferably 0.4-15.0cm$^2$;
preferably, the diameter of the drug-loaded rod core is 1-10 mm, preferably 1- 6 mm;
preferably, the length of the drug-loaded rod core is 1.0-10 cm, preferably 1.0- 4.0 cm;
optionally, the preparation method of the reservoir-type implant comprises the following steps: cutting the silicone tube into sections, filling the drug-loaded rod core, and sealing the two ends with silicone to obtain the reservoir-type implant.

**9.** A reservoir-type implant, which comprises a powder-type drug core and a silicone tube, the powder-type drug core comprises an active pharmaceutical ingredient, the average particle size of the active pharmaceutical ingredient is 1-1000 nm;

preferably, the silicone tube is the silicone tube according to claim 8;
preferably, the diameter of the drug-loaded sections in the reservoir-type implant is 1-10 mm, preferably 1- 6.0 mm;
preferably, the length of the drug-loaded sections in the reservoir-type implant is 1.0-10 cm, preferably 1.0- 4.0 cm;
preferably, the average particle size of the active pharmaceutical ingredient is 10-900 nm, preferably 20 nm, 40 nm, 60 nm, 80 nm, 100 nm, 120 nm, 150 nm, 198 nm, 200 nm, 208 nm, 212 nm, 220 nm, 300 nm, 308 nm, 350 nm, 400 nm, 430 nm, 500 nm, 600 nm, 646 nm, 700 nm, 800 nm, 820 nm, or 900 nm;
preferably, the powder-type drug core further comprises insoluble excipient;
optionally, the preparation method of the reservoir-type implant comprises the following steps: cutting the silicone tube into sections, one end is sealed with silicone, the powder-type drug core is filled therein, and the other end is sealed with silicone to obtain the reservoir-type implant.

**10.** A hollow matrix-type drug-loaded rod core, wherein, the raw material composition comprises the following components in parts by weight:

1-1000 parts of active pharmaceutical ingredient;
100 parts of R-vinyl silicone rubber;
0.45-20 parts of hydrogen-containing silicone oil;

≥ 0.000002 part of catalyst;
wherein:

in the R-vinyl silicone rubber, R is a substituted or unsubstituted $C_1$-$C_5$ linear alkane or branched alkane, or a substituted or unsubstituted $C_6$-$C_{20}$ aromatic hydrocarbon;
the content of vinyl in the R-vinyl silicone rubber is 0.1-0.5 mol%;
the content of Si-H groups in the hydrogen-containing silicone oil is 0.18-1.6 mol%;
the molar ratio of Si-H groups in the hydrogen-containing silicone oil to vinyl in the R-vinyl silicone rubber is (0.5-20):1.

**11.** The hollow matrix-type drug-loaded rod core according to claim 10, wherein, the raw material composition satisfies one or more of the following conditions:

(1) the amount of the active pharmaceutical ingredient is 1-800 parts;
(2) the content of vinyl in the R-vinyl silicone rubber is 0.17-0.23 mol%;
(3) the amount of the hydrogen-containing silicone oil is 0.5-20 parts;
(4) the content of Si-H groups in the hydrogen-containing silicone oil is 0.5-1.5 mol%;
(5) the molar ratio of Si-H groups in the hydrogen-containing silicone oil to vinyl in the R-vinyl silicone rubber is (0.8-20):1; and,
(6) the amount of the catalyst is 0.000002-1 part.

**12.** The hollow matrix-type drug-loaded rod core according to claim 11, wherein, the raw material composition satisfies one or more of the following conditions:

(1) the amount of the active pharmaceutical ingredient is 10 parts, 30 parts, 50 parts, 60 parts, 100 parts, 120 parts, 150 parts, 250 parts, 500 parts, or 600 parts;
(2) the content of vinyl in the R-vinyl silicone rubber is 0.17 mol%, 0.18 mol%, 0.19 mol%, 0.20 mol%, 0.22 mol% or 0.23 mol%;
(3) the amount of the hydrogen-containing silicone oil is 1.2-6.3 parts, such as 1.33 parts, 2 parts, 2.67 parts, 3 parts, 3.56 parts, or 4.44 parts;
(4) the content of Si-H groups in the hydrogen-containing silicone oil is 0.75mol%, 0.8 mol%, 1.0 mol% or 1.2 mol%;
(5) the molar ratio of Si-H groups in the hydrogen-containing silicone oil to vinyl in the R-vinyl silicone rubber is 0.8:1, 1.0:1, 1.2:1, 1.5:1, 1.8:1, 2.0:1, 3.0:1, 4:1, 4.5:1, 5:1, 8:1, 10:1 or 15:1, preferably, (3-8):1 or (3-6):1; and,
(6) the amount of the catalyst is 0.000002-0.5 part, such as 0.000005 part, 0.00001 part, 0.00002 part, 0.00003 part, 0.1 part, 0.2 part, 0.21 part, 0.25 part or 0.3 part, preferably 0.03-0.3 part, more preferably 0.1-0.3 part.

**13.** The hollow matrix-type drug-loaded rod core according to claim 10, wherein, the raw material composition satisfies one or more of the following conditions:

(1) R is a substituted or unsubstituted $C_1$-$C_5$ linear alkane in the R-vinyl silicone rubber;

when the R is methyl, the R-vinyl silicone rubber is methyl vinyl silicone rubber;
wherein, the methyl vinyl silicone rubber may be a methyl vinyl silicone rubber with a relative molecular weight of 100000-800000 g/mol;

(2) the catalyst is a rhodium catalyst, a palladium catalyst or a platinum catalyst; wherein, the concentration of platinum in the platinum catalyst may be 10000 ppm;
(3) the raw material composition further comprises an inhibitor, and the inhibitor is an inhibitor capable of inhibiting the addition reaction between the R-vinyl silicone rubber and the hydrogen-containing silicone oil;

the inhibitor may be an acetylenic alcohol compound, a nitrogen-containing compound, or an organic peroxide, for another example, methylbutynol, for another example, 2-methyl-3-butyn-2-ol;
the amount of the inhibitor may be 0.03-5 parts, preferably, 0.1-2.0 parts, more preferably, 0.3-1.0 part, such as 0.3 part, 0.5 part, 0.7 part, 0.9 part, or 0.98 part;

(4) the active pharmaceutical ingredient is one or more selected from the group consisting of macromolecular drugs with good solubility, micromolecule drugs with good solubility, macromolecular drugs with poor solubility

and micromolecule drugs with poor solubility;

wherein, the macromolecular drugs with good solubility refer to active drugs that use water as the solvent, have a solubility > 10 mg/mL, and a molecular weight > 800 Da;
the micromolecule drugs with good solubility refer to active drugs that use water as the solvent, have a solubility > 10 mg/mL, and a molecular weight ≤ 800 Da;
the macromolecular drugs with poor solubility refer to active drugs that use water as the solvent, have a solubility ≤ 10 mg/mL, and a molecular weight > 800 Da;
the micromolecule drugs with poor solubility refer to active drugs that use water as the solvent, have a solubility ≤ 10 mg/mL, and a molecular weight ≤ 800 Da;
preferably,
the macromolecular drugs with good solubility refer to active drugs that use water as the solvent, have a solubility > 30 mg/mL, and a molecular weight > 800 Da, such as one or more selected from the group consisting of exenatide, semaglutide, centella asiatica total glycosides, and human growth hormone;
the micromolecule drugs with good solubility refer to active drugs that use water as the solvent, have a solubility > 30 mg/mL, and a molecular weight ≤ 800 Da, such as metformin hydrochloride and/or doxorubicin hydrochloride,
the macromolecular drugs with poor solubility refer to active drugs that use water as the solvent, have a solubility ≤ 5 mg/mL (using water as the solvent), and a molecular weight > 800 Da, such as one or more selected from the group consisting of bovine insulin, paclitaxel, and levothyroxine sodium;
the micromolecule drugs with poor solubility refer to active drugs that use water as the solvent, have a solubility ≤ 5 mg/mL (using water as the solvent), and a molecular weight ≤ 800 Da, such as one or more selected from the group consisting of estradiol, gestodene, meloxicam, ganciclovir, puerarin and paliperidone;

(5) in the hollow matrix-type drug-loaded rod core, the ratio of the outer diameter to the inner diameter of the hollow matrix-type drug-loaded rod core is 1.5-50: 1, for example, 2.14:1, 3:1, 4:1, 5:1, 8:1, 10:1, 15:1, 20:1, 25:1, 30:1, 35:1 or 40:1;

or, the outer diameter of the hollow matrix-type drug-loaded rod core is 1 mm-10 mm, preferably 2.0 mm, 3.0 mm, 4 mm or 5 mm;
or, the inner diameter of the hollow matrix-type drug-loaded rod core is 0.1 mm-4.0 mm, preferably 0.4 mm-2.0 mm, such as 0.4 mm, 0.6 mm, 0.8 mm, 1.0 mm, 1.2 mm, 1.4 mm, 1.6 mm or 2.0 mm;
preferably, the outer diameter of the hollow matrix-type drug-loaded rod core is 3 mm, the inner diameter is 0.6-1.4mm, 0.6mm, 1.0mm or 1.4mm.

**14.** A preparation method for the hollow matrix drug-loaded rod core according to any one of claims 10 to 13, which comprises the following method:

S1: dividing the R- vinyl silicone rubber into a component A1 and a component B1;
when the raw material composition contains an inhibitor, the component A1 is mixed with the hydrogen-containing silicone oil and the inhibitor to obtain a component A2; the component B1 is mixed with the catalyst to obtain a component B2;
S2: compounding the component A2 and the component B2 to obtain a compounded rubber;
S3: compounding the compounded rubber and the active pharmaceutical ingredient to obtain a drug-loaded silicone;
S4: subjecting the drug-loaded silicone to extrusion molding and vulcanization to obtain a hollow matrix-type drug-loaded rod core;
preferably, the preparation method meets one or more of the following conditions:

(1) in S1, the R-vinyl silicone rubber is pretreated using the following method: drying at 30-60°C for 1-24 h;
(2) in S1, the preparation method of the component A1 or the component B1 comprises the following steps: mixing the respective components uniformly in an open mill, then performing thin-pass process for 4-6 times;
(3) in S2, the step of compounding the component A2 and the component B2 comprises the following steps: the component A2 and the component B2 are fed into an open mill according to the weight ratio of 1:1 for compounding and thin-pass process for 4-6 times so that the component B1 and the component B2 are sufficiently and uniformly mixed to form a compounded rubber;
(4) in S3, the steps of compounding the compounded rubber and the active pharmaceutical ingredient

comprises the following steps: the compounded rubber and the active pharmaceutical ingredient are fed into an open mill for compounding, so that the compounded rubber and the active pharmaceutical ingredient are sufficiently and uniformly mixed to form a drug-loaded silicone;
(5) in S4, during the extrusion, the screw speed of the extruder is 1-15 r $min^{-1}$, such as 4 r $min^{-1}$or 6 r $min^{-1}$;
(6) in S4, during the extrusion, the diameter of the die orifice used is 1-10 mm, such as 2 mm, 2.96 mm, 3.5 mm, 4 mm, 4.5 mm or 5 mm;
(7) in S4, during the extrusion, the diameter of the core mold used is 0.1-4 mm, such as 0.58 mm, 0.8 mm, 0.97 mm, 1.2 mm or 1.38 mm;
(8) in S4, the vulcanization temperature is 25-120°C, preferably 60-90°C, such as 30°C, 50°C, 60°C, 65°C, 70°C, 80°C, 90°C, 100°C or 120°C;
(9) in S4, the vulcanization time is 10-200 min, preferably 10-60 min, such as 10 min, 20 min, 30 min, 40 min or 50 min.

**15.** Use of the rod core according to claim 4, the raw material composition of the drug-loaded rod core according to claim 6, the drug-loaded rod core according to claim 7, or the reservoir-type implant according to claim 8 or 9, or the hollow matrix-type drug-loaded rod core according to any one of claims 10 to 13, as a release rate-regulating medium in a sustained and controlled release formulation.

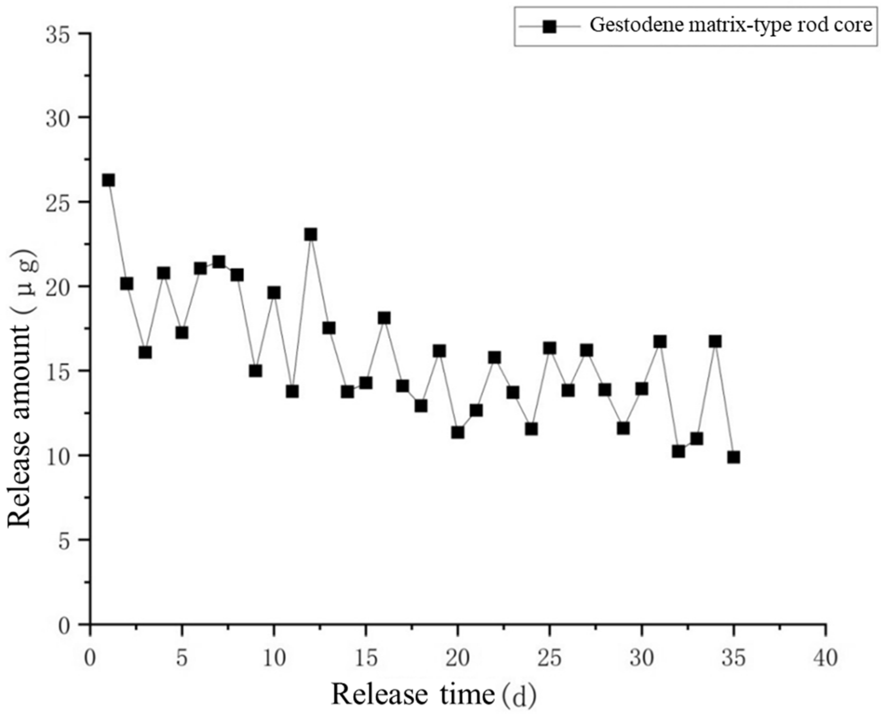
Gestodene matrix-type rod core
Release amount (μg)
Release time (d)
0
5
10
15
20
25
30
35
40

FIG. 1

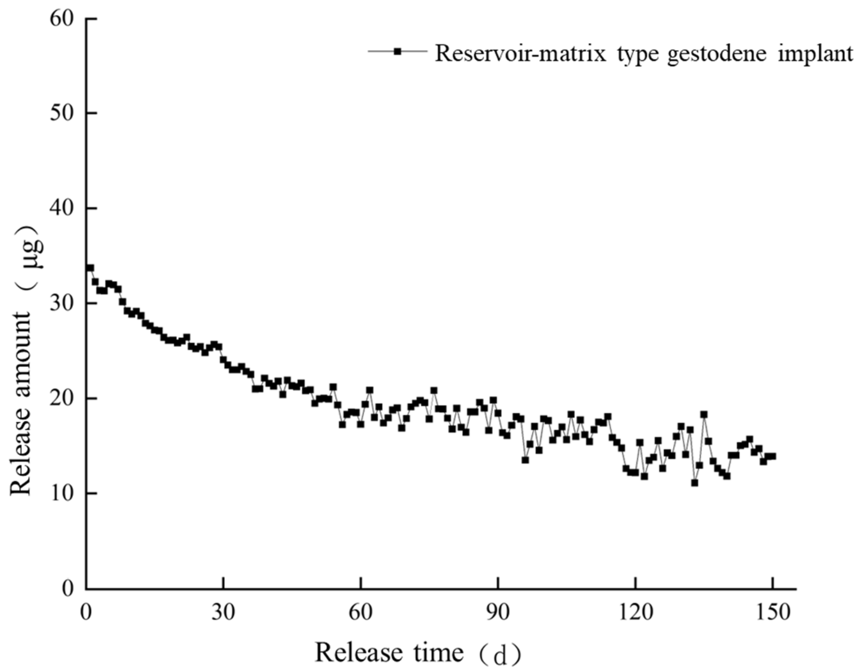
Reservoir-matrix type gestodene implant
Release amount (μg)
Release time (d)
0
10
20
30
40
50
60
90
120
150

FIG. 2

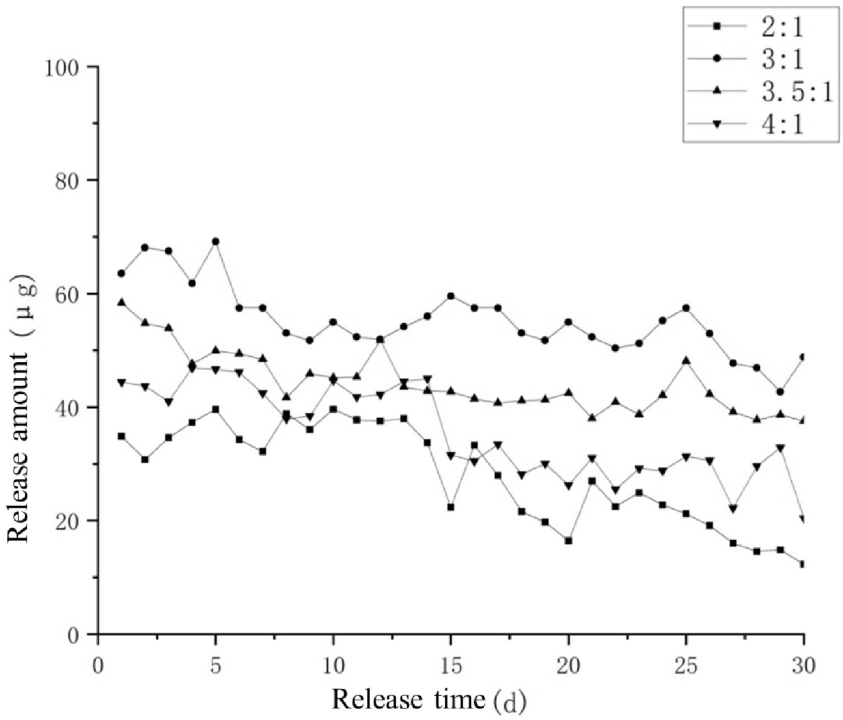
2:1
3:1
3.5:1
4:1
Release amount (μg)
Release time (d)
100
80
60
40
20
0
0
5
10
15
20
25
30

FIG. 3

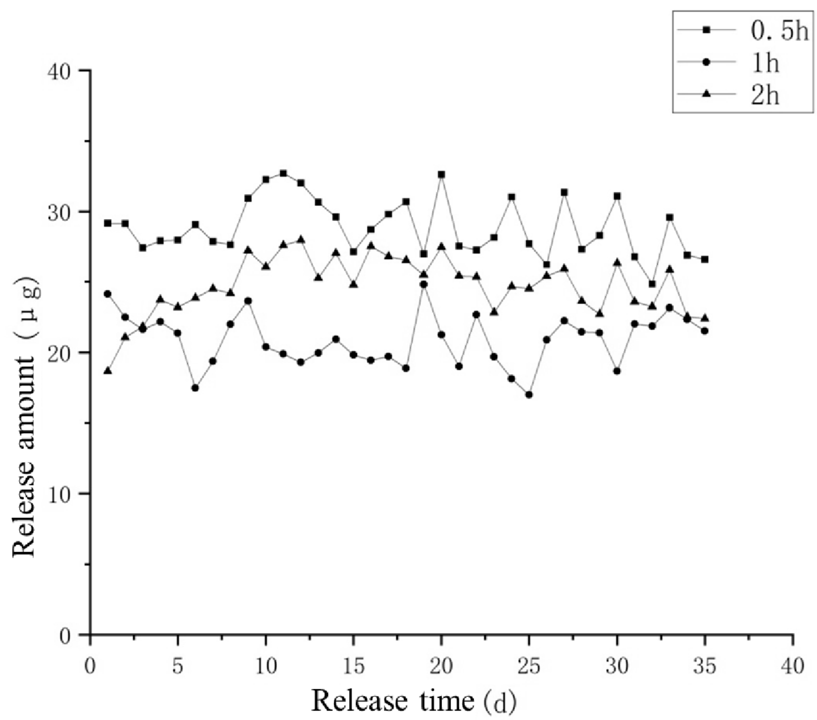
0.5h
1h
2h
Release amount (μg)
Release time (d)
40
30
20
10
0
0
5
10
15
20
25
30
35
40

FIG. 4

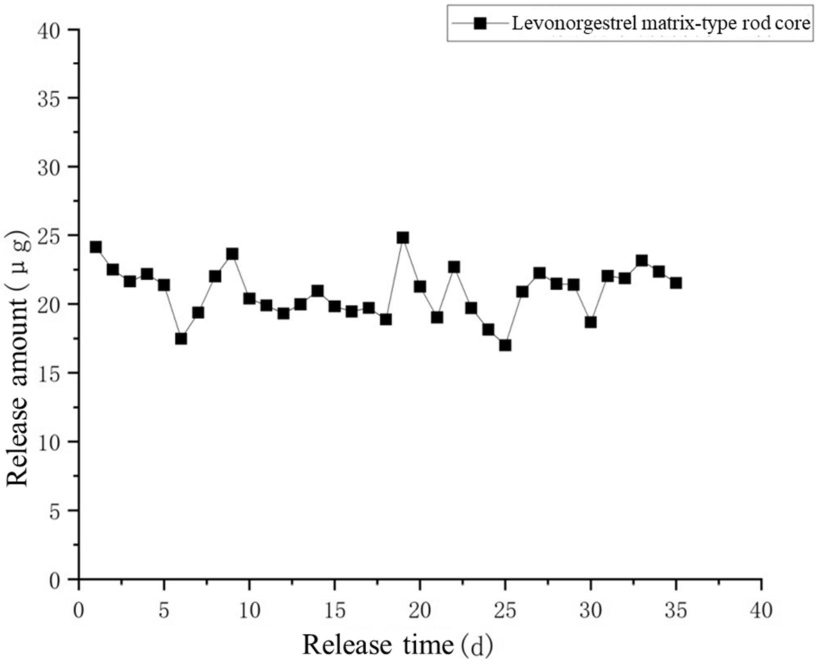
Levonorgestrel matrix-type rod core
Release amount ( μ g)
Release time (d)
0
5
10
15
20
25
30
35
40

FIG. 5

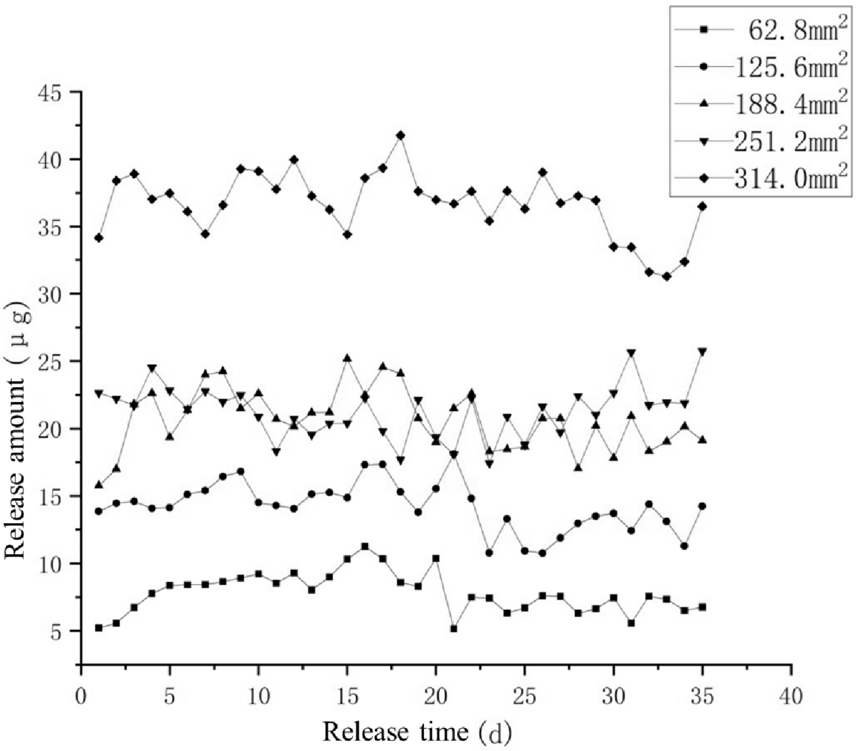
62. 8mm$^2$
125. 6mm$^2$
188. 4mm$^2$
251. 2mm$^2$
314. 0mm$^2$
Release amount ( μ g)
Release time (d)
0
5
10
15
20
25
30
35
40
45

FIG. 6

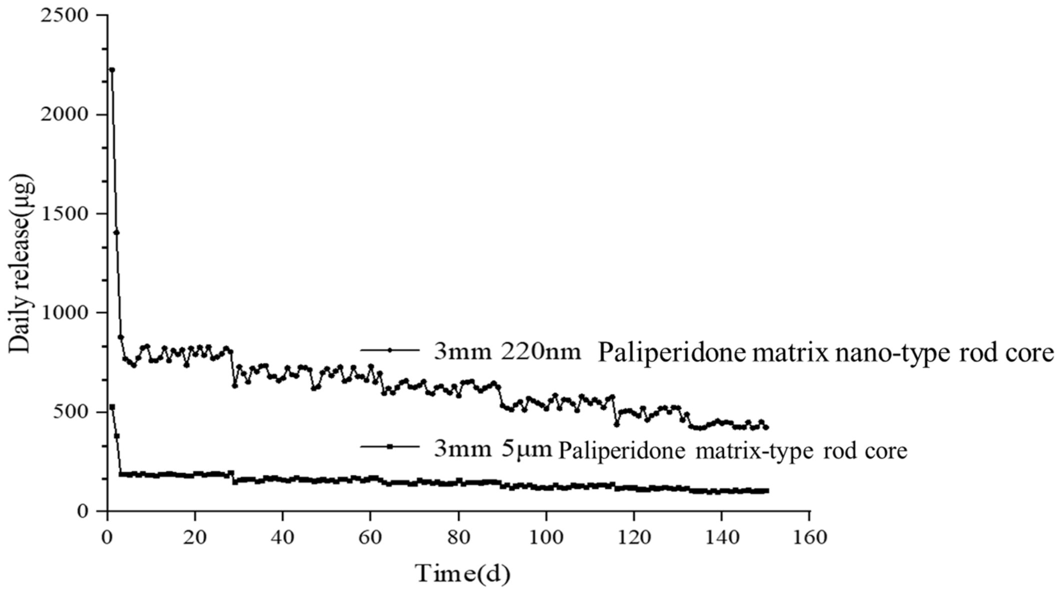

2500
2000
1500
1000
500
0
Daily release(μg)
0
20
40
60
80
100
120
140
160
Time(d)
3mm 220nm Paliperidone matrix nano-type rod core
3mm 5μm Paliperidone matrix-type rod core


FIG. 7

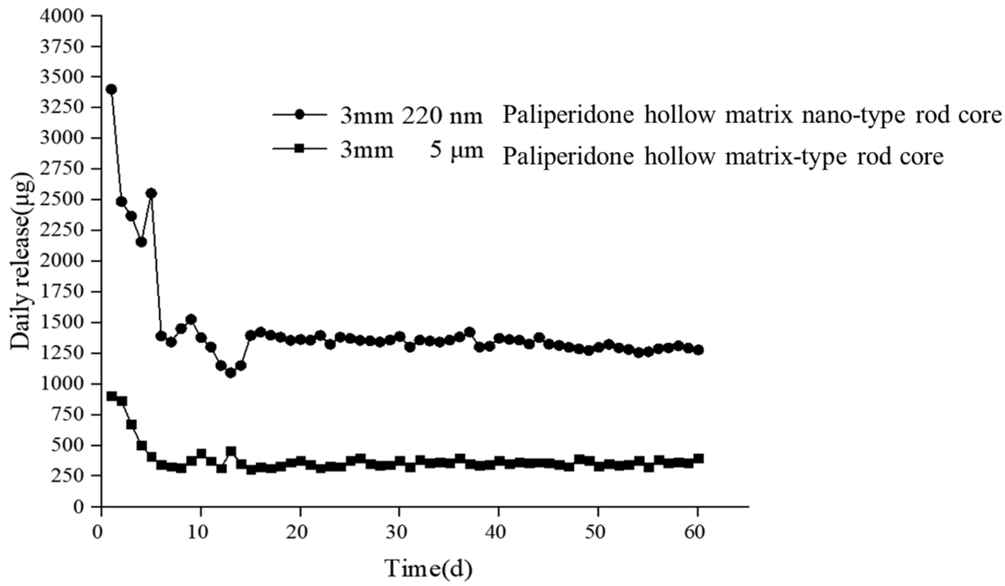

4000
3750
3500
3250
3000
2750
2500
2250
2000
1750
1500
1250
1000
750
500
250
0
Daily release(μg)
0
10
20
30
40
50
60
Time(d)
3mm 220 nm Paliperidone hollow matrix nano-type rod core
3mm 5 μm Paliperidone hollow matrix-type rod core


FIG. 8

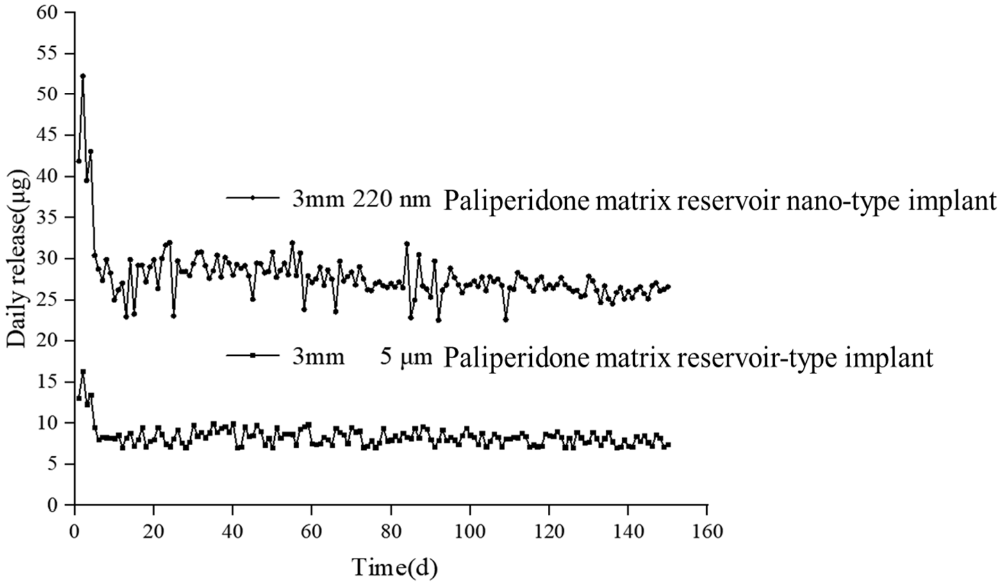

Daily release(μg)
Time(d)
3mm 220 nm Paliperidone matrix reservoir nano-type implant
3mm 5 μm Paliperidone matrix reservoir-type implant


FIG. 9

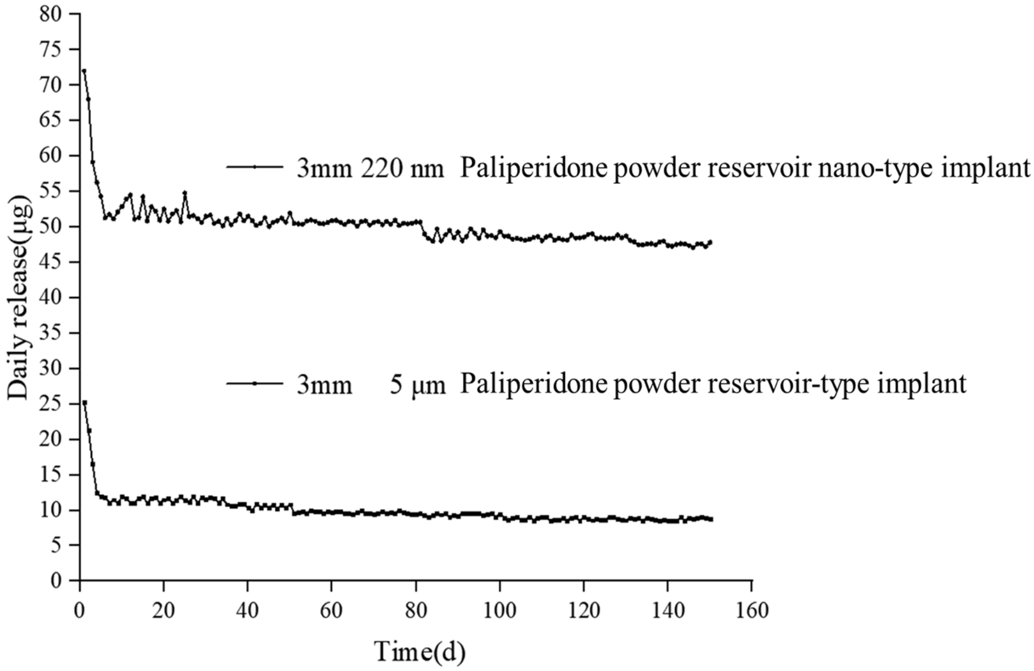

Daily release(μg)
Time(d)
3mm 220 nm Paliperidone powder reservoir nano-type implant
3mm 5 μm Paliperidone powder reservoir-type implant


FIG. 10

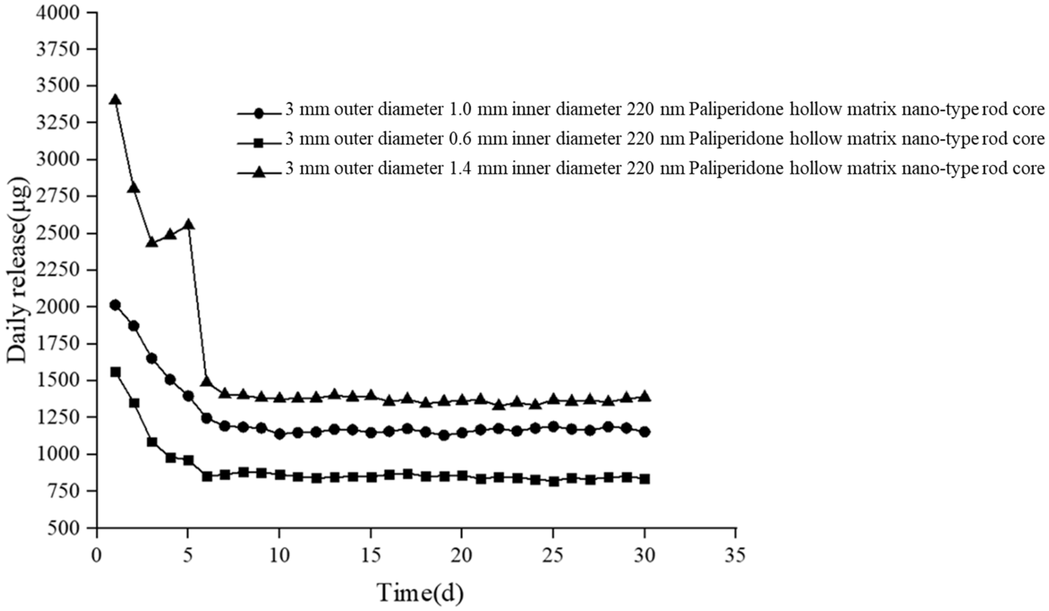
4000
3750
3500
3250
3000
2750
2500
2250
2000
1750
1500
1250
1000
750
500
0
5
10
15
20
25
30
35
Daily release(μg)
Time(d)
3 mm outer diameter 1.0 mm inner diameter 220 nm Paliperidone hollow matrix nano-type rod core
3 mm outer diameter 0.6 mm inner diameter 220 nm Paliperidone hollow matrix nano-type rod core
3 mm outer diameter 1.4 mm inner diameter 220 nm Paliperidone hollow matrix nano-type rod core

FIG. 11

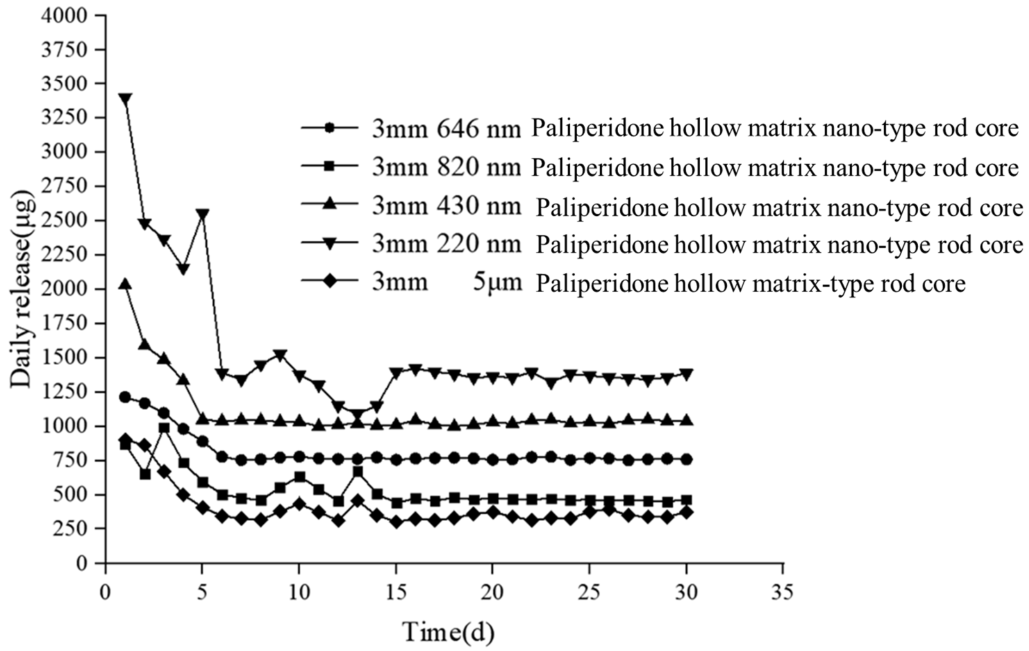
4000
3750
3500
3250
3000
2750
2500
2250
2000
1750
1500
1250
1000
750
500
250
0
0
5
10
15
20
25
30
35
Daily release(μg)
Time(d)
3mm 646 nm Paliperidone hollow matrix nano-type rod core
3mm 820 nm Paliperidone hollow matrix nano-type rod core
3mm 430 nm Paliperidone hollow matrix nano-type rod core
3mm 220 nm Paliperidone hollow matrix nano-type rod core
3mm 5μm Paliperidone hollow matrix-type rod core

FIG. 12

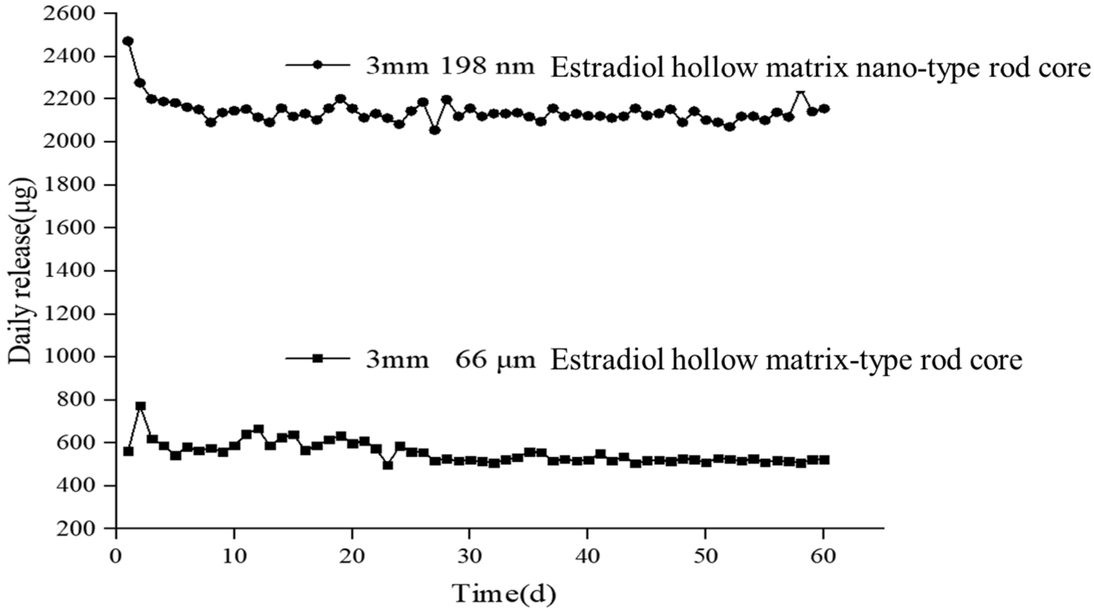

2600
2400
2200
2000
1800
1600
1400
1200
1000
800
600
400
200
Daily release(µg)
3mm 198 nm Estradiol hollow matrix nano-type rod core
3mm 66 µm Estradiol hollow matrix-type rod core
0
10
20
30
40
50
60
Time(d)


FIG. 13

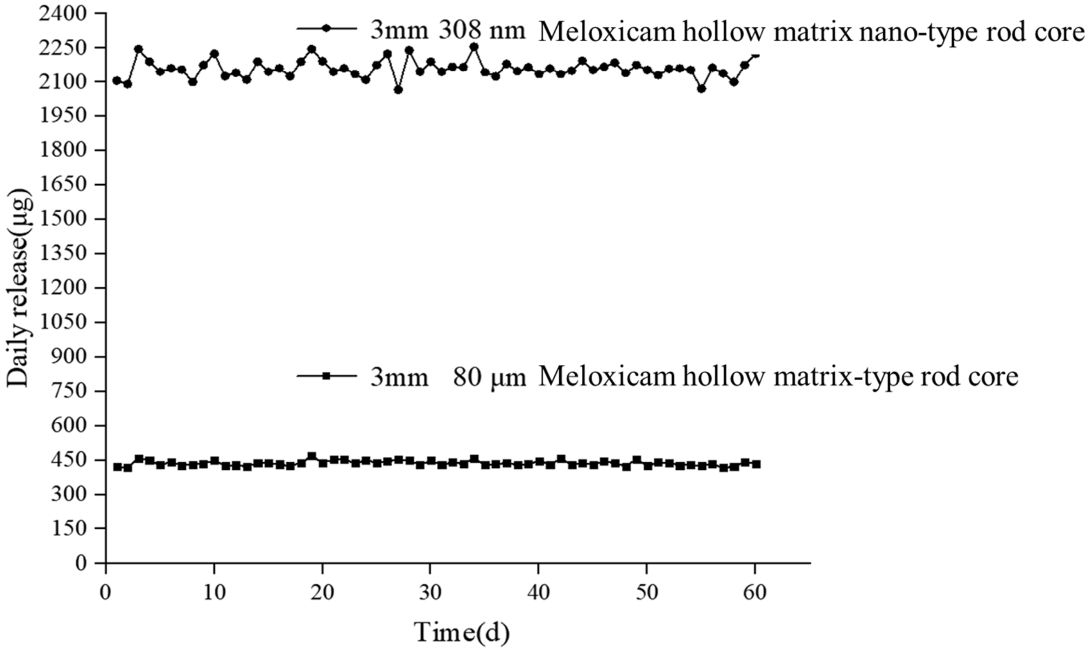

2400
2250
2100
1950
1800
1650
1500
1350
1200
1050
900
750
600
450
300
150
0
Daily release(µg)
3mm 308 nm Meloxicam hollow matrix nano-type rod core
3mm 80 µm Meloxicam hollow matrix-type rod core
0
10
20
30
40
50
60
Time(d)


FIG. 14

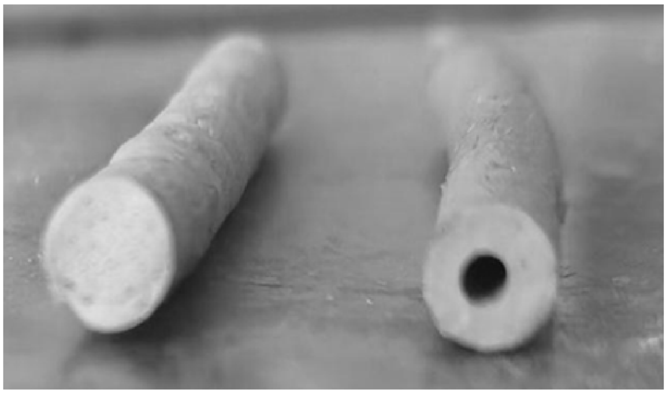

FIG. 15

**INTERNATIONAL SEARCH REPORT**

International application No.
**PCT/CN2024/118883**

**A. CLASSIFICATION OF SUBJECT MATTER**

A61K9/00(2006.01)i; A61K47/34(2017.01)i; A61K31/57(2006.01)i; A61K31/519(2006.01)i; A61K31/198(2006.01)i; A61P25/18(2006.01)i; A61P15/18(2006.01)i; A61P5/14(2006.01)i

According to International Patent Classification (IPC) or to both national classification and IPC

**B. FIELDS SEARCHED**

Minimum documentation searched (classification system followed by classification symbols)

IPC: A61K, A61P

Documentation searched other than minimum documentation to the extent that such documents are included in the fields searched

Electronic data base consulted during the international search (name of data base and, where practicable, search terms used)

CNTXT, WPABS, WPABSC, ENTXT, ENTXTC, DWPI, VEN, CJFD, CNKI, ISI WEB OF SCIENCE, REGISTRY, CAPLUS: 硅橡胶, 聚硅氧烷, 甲基, 乙烯基, 硅油, 催化剂, 铂, 埋植, 植入, 孕二烯酮, 左炔诺孕酮, 帕利哌酮, 雌二醇, 美洛昔康, 左旋甲状腺素钠, 二甲双胍, 紫杉醇, 积雪草, silicone elastomer, silicone rubber, polysiloxane, methyl, vinyl, silicone oil, pt, catalyst, implant+, Norplant, Jadelle, Implanon, pregnanedione, levonorgestrel, paliperidone, estradiol, meloxicam, levothyroxine sodium, metformin, paclitaxel, centella asiatica

**C. DOCUMENTS CONSIDERED TO BE RELEVANT**

| Category* | Citation of document, with indication, where appropriate, of the relevant passages | Relevant to claim No. |
|---|---|---|
| PX | WO 2023174450 A2 (SHENYANG XINGHUA PHARMACEUTICAL TECHNOLOGY CO., LTD.) 21 September 2023 (2023-09-21)<br>claims 1-27 | 1-15 |
| X | CN 1727409 A (SHANGHAI PLASTIC PRODUCTS INSTITUTE) 01 February 2006 (2006-02-01)<br>claims 1-6, and description, pages 3-4 and 6-7, and embodiments 1-4 | 1-3 |
| X | CN 113413358 A (SHANGHAI DAHUA PHARMACEUTICAL CO., LTD.) 21 September 2021 (2021-09-21)<br>claims 1-6, and description, paragraphs 3 and 26, preparation examples BI-B3, and the embodiments | 1-8, 10-15 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **11 December 2024** | **30 December 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/118883**

**C. DOCUMENTS CONSIDERED TO BE RELEVANT**

| Category* | Citation of document, with indication, where appropriate, of the relevant passages | Relevant to claim No. |
|---|---|---|
| Y | 刘伟等 (LIU, Wei et al.). "长效避孕皮下埋植剂体外缓释行为研究 (the Study for the Drug Releasing Behavior in Vitro of the Long Contraceptive Subcutaneous Implants)" *山东生物医学工程 (Shandong Journal of Biomedical Engineering),* Vol. 16, No. 2, 31 December 1997 (1997-12-31), pages 24-27<br>in particular, page 24 | 1-8, 10-15 |
| Y | 刘伟等 (LIU, Wei et al.). "医用级硅橡胶残存单体及低聚物的测定 (Determination of the Residue of the Monomer and Oligomer in Medical Silicone Rubber)" *有机硅材料及应用 (Silicone Material & Applications),* No. 1, 31 December 1999 (1999-12-31), pages 18-19 and 22<br>in particular, page 18 | 1-8, 10-15 |
| X | CN 1179942 A (SHANGHAI INSTITUTE OF PLANNED PARENTHOOD RESEARCH) 29 April 1998 (1998-04-29)<br>claim 2, and embodiments 1-14 | 9, 15 |
| X | CN 115252914 A (SHANDONG BRANDEN MEDICAL DEVICES CO., LTD.) 01 November 2022 (2022-11-01)<br>claims 1-9 | 1-3 |
| X | US 4012497 A (SCHERING AG) 15 March 1977 (1977-03-15)<br>embodiments | 1-3 |
| X | US 5141748 A (HOFFMANN-LA ROCHE LTD.) 25 August 1992 (1992-08-25)<br>description, columns 2-4, and the embodiments | 1-3 |
| X | 郈秀菊等 (HOU, Xiuju et al.). "青蒿琥酯硅橡胶埋植剂的制备与体外释放量的测试 (Preparation and the Test of Release Quantity in vitro of Artesunate Silicone Rubber Implant)" *生物医学工程研究 (Journal of Biomedical Engineering Research),* Vol. 25, No. 2, 31 December 2006 (2006-12-31), pages 113-115<br>in particular, page 114 | 1-3 |
| X | 刘新民等主编 (editor-in-chief LIU, Xinmin et al.). "避孕药 (Non-official translation: Contraceptive)" *实用临床治疗药典 (Practical Clinical Therapy Pharmacopoeia),* 31 January 2003 (2003-01-31), pages 561-565<br>in particular, pages 562-564 | 9, 15 |
| X | 张金哲等 (ZHANG, Jinzhe et al.). "非生物降解型及生物降解型长效皮下埋植避孕剂的理论研究与应用进展 (Non-Biodegradable and Biodegradable Long-term Subdermal Contraceptive Implants: Advances in Theoretical Research and Application)" *中国组织工程研究 (Chinese Journal of Tissue Engineering Research),* Vol. 21, No. 22, 08 August 2017 (2017-08-08), pages 3595-3601<br>in particular, page 3597 | 9, 15 |
| A | CN 101686933 A (BAYER SCHERING PHARMA OY) 31 March 2010 (2010-03-31)<br>claims 1-10 | 1-15 |
| A | Major I. et al. "The Production of Solid Dosage Forms from Non-Degradable Polymers" *Current Pharmaceutical Design,* Vol. 22, 31 December 2016 (2016-12-31), pages 2738-2760<br>in particular, pages 2738-2753 | 1-15 |
| A | 于鲲梦等 (YU, Kunmeng et al.). "植入型给药系统的应用与发展趋势 (Application and Development Trend of Implantable Drug Delivery System)" *药学进展 (Progress in Pharmaceutical Sciences),* Vol. 44, No. 5, 31 December 2020 (2020-12-31), pages 361-370<br>in particular, pages 361-367 | 1-15 |
| A | 叶智厚等 (YE, Zhihou et al.). "一根型双烯高诺酮避孕埋植剂研究 (Contraceptive Effect of a Single Rod Implant Containing Gestodene)" *生殖与避孕 (Reproduction & Contraception),* Vol. 16, No. 3, 31 December 1996 (1996-12-31), pages 187-193<br>in particular, pages 187-188 | 1-15 |

Form PCT/ISA/210 (second sheet) (July 2022)

**INTERNATIONAL SEARCH REPORT**
**Information on patent family members**

International application No.
**PCT/CN2024/118883**

| Patent document cited in search report | Publication date (day/month/year) | Patent family member(s) | Publication date (day/month/year) |
|---|---|---|---|
| WO 2023174450 A2 | 21 September 2023 | WO 2023174450 A3 | 09 November 2023 |
| | | CN 116808226 A | 29 September 2023 |
| | | CN 116808314 A | 29 September 2023 |
| | | CN 117017897 A | 10 November 2023 |
| | | CN 117138051 A | 01 December 2023 |
| | | AU 2023236675 A1 | 31 October 2024 |
| CN 1727409 A | 01 February 2006 | CN 100494282 C | 03 June 2009 |
| CN 113413358 A | 21 September 2021 | CN 113413358 B | 15 November 2022 |
| CN 1179942 A | 29 April 1998 | CN 1088993 C | 14 August 2002 |
| CN 115252914 A | 01 November 2022 | CN 115252914 B | 29 November 2022 |
| US 4012497 A | 15 March 1977 | DD 122197 A5 | 20 September 1976 |
| | | EG 11859 A | 29 March 1978 |
| | | SU 605547 A3 | 30 April 1978 |
| | | NL 7511244 A | 26 March 1976 |
| | | CH 624580 A5 | 14 August 1981 |
| | | SE 7510658 L | 25 March 1976 |
| | | GB 1521505 A | 16 August 1978 |
| | | AU 8506475 A | 31 March 1977 |
| | | AU 498204 B2 | 22 February 1979 |
| | | DK 428175 A | 25 March 1976 |
| | | DK 143384 B | 17 August 1981 |
| | | DK 143384 C | 14 December 1981 |
| | | ATA 724275 A | 15 May 1978 |
| | | AT 347588 B | 10 January 1979 |
| | | PL 183556 A1 | 24 April 1978 |
| | | PL 105375 B1 | 31 October 1979 |
| | | FI 752660 A | 25 March 1976 |
| | | FI 58871 B | 30 January 1981 |
| | | FI 58871 C | 11 May 1981 |
| | | IT 1042731 B | 30 January 1980 |
| | | HU 175675 B | 28 September 1980 |
| | | BG 27880 A3 | 15 January 1980 |
| | | ZA 7506082 B | 29 September 1976 |
| | | DE 2445971 A1 | 08 April 1976 |
| | | ES 441203 A1 | 16 March 1977 |
| | | IL 48100 A0 | 25 November 1975 |
| | | IL 48100 A | 31 January 1979 |
| | | IE 41717 L | 24 March 1976 |
| | | IE 41717 B1 | 12 March 1980 |
| | | BE 833762 A | 24 March 1976 |
| | | JPS 5161618 A | 28 May 1976 |
| | | IN 142359 B | 25 June 1977 |
| | | FR 2285895 A1 | 23 April 1976 |
| | | FR 2285895 B1 | 09 May 1980 |
| | | NO 753230 L | 25 March 1976 |
| | | CA 1049408 A | 27 February 1979 |
| | | ZA 756082 B | 29 September 1976 |
| US 5141748 A | 25 August 1992 | None | |
| CN 101686933 A | 31 March 2010 | MX 2009011539 A | 11 November 2009 |

Form PCT/ISA/210 (patent family annex) (July 2022)

**INTERNATIONAL SEARCH REPORT**
**Information on patent family members**

International application No.
**PCT/CN2024/118883**

| Patent document cited in search report | Publication date (day/month/year) | Patent family member(s) | | | Publication date (day/month/year) |
|---|---|---|---|---|---|
| | | EG | 25756 | A | 26 June 2012 |
| | | AU | 2008244162 | A1 | 06 November 2008 |
| | | AU | 2008244162 | B2 | 07 February 2013 |
| | | CA | 2685467 | A1 | 06 November 2008 |
| | | US | 2010062034 | A1 | 11 March 2010 |
| | | US | 8152793 | B2 | 10 April 2012 |
| | | WO | 2008132274 | A1 | 06 November 2008 |
| | | HK | 1139874 | A1 | 30 September 2010 |
| | | EP | 2139450 | A1 | 06 January 2010 |
| | | EP | 2139450 | A4 | 27 March 2013 |
| | | BRPI | 0809806 | A2 | 07 October 2014 |
| | | IL | 201746 | A0 | 16 June 2010 |
| | | IL | 201746 | A | 31 March 2014 |
| | | JP | 2010524615 | A | 22 July 2010 |
| | | JP | 5406825 | B2 | 05 February 2014 |
| | | KR | 20100017377 | A | 16 February 2010 |
| | | KR | 101461192 | B1 | 13 November 2014 |
| | | RU | 2009143915 | A | 10 June 2011 |
| | | RU | 2462233 | C2 | 27 September 2012 |
| | | FIU | 20070171 | U0 | 27 April 2007 |
| | | FI | 7960 | U1 | 28 July 2008 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202311180554X
- CN 202311180553
- CN 202311180555